# EUROPEAN PATENT APPLICATION

(11) **EP 3 932 934 A1**
(43) Date of publication of application: **05.01.2022**
(21) Application number: 20763771.1
(22) Date of filing: 26.02.2020
(51) Int. Cl.: C07K 1/13, C07K 14/435, C07K 14/81, C07K 16/00, C07K 19/00, G01N 21/64, C12N 5/10, C12N 1/15, C12N 1/19, C12N 1/21, C12N 15/62, C12N 9/14, G01N 33/483, G01N 33/68

(54) **FUSION PROTEIN OF ANTIGEN-BINDING PROTEIN AND FLUORESCENT PROTEIN OR FLUORESCENCE-LABELED TAG PROTEIN**

(30) Priority: 27.02.2019 JP 2019034315
(71) Applicant: National University Corporation Tokyo Medical and Dental University, Tokyo 113-8510 (JP)
(72) Inventor: TERADA, Sumio, Tokyo 113-8510 (JP); SATO, Keisuke, Tokyo 113-8510 (JP); NAKAI, Nori, Tokyo 113-8510 (JP); SAITO, Kenta, Tokyo 113-8510 (JP); KAWAGISHI, Masahiko, Tokyo 113-8510 (JP)
(74) Representative: Fairbairn, Angus Chisholm
(86) International application number: PCT/JP2020/007568
(87) International publication number: WO 2020/175502

(57) **Abstract**

The present invention provides a fusion protein of an antigen-binding protein and a fluorescent protein or a fluorescence-labeled tag protein. The present invention provides a fusion protein of an antigen-binding protein and a fluorescent protein or a fluorescence-labeled tag, wherein the antigen-binding protein is an antigen-binding protein having a helix structure or a β-sheet structure at a terminus, the fluorescent protein or the fluorescence-labeled tag is a fluorescent protein or a fluorescence-labeled tag having a helix structure or a β-sheet structure at a terminus, and the helix at the terminus and the helix at the terminus are linked, or the β-sheet structure at the terminus and the β-sheet structure at the terminus are linked.

SEQUENCE LISTING

<110>  National University Corporation Tokyo Medical and Dental University

<120>  Fusion protein of antigen-binding protein and fluorescent protein

<130>  PTS-9002WO

<150>  JP 2019-034315
<151>  2019-02-27

<160>  43

<170>  PatentIn version 3.5

<210>  1
<211>  241
<212>  PRT
<213>  Artificial Sequence

<220>
<223>  cpGFP

<400>  1

Met Gly Val Val Pro Ile Leu Val Glu Leu Asp Gly Asp Val Asn Gly
1               5                   10                  15

His Lys Phe Ser Val Arg Gly Glu Gly Glu Gly Asp Ala Thr Ile Gly
            20                  25                  30

Lys Leu Thr Leu Lys Phe Ile Cys Thr Thr Gly Lys Leu Pro Val Pro
        35                  40                  45

Trp Pro Thr Leu Val Thr Thr Leu Thr Tyr Gly Val Gln Cys Phe Ser
    50                  55                  60

Arg Tyr Pro Asp His Met Lys Arg His Asp Phe Phe Lys Ser Ala Met
65                  70                  75                  80

Pro Glu Gly Tyr Val Gln Glu Arg Thr Ile Ser Phe Lys Asp Asp Gly
                85                  90                  95

Lys Tyr Lys Thr Arg Ala Val Val Lys Phe Glu Gly Asp Thr Leu Val
            100                 105                 110

Asn Arg Ile Glu Leu Lys Gly Thr Asp Phe Lys Glu Asp Gly Asn Ile
        115                 120                 125

Leu Gly His Lys Leu Glu Tyr Asn Phe Asn Ser His Asn Val Tyr Ile
    130                 135                 140

Thr Ala Asp Lys Gln Lys Asn Gly Ile Lys Ala Asn Phe Thr Val Arg
145                 150                 155                 160

His Asn Val Glu Asp Gly Ser Val Gln Leu Ala Asp His Tyr Gln Gln
                165                 170                 175

Asn Thr Pro Ile Gly Asp Gly Pro Val Leu Leu Pro Asp Asn His Tyr
            180                 185                 190

Leu Ser Thr Gln Thr Val Leu Ser Lys Asp Pro Asn Glu Lys Arg Asp
        195                 200                 205

His Met Val Leu His Glu Tyr Val Asn Ala Ala Gly Ile Thr Leu Gly
    210                 215                 220

Met Asp Glu Leu Tyr Lys Gly Gly Gly Ser Ser Lys Gly Glu Glu Leu
225                 230                 235                 240

Phe

<210>  2
<211>  91
<212>  PRT
<213>  Artificial Sequence

<220>
<223>  Affimer that binds to actin

<400>  2

Ser Leu Glu Ile Glu Glu Leu Ala Arg Phe Ala Val Asp Glu His Asn
1               5                   10                  15

Lys Lys Glu Asn Ala Leu Leu Glu Phe Val Arg Val Val Lys Ala Lys
            20                  25                  30

Glu Gln Ser Ser Val Pro His Trp Trp Trp Thr Thr Met Tyr Tyr Leu
        35                  40                  45

Thr Leu Glu Ala Lys Asp Gly Gly Lys Lys Lys Leu Tyr Glu Ala Lys
    50                  55                  60

Val Trp Val Lys Arg Asp Pro Asn Met Ile Phe Lys Ile Asn Phe Lys
65                  70                  75                  80

Glu Leu Gln Glu Phe Lys Pro Val Gly Asp Ala
                85                  90

<210>  3
<211>  6
<212>  PRT

<213>  Artificial Sequence

<220>
<223>  helix linker

<400>  3

Glu Ala Ala Ala Lys Ala
1               5


<210>  4
<211>  357
<212>  PRT
<213>  Artificial Sequence

<220>
<223>  cpGFP-Actin-binding Affimer

<400>  4

Met Gly Val Val Pro Ile Leu Val Glu Leu Asp Gly Asp Val Asn Gly
1               5                   10                  15

His Lys Phe Ser Val Arg Gly Glu Gly Glu Gly Asp Ala Thr Ile Gly
            20                  25                  30

Lys Leu Thr Leu Lys Phe Ile Cys Thr Thr Gly Lys Leu Pro Val Pro
        35                  40                  45

Trp Pro Thr Leu Val Thr Thr Leu Thr Tyr Gly Val Gln Cys Phe Ser
    50                  55                  60

Arg Tyr Pro Asp His Met Lys Arg His Asp Phe Phe Lys Ser Ala Met
65                  70                  75                  80

Pro Glu Gly Tyr Val Gln Glu Arg Thr Ile Ser Phe Lys Asp Asp Gly
                85                  90                  95

Lys Tyr Lys Thr Arg Ala Val Val Lys Phe Glu Gly Asp Thr Leu Val
            100                 105                 110

Asn Arg Ile Glu Leu Lys Gly Thr Asp Phe Lys Glu Asp Gly Asn Ile
        115                 120                 125

Leu Gly His Lys Leu Glu Tyr Asn Phe Asn Ser His Asn Val Tyr Ile
    130                 135                 140

Thr Ala Asp Lys Gln Lys Asn Gly Ile Lys Ala Asn Phe Thr Val Arg
145                 150                 155                 160

His Asn Val Glu Asp Gly Ser Val Gln Leu Ala Asp His Tyr Gln Gln
                165                 170                 175

Asn Thr Pro Ile Gly Asp Gly Pro Val Leu Leu Pro Asp Asn His Tyr
            180                 185                 190

Leu Ser Thr Gln Thr Val Leu Ser Lys Asp Pro Asn Glu Lys Arg Asp
        195                 200                 205

His Met Val Leu His Glu Tyr Val Asn Ala Ala Gly Ile Thr Leu Gly
    210                 215                 220

Met Asp Glu Leu Tyr Lys Gly Gly Gly Ser Ser Lys Gly Glu Glu Leu
225                 230                 235                 240

Phe Glu Ala Ala Ala Lys Ala Ser Leu Glu Ile Glu Glu Leu Ala Arg
                245                 250                 255

Phe Ala Val Asp Glu His Asn Lys Lys Glu Asn Ala Leu Leu Glu Phe
            260                 265                 270

Val Arg Val Val Lys Ala Lys Glu Gln Ser Ser Val Pro His Trp Trp
        275                 280                 285

Trp Thr Thr Met Tyr Tyr Leu Thr Leu Glu Ala Lys Asp Gly Gly Lys
    290                 295                 300

Lys Lys Leu Tyr Glu Ala Lys Val Trp Val Lys Arg Asp Pro Asn Met
305                 310                 315                 320

Ile Phe Lys Ile Asn Phe Lys Glu Leu Gln Glu Phe Lys Pro Val Gly
                325                 330                 335

Asp Ala Gly Gly Ser Gly Gly Gly Ser Gly Gly Tyr Pro Tyr Asp Val
            340                 345                 350

Pro Asp Tyr Ala Ser
        355

<210>  5
<211>  241
<212>  PRT
<213>  Artificial Sequence

<220>
<223>  cpmVenus

<400>  5

Met Gly Val Val Pro Ile Leu Val Glu Leu Asp Gly Asp Val Asn Gly
1               5                   10                  15

His Lys Phe Ser Val Ser Gly Glu Gly Glu Gly Asp Ala Thr Tyr Gly
            20                  25                  30

Lys Leu Thr Leu Lys Leu Ile Cys Thr Thr Gly Lys Leu Pro Val Pro
        35                  40                  45

Trp Pro Thr Leu Val Thr Thr Leu Gly Tyr Gly Leu Gln Cys Phe Ala
    50                  55                  60

Arg Tyr Pro Asp His Met Lys Gln His Asp Phe Phe Lys Ser Ala Met
65                  70                  75                  80

Pro Glu Gly Tyr Val Gln Glu Arg Thr Ile Phe Phe Lys Asp Asp Gly
                85                  90                  95

Asn Tyr Lys Thr Arg Ala Glu Val Lys Phe Glu Gly Asp Thr Leu Val
            100                 105                 110

Asn Arg Ile Glu Leu Lys Gly Ile Asp Phe Lys Glu Asp Gly Asn Ile
        115                 120                 125

Leu Gly His Lys Leu Glu Tyr Asn Tyr Asn Ser His Asn Val Tyr Ile
    130                 135                 140

Thr Ala Asp Lys Gln Lys Asn Gly Ile Lys Ala Asn Phe Lys Ile Arg
145                 150                 155                 160

His Asn Ile Glu Asp Gly Gly Val Gln Leu Ala Asp His Tyr Gln Gln
                165                 170                 175

Asn Thr Pro Ile Gly Asp Gly Pro Val Leu Leu Pro Asp Asn His Tyr
            180                 185                 190

Leu Ser Tyr Gln Ser Lys Leu Ser Lys Asp Pro Asn Glu Lys Arg Asp
        195                 200                 205

His Met Val Leu Leu Glu Phe Val Thr Ala Ala Gly Ile Thr Leu Gly
    210                 215                 220

Met Asp Glu Leu Tyr Lys Gly Gly Gly Ser Ser Lys Gly Glu Glu Leu
225                 230                 235                 240

Phe

<210>  6
<211>  352

<212>  PRT
<213>  Artificial Sequence

<220>
<223>  cpmVenus-Actin-binding affimer

<400>  6

Met Gly Val Val Pro Ile Leu Val Glu Leu Asp Gly Asp Val Asn Gly
1               5                   10                  15

His Lys Phe Ser Val Ser Gly Glu Gly Glu Gly Asp Ala Thr Tyr Gly
            20                  25                  30

Lys Leu Thr Leu Lys Leu Ile Cys Thr Thr Gly Lys Leu Pro Val Pro
        35                  40                  45

Trp Pro Thr Leu Val Thr Thr Leu Gly Tyr Gly Leu Gln Cys Phe Ala
    50                  55                  60

Arg Tyr Pro Asp His Met Lys Gln His Asp Phe Phe Lys Ser Ala Met
65                  70                  75                  80

Pro Glu Gly Tyr Val Gln Glu Arg Thr Ile Phe Phe Lys Asp Asp Gly
                85                  90                  95

Asn Tyr Lys Thr Arg Ala Glu Val Lys Phe Glu Gly Asp Thr Leu Val
            100                 105                 110

Asn Arg Ile Glu Leu Lys Gly Ile Asp Phe Lys Glu Asp Gly Asn Ile
        115                 120                 125

Leu Gly His Lys Leu Glu Tyr Asn Tyr Asn Ser His Asn Val Tyr Ile
    130                 135                 140

Thr Ala Asp Lys Gln Lys Asn Gly Ile Lys Ala Asn Phe Lys Ile Arg
145                 150                 155                 160

His Asn Ile Glu Asp Gly Gly Val Gln Leu Ala Asp His Tyr Gln Gln
                165                 170                 175

Asn Thr Pro Ile Gly Asp Gly Pro Val Leu Leu Pro Asp Asn His Tyr
            180                 185                 190

Leu Ser Tyr Gln Ser Lys Leu Ser Lys Asp Pro Asn Glu Lys Arg Asp
        195                 200                 205

His Met Val Leu Leu Glu Phe Val Thr Ala Ala Gly Ile Thr Leu Gly
    210                 215                 220

Met Asp Glu Leu Tyr Lys Gly Gly Gly Ser Ser Lys Gly Glu Glu Leu
225                 230                 235                 240

Phe Ala Ser Leu Glu Ile Glu Glu Leu Ala Arg Phe Ala Val Asp Glu
                245                 250                 255

His Asn Lys Lys Glu Asn Ala Leu Leu Glu Phe Val Arg Val Val Lys
            260                 265                 270

Ala Lys Glu Gln Ser Ser Val Pro His Trp Trp Trp Thr Thr Met Tyr
        275                 280                 285

Tyr Leu Thr Leu Glu Ala Lys Asp Gly Gly Lys Lys Lys Leu Tyr Glu
    290                 295                 300

Ala Lys Val Trp Val Lys Arg Asp Pro Asn Met Ile Phe Lys Ile Asn
305                 310                 315                 320

Phe Lys Glu Leu Gln Glu Phe Lys Pro Val Gly Asp Ala Gly Gly Ser
                325                 330                 335

Gly Gly Gly Ser Gly Gly Tyr Pro Tyr Asp Val Pro Asp Tyr Ala Ser
            340                 345                 350

<210>  7
<211>  241
<212>  PRT
<213>  Artificial Sequence

<220>
<223>  cpmTurquoise2

<400>  7

Met Gly Val Val Pro Ile Leu Val Glu Leu Asp Gly Asp Val Asn Gly
1               5                   10                  15

His Lys Phe Ser Val Ser Gly Glu Gly Glu Gly Asp Ala Thr Tyr Gly
            20                  25                  30

Lys Leu Thr Leu Lys Phe Ile Cys Thr Thr Gly Lys Leu Pro Val Pro
        35                  40                  45

Trp Pro Thr Leu Val Thr Thr Leu Ser Trp Gly Val Gln Cys Phe Ala
    50                  55                  60

Arg Tyr Pro Asp His Met Lys Gln His Asp Phe Phe Lys Ser Ala Met
65                  70                  75                  80

Pro Glu Gly Tyr Val Gln Glu Arg Thr Ile Phe Phe Lys Asp Asp Gly
                85                  90                  95

Asn Tyr Lys Thr Arg Ala Glu Val Lys Phe Glu Gly Asp Thr Leu Val
            100                 105                 110

Asn Arg Ile Glu Leu Lys Gly Ile Asp Phe Lys Glu Asp Gly Asn Ile
        115                 120                 125

Leu Gly His Lys Leu Glu Tyr Asn Tyr Phe Ser Asp Asn Val Tyr Ile
    130                 135                 140

Thr Ala Asp Lys Gln Lys Asn Gly Ile Lys Ala Asn Phe Lys Ile Arg
145                 150                 155                 160

His Asn Ile Glu Asp Gly Gly Val Gln Leu Ala Asp His Tyr Gln Gln
                165                 170                 175

Asn Thr Pro Ile Gly Asp Gly Pro Val Leu Leu Pro Asp Asn His Tyr
            180                 185                 190

Leu Ser Thr Gln Ser Lys Leu Ser Lys Asp Pro Asn Glu Lys Arg Asp
        195                 200                 205

His Met Val Leu Leu Glu Phe Val Thr Ala Ala Gly Ile Thr Leu Gly
    210                 215                 220

Met Asp Glu Leu Tyr Lys Gly Gly Gly Ser Ser Lys Gly Glu Glu Leu
225                 230                 235                 240

Phe

<210>  8
<211>  352
<212>  PRT
<213>  Artificial Sequence

<220>
<223>  cpmTurquoise2-Actin-binding affimer

<400>  8

Met Gly Val Val Pro Ile Leu Val Glu Leu Asp Gly Asp Val Asn Gly
1               5                   10                  15

His Lys Phe Ser Val Ser Gly Glu Gly Glu Gly Asp Ala Thr Tyr Gly
            20                  25                  30

Lys Leu Thr Leu Lys Phe Ile Cys Thr Thr Gly Lys Leu Pro Val Pro

35                  40                  45

Trp Pro Thr Leu Val Thr Thr Leu Ser Trp Gly Val Gln Cys Phe Ala
    50                  55                  60

Arg Tyr Pro Asp His Met Lys Gln His Asp Phe Phe Lys Ser Ala Met
65                  70                  75                  80

Pro Glu Gly Tyr Val Gln Glu Arg Thr Ile Phe Phe Lys Asp Asp Gly
                85                  90                  95

Asn Tyr Lys Thr Arg Ala Glu Val Lys Phe Glu Gly Asp Thr Leu Val
            100                 105                 110

Asn Arg Ile Glu Leu Lys Gly Ile Asp Phe Lys Glu Asp Gly Asn Ile
        115                 120                 125

Leu Gly His Lys Leu Glu Tyr Asn Tyr Phe Ser Asp Asn Val Tyr Ile
    130                 135                 140

Thr Ala Asp Lys Gln Lys Asn Gly Ile Lys Ala Asn Phe Lys Ile Arg
145                 150                 155                 160

His Asn Ile Glu Asp Gly Gly Val Gln Leu Ala Asp His Tyr Gln Gln
                165                 170                 175

Asn Thr Pro Ile Gly Asp Gly Pro Val Leu Leu Pro Asp Asn His Tyr
            180                 185                 190

Leu Ser Thr Gln Ser Lys Leu Ser Lys Asp Pro Asn Glu Lys Arg Asp
        195                 200                 205

His Met Val Leu Leu Glu Phe Val Thr Ala Ala Gly Ile Thr Leu Gly
    210                 215                 220

Met Asp Glu Leu Tyr Lys Gly Gly Gly Ser Ser Lys Gly Glu Glu Leu
225                 230                 235                 240

Phe Ala Ser Leu Glu Ile Glu Glu Leu Ala Arg Phe Ala Val Asp Glu
                245                 250                 255

His Asn Lys Lys Glu Asn Ala Leu Leu Glu Phe Val Arg Val Val Lys
            260                 265                 270

Ala Lys Glu Gln Ser Ser Val Pro His Trp Trp Trp Thr Thr Met Tyr
        275                 280                 285

Tyr Leu Thr Leu Glu Ala Lys Asp Gly Gly Lys Lys Lys Leu Tyr Glu
    290                 295                 300

Ala Lys Val Trp Val Lys Arg Asp Pro Asn Met Ile Phe Lys Ile Asn
305                 310                 315                 320

Phe Lys Glu Leu Gln Glu Phe Lys Pro Val Gly Asp Ala Gly Gly Ser
                325                 330                 335

Gly Gly Gly Ser Gly Gly Tyr Pro Tyr Asp Val Pro Asp Tyr Ala Ser
            340                 345                 350

<210>  9
<211>  290
<212>  PRT
<213>  Artificial Sequence

<220>
<223>  Halotag

<400>  9

Met Ala Glu Ile Gly Thr Gly Phe Pro Phe Asp Pro His Tyr Val Glu
1               5                   10                  15

Val Leu Gly Glu Arg Met His Tyr Val Asp Val Gly Pro Arg Asp Gly
            20                  25                  30

Thr Pro Val Leu Phe Leu His Gly Asn Pro Thr Ser Ser Tyr Val Trp
        35                  40                  45

Arg Asn Ile Ile Pro His Val Ala Pro Thr His Arg Cys Ile Ala Pro
    50                  55                  60

Asp Leu Ile Gly Met Gly Lys Ser Asp Lys Pro Asp Leu Gly Tyr Phe
65                  70                  75                  80

Phe Asp Asp His Val Arg Phe Met Asp Ala Phe Ile Glu Ala Leu Gly
                85                  90                  95

Leu Glu Glu Val Val Leu Val Ile His Asp Trp Gly Ser Ala Leu Gly
            100                 105                 110

Phe His Trp Ala Lys Arg Asn Pro Glu Arg Val Lys Gly Ile Ala Phe
        115                 120                 125

Met Glu Phe Ile Arg Pro Ile Pro Thr Trp Asp Glu Trp Pro Glu Phe
    130                 135                 140

Ala Arg Glu Thr Phe Gln Ala Phe Arg Thr Thr Asp Val Gly Arg Lys

145                 150                 155                 160

Leu Ile Ile Asp Gln Asn Val Phe Ile Glu Gly Thr Leu Pro Met Gly
                165                 170                 175

Val Val Arg Pro Leu Thr Glu Val Glu Met Asp His Tyr Arg Glu Pro
            180                 185                 190

Phe Leu Asn Pro Val Asp Arg Glu Pro Leu Trp Arg Phe Pro Asn Glu
        195                 200                 205

Leu Pro Ile Ala Gly Glu Pro Ala Asn Ile Val Ala Leu Val Glu Glu
    210                 215                 220

Tyr Met Asp Trp Leu His Gln Ser Pro Val Pro Lys Leu Leu Phe Trp
225                 230                 235                 240

Gly Thr Pro Gly Val Leu Ile Pro Pro Ala Glu Ala Ala Arg Leu Ala
                245                 250                 255

Lys Ser Leu Pro Asn Cys Lys Ala Val Asp Ile Gly Pro Gly Leu Asn
            260                 265                 270

Leu Leu Gln Glu Asp Asn Pro Asp Leu Ile Gly Ser Glu Ile Ala Arg
        275                 280                 285

Trp Leu
    290

<210>  10
<211>  4
<212>  PRT
<213>  Artificial Sequence

<220>
<223>  helix linker 2

<400>  10

Glu Ala Ala Ala
1

<210>  11
<211>  385
<212>  PRT
<213>  Artificial Sequence

<220>
<223>  Halotag-Actin-binding affimer

<400>  11

Met Ala Glu Ile Gly Thr Gly Phe Pro Phe Asp Pro His Tyr Val Glu
1               5                   10                  15

Val Leu Gly Glu Arg Met His Tyr Val Asp Val Gly Pro Arg Asp Gly
            20                  25                  30

Thr Pro Val Leu Phe Leu His Gly Asn Pro Thr Ser Ser Tyr Val Trp
        35                  40                  45

Arg Asn Ile Ile Pro His Val Ala Pro Thr His Arg Cys Ile Ala Pro
    50                  55                  60

Asp Leu Ile Gly Met Gly Lys Ser Asp Lys Pro Asp Leu Gly Tyr Phe
65                  70                  75                  80

Phe Asp Asp His Val Arg Phe Met Asp Ala Phe Ile Glu Ala Leu Gly
                85                  90                  95

Leu Glu Glu Val Val Leu Val Ile His Asp Trp Gly Ser Ala Leu Gly
            100                 105                 110

Phe His Trp Ala Lys Arg Asn Pro Glu Arg Val Lys Gly Ile Ala Phe
        115                 120                 125

Met Glu Phe Ile Arg Pro Ile Pro Thr Trp Asp Glu Trp Pro Glu Phe
    130                 135                 140

Ala Arg Glu Thr Phe Gln Ala Phe Arg Thr Thr Asp Val Gly Arg Lys
145                 150                 155                 160

Leu Ile Ile Asp Gln Asn Val Phe Ile Glu Gly Thr Leu Pro Met Gly
                165                 170                 175

Val Val Arg Pro Leu Thr Glu Val Glu Met Asp His Tyr Arg Glu Pro
            180                 185                 190

Phe Leu Asn Pro Val Asp Arg Glu Pro Leu Trp Arg Phe Pro Asn Glu
        195                 200                 205

Leu Pro Ile Ala Gly Glu Pro Ala Asn Ile Val Ala Leu Val Glu Glu
    210                 215                 220

Tyr Met Asp Trp Leu His Gln Ser Pro Val Pro Lys Leu Leu Phe Trp
225                 230                 235                 240

Gly Thr Pro Gly Val Leu Ile Pro Pro Ala Glu Ala Ala Arg Leu Ala
                245                 250                 255

Lys Ser Leu Pro Asn Cys Lys Ala Val Asp Ile Gly Pro Gly Leu Asn
            260                 265                 270

Leu Leu Gln Glu Asp Asn Pro Asp Leu Ile Gly Ser Glu Ile Ala Arg
        275                 280                 285

Trp Leu Glu Ala Ala Ala Ser Leu Glu Ile Glu Glu Leu Ala Arg Phe
    290                 295                 300

Ala Val Asp Glu His Asn Lys Lys Glu Asn Ala Leu Leu Glu Phe Val
305                 310                 315                 320

Arg Val Val Lys Ala Lys Glu Gln Ser Ser Val Pro His Trp Trp Trp
                325                 330                 335

Thr Thr Met Tyr Tyr Leu Thr Leu Glu Ala Lys Asp Gly Gly Lys Lys
            340                 345                 350

Lys Leu Tyr Glu Ala Lys Val Trp Val Lys Arg Asp Pro Asn Met Ile
        355                 360                 365

Phe Lys Ile Asn Phe Lys Glu Leu Gln Glu Phe Lys Pro Val Gly Asp
    370                 375                 380

Ala
385

<210> 12
<211> 3
<212> PRT
<213> Artificial Sequence

<220>
<223> Helix linker 3

<400> 12

Glu Ala Ala
1

<210> 13
<211> 384
<212> PRT
<213> Artificial Sequence

<220>
<223> Halotag-Affmer rotation 1

<400> 13

Met Ala Glu Ile Gly Thr Gly Phe Pro Phe Asp Pro His Tyr Val Glu
1               5                   10                  15

Val Leu Gly Glu Arg Met His Tyr Val Asp Val Gly Pro Arg Asp Gly
            20                  25                  30

Thr Pro Val Leu Phe Leu His Gly Asn Pro Thr Ser Ser Tyr Val Trp
        35                  40                  45

Arg Asn Ile Ile Pro His Val Ala Pro Thr His Arg Cys Ile Ala Pro
    50                  55                  60

Asp Leu Ile Gly Met Gly Lys Ser Asp Lys Pro Asp Leu Gly Tyr Phe
65                  70                  75                  80

Phe Asp Asp His Val Arg Phe Met Asp Ala Phe Ile Glu Ala Leu Gly
                85                  90                  95

Leu Glu Glu Val Val Leu Val Ile His Asp Trp Gly Ser Ala Leu Gly
            100                 105                 110

Phe His Trp Ala Lys Arg Asn Pro Glu Arg Val Lys Gly Ile Ala Phe
        115                 120                 125

Met Glu Phe Ile Arg Pro Ile Pro Thr Trp Asp Glu Trp Pro Glu Phe
    130                 135                 140

Ala Arg Glu Thr Phe Gln Ala Phe Arg Thr Thr Asp Val Gly Arg Lys
145                 150                 155                 160

Leu Ile Ile Asp Gln Asn Val Phe Ile Glu Gly Thr Leu Pro Met Gly
                165                 170                 175

Val Val Arg Pro Leu Thr Glu Val Glu Met Asp His Tyr Arg Glu Pro
            180                 185                 190

Phe Leu Asn Pro Val Asp Arg Glu Pro Leu Trp Arg Phe Pro Asn Glu
        195                 200                 205

Leu Pro Ile Ala Gly Glu Pro Ala Asn Ile Val Ala Leu Val Glu Glu
    210                 215                 220

Tyr Met Asp Trp Leu His Gln Ser Pro Val Pro Lys Leu Leu Phe Trp
225                 230                 235                 240

Gly Thr Pro Gly Val Leu Ile Pro Pro Ala Glu Ala Ala Arg Leu Ala
                245                 250                 255

Lys Ser Leu Pro Asn Cys Lys Ala Val Asp Ile Gly Pro Gly Leu Asn

            260                 265                 270

Leu Leu Gln Glu Asp Asn Pro Asp Leu Ile Gly Ser Glu Ile Ala Arg
        275                 280                 285

Trp Leu Glu Ala Ala Ser Leu Glu Ile Glu Glu Leu Ala Arg Phe Ala
    290                 295                 300

Val Asp Glu His Asn Lys Lys Glu Asn Ala Leu Leu Glu Phe Val Arg
305                 310                 315                 320

Val Val Lys Ala Lys Glu Gln Ser Ser Val Pro His Trp Trp Trp Thr
                325                 330                 335

Thr Met Tyr Tyr Leu Thr Leu Glu Ala Lys Asp Gly Gly Lys Lys Lys
            340                 345                 350

Leu Tyr Glu Ala Lys Val Trp Val Lys Arg Asp Pro Asn Met Ile Phe
        355                 360                 365

Lys Ile Asn Phe Lys Glu Leu Gln Glu Phe Lys Pro Val Gly Asp Ala
    370                 375                 380

<210> 14
<211> 2
<212> PRT
<213> Artificial Sequence

<220>
<223> Helix linker 4

<400> 14

Glu Ala
1

<210> 15
<211> 383
<212> PRT
<213> Artificial Sequence

<220>
<223> Halotag-Affimer rotation 2

<400> 15

Met Ala Glu Ile Gly Thr Gly Phe Pro Phe Asp Pro His Tyr Val Glu
1               5                   10                  15

Val Leu Gly Glu Arg Met His Tyr Val Asp Val Gly Pro Arg Asp Gly
            20                  25                  30

Thr Pro Val Leu Phe Leu His Gly Asn Pro Thr Ser Ser Tyr Val Trp
        35                  40                  45

Arg Asn Ile Ile Pro His Val Ala Pro Thr His Arg Cys Ile Ala Pro
    50                  55                  60

Asp Leu Ile Gly Met Gly Lys Ser Asp Lys Pro Asp Leu Gly Tyr Phe
65                  70                  75                  80

Phe Asp Asp His Val Arg Phe Met Asp Ala Phe Ile Glu Ala Leu Gly
                85                  90                  95

Leu Glu Glu Val Val Leu Val Ile His Asp Trp Gly Ser Ala Leu Gly
            100                 105                 110

Phe His Trp Ala Lys Arg Asn Pro Glu Arg Val Lys Gly Ile Ala Phe
        115                 120                 125

Met Glu Phe Ile Arg Pro Ile Pro Thr Trp Asp Glu Trp Pro Glu Phe
    130                 135                 140

Ala Arg Glu Thr Phe Gln Ala Phe Arg Thr Thr Asp Val Gly Arg Lys
145                 150                 155                 160

Leu Ile Ile Asp Gln Asn Val Phe Ile Glu Gly Thr Leu Pro Met Gly
                165                 170                 175

Val Val Arg Pro Leu Thr Glu Val Glu Met Asp His Tyr Arg Glu Pro
            180                 185                 190

Phe Leu Asn Pro Val Asp Arg Glu Pro Leu Trp Arg Phe Pro Asn Glu
        195                 200                 205

Leu Pro Ile Ala Gly Glu Pro Ala Asn Ile Val Ala Leu Val Glu Glu
    210                 215                 220

Tyr Met Asp Trp Leu His Gln Ser Pro Val Pro Lys Leu Leu Phe Trp
225                 230                 235                 240

Gly Thr Pro Gly Val Leu Ile Pro Pro Ala Glu Ala Ala Arg Leu Ala
                245                 250                 255

Lys Ser Leu Pro Asn Cys Lys Ala Val Asp Ile Gly Pro Gly Leu Asn
            260                 265                 270

Leu Leu Gln Glu Asp Asn Pro Asp Leu Ile Gly Ser Glu Ile Ala Arg
        275                 280                 285

Trp Leu Glu Ala Ser Leu Glu Ile Glu Glu Leu Ala Arg Phe Ala Val
    290                 295                 300

Asp Glu His Asn Lys Lys Glu Asn Ala Leu Leu Glu Phe Val Arg Val
305                 310                 315                 320

Val Lys Ala Lys Glu Gln Ser Ser Val Pro His Trp Trp Trp Thr Thr
                325                 330                 335

Met Tyr Tyr Leu Thr Leu Glu Ala Lys Asp Gly Gly Lys Lys Lys Leu
            340                 345                 350

Tyr Glu Ala Lys Val Trp Val Lys Arg Asp Pro Asn Met Ile Phe Lys
        355                 360                 365

Ile Asn Phe Lys Glu Leu Gln Glu Phe Lys Pro Val Gly Asp Ala
    370                 375                 380

<210>  16
<211>  115
<212>  PRT
<213>  Artificial Sequence

<220>
<223>  Nanobody that binds to vimentin

<400>  16

Met Gln Val Gln Leu Val Glu Ser Gly Gly Gly Leu Val Gln Ser Gly
1               5                   10                  15

Gly Ser Leu Thr Leu Thr Cys Ala Ala Ser Gly Phe Thr Phe Ser Ala
            20                  25                  30

Ala Ser Met Arg Trp Val Arg Gln Val Pro Gly Lys Gly Leu Glu Trp
        35                  40                  45

Val Ala Thr Ile Asp Gly Thr Gly Ala Asn Ser Tyr Tyr Ser Glu Ser
    50                  55                  60

Ala Lys Gly Arg Phe Thr Ile Ser Arg Asp Asn Ala Arg Asn Thr Leu
65                  70                  75                  80

Arg Leu Gln Met Asn Asn Leu Lys Pro Asp Asp Thr Ala Val Tyr Tyr
                85                  90                  95

Cys Ala Asn Phe Gly Arg Asn Tyr Trp Gly Lys Gly Thr Gln Val Thr
            100                 105                 110

Val Ser Ser
        115

<210>  17
<211>  379
<212>  PRT
<213>  Artificial Sequence

<220>
<223>  Vimentin-binding nanobody-cpsfGFP

<400>  17

Met Arg Gly Ser His His His His His His Gly Met Ala Ser Met Thr
1               5                   10                  15

Gly Gly Gln Gln Met Gly Arg Gly Ser Glu Phe Met Gln Val Gln Leu
            20                  25                  30

Val Glu Ser Gly Gly Gly Leu Val Gln Ser Gly Gly Ser Leu Thr Leu
        35                  40                  45

Thr Cys Ala Ala Ser Gly Phe Thr Phe Ser Ala Ala Ser Met Arg Trp
    50                  55                  60

Val Arg Gln Val Pro Gly Lys Gly Leu Glu Trp Val Ala Thr Ile Asp
65                  70                  75                  80

Gly Thr Gly Ala Asn Ser Tyr Tyr Ser Glu Ser Ala Lys Gly Arg Phe
                85                  90                  95

Thr Ile Ser Arg Asp Asn Ala Arg Asn Thr Leu Arg Leu Gln Met Asn
            100                 105                 110

Asn Leu Lys Pro Asp Asp Thr Ala Val Tyr Tyr Cys Ala Asn Phe Gly
        115                 120                 125

Arg Asn Tyr Trp Gly Lys Gly Thr Gln Val Thr Val Val Val Pro Ile
    130                 135                 140

Leu Val Glu Leu Asp Gly Asp Val Asn Gly His Lys Phe Ser Val Arg
145                 150                 155                 160

Gly Glu Gly Glu Gly Asp Ala Thr Ile Gly Lys Leu Thr Leu Lys Phe
                165                 170                 175

Ile Cys Thr Thr Gly Lys Leu Pro Val Pro Trp Pro Thr Leu Val Thr
            180                 185                 190

Thr Leu Thr Tyr Gly Val Gln Cys Phe Ser Arg Tyr Pro Asp His Met

195                 200                 205

Lys Arg His Asp Phe Phe Lys Ser Ala Met Pro Glu Gly Tyr Val Gln
    210                 215                 220

Glu Arg Thr Ile Ser Phe Lys Asp Asp Gly Lys Tyr Lys Thr Arg Ala
225                 230                 235                 240

Val Val Lys Phe Glu Gly Asp Thr Leu Val Asn Arg Ile Glu Leu Lys
                245                 250                 255

Gly Thr Asp Phe Lys Glu Asp Gly Asn Ile Leu Gly His Lys Leu Glu
            260                 265                 270

Tyr Asn Phe Asn Ser His Asn Val Tyr Ile Thr Ala Asp Lys Gln Lys
        275                 280                 285

Asn Gly Ile Lys Ala Asn Phe Thr Val Arg His Asn Val Glu Asp Gly
    290                 295                 300

Ser Val Gln Leu Ala Asp His Tyr Gln Gln Asn Thr Pro Ile Gly Asp
305                 310                 315                 320

Gly Pro Val Leu Leu Pro Asp Asn His Tyr Leu Ser Thr Gln Thr Val
                325                 330                 335

Leu Ser Lys Asp Pro Asn Glu Lys Arg Asp His Met Val Leu His Glu
            340                 345                 350

Tyr Val Asn Ala Ala Gly Ile Thr Leu Gly Met Asp Glu Leu Tyr Lys
        355                 360                 365

Gly Gly Gly Ser Ser Lys Gly Glu Glu Leu Phe
    370                 375

<210>  18
<211>  236
<212>  PRT
<213>  Artificial Sequence

<220>
<223>  mApple

<400>  18

Met Val Ser Lys Gly Glu Glu Asn Asn Met Ala Ile Ile Lys Glu Phe
1               5                   10                  15

Met Arg Phe Lys Val His Met Glu Gly Ser Val Asn Gly His Glu Phe
            20                  25                  30

Glu Ile Glu Gly Glu Gly Glu Gly Arg Pro Tyr Glu Ala Phe Gln Thr
        35                  40                  45

Ala Lys Leu Lys Val Thr Lys Gly Gly Pro Leu Pro Phe Ala Trp Asp
    50                  55                  60

Ile Leu Ser Pro Gln Phe Met Tyr Gly Ser Lys Val Tyr Ile Lys His
65                  70                  75                  80

Pro Ala Asp Ile Pro Asp Tyr Phe Lys Leu Ser Phe Pro Glu Gly Phe
                85                  90                  95

Arg Trp Glu Arg Val Met Asn Phe Glu Asp Gly Gly Ile Ile His Val
            100                 105                 110

Asn Gln Asp Ser Ser Leu Gln Asp Gly Val Phe Ile Tyr Lys Val Lys
        115                 120                 125

Leu Arg Gly Thr Asn Phe Pro Ser Asp Gly Pro Val Met Gln Lys Lys
    130                 135                 140

Thr Met Gly Trp Glu Ala Ser Glu Glu Arg Met Tyr Pro Glu Asp Gly
145                 150                 155                 160

Ala Leu Lys Ser Glu Ile Lys Lys Arg Leu Lys Leu Lys Asp Gly Gly
                165                 170                 175

His Tyr Ala Ala Glu Val Lys Thr Thr Tyr Lys Ala Lys Lys Pro Val
            180                 185                 190

Gln Leu Pro Gly Ala Tyr Ile Val Asp Ile Lys Leu Asp Ile Val Ser
        195                 200                 205

His Asn Glu Asp Tyr Thr Ile Val Glu Gln Tyr Glu Arg Ala Glu Gly
    210                 215                 220

Arg His Ser Thr Gly Gly Met Asp Glu Leu Tyr Lys
225                 230                 235

<210>  19
<211>  367
<212>  PRT
<213>  Artificial Sequence

<220>
<223>  Vimentin-binding nanobody-mApple

<400>  19

Met Arg Gly Ser His His His His His His Gly Met Ala Ser Met Thr
1               5                   10                  15

Gly Gly Gln Gln Met Gly Arg Gly Ser Glu Phe Met Gln Val Gln Leu
            20                  25                  30

Val Glu Ser Gly Gly Gly Leu Val Gln Ser Gly Gly Ser Leu Thr Leu
        35                  40                  45

Thr Cys Ala Ala Ser Gly Phe Thr Phe Ser Ala Ala Ser Met Arg Trp
    50                  55                  60

Val Arg Gln Val Pro Gly Lys Gly Leu Glu Trp Val Ala Thr Ile Asp
65                  70                  75                  80

Gly Thr Gly Ala Asn Ser Tyr Tyr Ser Glu Ser Ala Lys Gly Arg Phe
                85                  90                  95

Thr Ile Ser Arg Asp Asn Ala Arg Asn Thr Leu Arg Leu Gln Met Asn
            100                 105                 110

Asn Leu Lys Pro Asp Asp Thr Ala Val Tyr Tyr Cys Ala Asn Phe Gly
        115                 120                 125

Arg Asn Tyr Trp Gly Lys Gly Thr Gln Val Thr Val Met Ala Ile Ile
    130                 135                 140

Lys Glu Phe Met Arg Phe Lys Val His Met Glu Gly Ser Val Asn Gly
145                 150                 155                 160

His Glu Phe Glu Ile Glu Gly Glu Gly Glu Gly Arg Pro Tyr Glu Ala
                165                 170                 175

Phe Gln Thr Ala Lys Leu Lys Val Thr Lys Gly Gly Pro Leu Pro Phe
            180                 185                 190

Ala Trp Asp Ile Leu Ser Pro Gln Phe Met Tyr Gly Ser Lys Val Tyr
        195                 200                 205

Ile Lys His Pro Ala Asp Ile Pro Asp Tyr Phe Lys Leu Ser Phe Pro
    210                 215                 220

Glu Gly Phe Arg Trp Glu Arg Val Met Asn Phe Glu Asp Gly Gly Ile
225                 230                 235                 240

Ile His Val Asn Gln Asp Ser Ser Leu Gln Asp Gly Val Phe Ile Tyr
                245                 250                 255

Lys Val Lys Leu Arg Gly Thr Asn Phe Pro Ser Asp Gly Pro Val Met
            260                 265                 270

Gln Lys Lys Thr Met Gly Trp Glu Ala Ser Glu Glu Arg Met Tyr Pro
        275                 280                 285

Glu Asp Gly Ala Leu Lys Ser Glu Ile Lys Lys Arg Leu Lys Leu Lys
    290                 295                 300

Asp Gly Gly His Tyr Ala Ala Glu Val Lys Thr Thr Tyr Lys Ala Lys
305                 310                 315                 320

Lys Pro Val Gln Leu Pro Gly Ala Tyr Ile Val Asp Ile Lys Leu Asp
                325                 330                 335

Ile Val Ser His Asn Glu Asp Tyr Thr Ile Val Glu Gln Tyr Glu Arg
            340                 345                 350

Ala Glu Gly Arg His Ser Thr Gly Gly Met Asp Glu Leu Tyr Lys
        355                 360                 365

<210> 20
<211> 243
<212> PRT
<213> Artificial Sequence

<220>
<223> ScFV that binds to Non-muscle myosin IIA

<400> 20

Met Ala Glu Val Gln Leu Val Glu Ser Gly Gly Gly Leu Val Gln Pro
1               5                   10                  15

Gly Gly Ser Leu Arg Leu Ser Cys Ala Ala Ser Gly Phe Thr Phe Ser
            20                  25                  30

Ser Tyr Ala Met Ser Trp Val Arg Gln Ala Pro Gly Lys Gly Leu Glu
        35                  40                  45

Trp Val Ser Ala Ile Ser Gly Ser Gly Gly Ser Thr Tyr Tyr Ala Asp
    50                  55                  60

Ser Val Lys Gly Arg Phe Thr Ile Ser Arg Asp Asn Ser Lys Asn Thr
65                  70                  75                  80

Leu Tyr Leu Gln Met Asn Ser Leu Arg Ala Glu Asp Thr Ala Val Tyr
                85                  90                  95

Tyr Cys Ala Arg Gly Leu Phe Ser Trp Leu Leu Trp Gly Gln Gly Thr
            100                 105                 110

Leu Val Thr Val Ser Ser Gly Gly Gly Gly Ser Gly Gly Gly Gly Ser
        115                 120                 125

Gly Gly Ser Ala Leu Gln Ser Val Leu Thr Gln Pro Pro Ser Ala Ser
    130                 135                 140

Gly Thr Pro Gly Gln Arg Val Thr Ile Ser Cys Ser Gly Ser Ser Ser
145                 150                 155                 160

Asn Ile Gly Ser Asn Tyr Val Tyr Trp Tyr Gln Gln Leu Pro Gly Thr
                165                 170                 175

Ala Pro Lys Leu Leu Ile Tyr Arg Asn Asn Gln Arg Pro Ser Gly Val
            180                 185                 190

Pro Asp Arg Phe Ser Gly Ser Lys Ser Gly Thr Ser Ala Ser Leu Ala
        195                 200                 205

Ile Ser Gly Leu Arg Ser Glu Asp Glu Ala Asp Tyr Tyr Cys Ala Ala
    210                 215                 220

Trp Asp Asp Ser Leu Leu Ala Leu Val Phe Gly Gly Gly Thr Lys Leu
225                 230                 235                 240

Thr Val Leu

<210>  21
<211>  508
<212>  PRT
<213>  Artificial Sequence

<220>
<223>  Non-muscle myosin IIA binding scFv-cpsfGFP

<400>  21

Met Arg Gly Ser His His His His His His Gly Met Ala Ser Met Thr
1               5                   10                  15

Gly Gly Gln Gln Met Gly Arg Gly Ser Glu Phe Met Ala Glu Val Gln
            20                  25                  30

Leu Val Glu Ser Gly Gly Gly Leu Val Gln Pro Gly Gly Ser Leu Arg
        35                  40                  45

Leu Ser Cys Ala Ala Ser Gly Phe Thr Phe Ser Ser Tyr Ala Met Ser
    50                  55                  60

Trp Val Arg Gln Ala Pro Gly Lys Gly Leu Glu Trp Val Ser Ala Ile
65                  70                  75                  80

Ser Gly Ser Gly Gly Ser Thr Tyr Tyr Ala Asp Ser Val Lys Gly Arg
                85                  90                  95

Phe Thr Ile Ser Arg Asp Asn Ser Lys Asn Thr Leu Tyr Leu Gln Met
            100                 105                 110

Asn Ser Leu Arg Ala Glu Asp Thr Ala Val Tyr Tyr Cys Ala Arg Gly
        115                 120                 125

Leu Phe Ser Trp Leu Leu Trp Gly Gln Gly Thr Leu Val Thr Val Ser
    130                 135                 140

Ser Gly Gly Gly Gly Ser Gly Gly Gly Gly Ser Gly Gly Ser Ala Leu
145                 150                 155                 160

Gln Ser Val Leu Thr Gln Pro Pro Ser Ala Ser Gly Thr Pro Gly Gln
                165                 170                 175

Arg Val Thr Ile Ser Cys Ser Gly Ser Ser Ser Asn Ile Gly Ser Asn
            180                 185                 190

Tyr Val Tyr Trp Tyr Gln Gln Leu Pro Gly Thr Ala Pro Lys Leu Leu
        195                 200                 205

Ile Tyr Arg Asn Asn Gln Arg Pro Ser Gly Val Pro Asp Arg Phe Ser
    210                 215                 220

Gly Ser Lys Ser Gly Thr Ser Ala Ser Leu Ala Ile Ser Gly Leu Arg
225                 230                 235                 240

Ser Glu Asp Glu Ala Asp Tyr Tyr Cys Ala Ala Trp Asp Asp Ser Leu
                245                 250                 255

Leu Ala Leu Val Phe Gly Gly Gly Thr Lys Leu Thr Val Val Val Pro
            260                 265                 270

Ile Leu Val Glu Leu Asp Gly Asp Val Asn Gly His Lys Phe Ser Val
        275                 280                 285

Arg Gly Glu Gly Glu Gly Asp Ala Thr Ile Gly Lys Leu Thr Leu Lys
    290                 295                 300

Phe Ile Cys Thr Thr Gly Lys Leu Pro Val Pro Trp Pro Thr Leu Val
305                 310                 315                 320

Thr Thr Leu Thr Tyr Gly Val Gln Cys Phe Ser Arg Tyr Pro Asp His
                325                 330                 335

Met Lys Arg His Asp Phe Phe Lys Ser Ala Met Pro Glu Gly Tyr Val
            340                 345                 350

Gln Glu Arg Thr Ile Ser Phe Lys Asp Asp Gly Lys Tyr Lys Thr Arg
        355                 360                 365

Ala Val Val Lys Phe Glu Gly Asp Thr Leu Val Asn Arg Ile Glu Leu
    370                 375                 380

Lys Gly Thr Asp Phe Lys Glu Asp Gly Asn Ile Leu Gly His Lys Leu
385                 390                 395                 400

Glu Tyr Asn Phe Asn Ser His Asn Val Tyr Ile Thr Ala Asp Lys Gln
                405                 410                 415

Lys Asn Gly Ile Lys Ala Asn Phe Thr Val Arg His Asn Val Glu Asp
            420                 425                 430

Gly Ser Val Gln Leu Ala Asp His Tyr Gln Gln Asn Thr Pro Ile Gly
        435                 440                 445

Asp Gly Pro Val Leu Leu Pro Asp Asn His Tyr Leu Ser Thr Gln Thr
    450                 455                 460

Val Leu Ser Lys Asp Pro Asn Glu Lys Arg Asp His Met Val Leu His
465                 470                 475                 480

Glu Tyr Val Asn Ala Ala Gly Ile Thr Leu Gly Met Asp Glu Leu Tyr
                485                 490                 495

Lys Gly Gly Gly Ser Ser Lys Gly Glu Glu Leu Phe
            500                 505

<210>  22
<211>  315
<212>  PRT
<213>  Artificial Sequence

<220>
<223>  miRFP670

<400>  22

Met Val Ala Gly His Ala Ser Gly Ser Pro Ala Phe Gly Thr Ala Ser
1               5                   10                  15

His Ser Asn Cys Glu His Glu Glu Ile His Leu Ala Gly Ser Ile Gln
            20                  25                  30

Pro His Gly Ala Leu Leu Val Val Ser Glu His Asp His Arg Val Ile
        35                  40                  45

Gln Ala Ser Ala Asn Ala Ala Glu Phe Leu Asn Leu Gly Ser Val Leu
    50                  55                  60

Gly Val Pro Leu Ala Glu Ile Asp Gly Asp Leu Leu Ile Lys Ile Leu
65                  70                  75                  80

Pro His Leu Asp Pro Thr Ala Glu Gly Met Pro Val Ala Val Arg Cys
                85                  90                  95

Arg Ile Gly Asn Pro Ser Thr Glu Tyr Cys Gly Leu Met His Arg Pro
            100                 105                 110

Pro Glu Gly Gly Leu Ile Ile Glu Leu Glu Arg Ala Gly Pro Ser Ile
        115                 120                 125

Asp Leu Ser Gly Thr Leu Ala Pro Ala Leu Glu Arg Ile Arg Thr Ala
    130                 135                 140

Gly Ser Leu Arg Ala Leu Cys Asp Asp Thr Val Leu Leu Phe Gln Gln
145                 150                 155                 160

Cys Thr Gly Tyr Asp Arg Val Met Val Tyr Arg Phe Asp Glu Gln Gly
                165                 170                 175

His Gly Leu Val Phe Ser Glu Cys His Val Pro Gly Leu Glu Ser Tyr
            180                 185                 190

Phe Gly Asn Arg Tyr Pro Ser Ser Thr Val Pro Gln Met Ala Arg Gln
        195                 200                 205

Leu Tyr Val Arg Gln Arg Val Arg Val Leu Val Asp Val Thr Tyr Gln
    210                 215                 220

Pro Val Pro Leu Glu Pro Arg Leu Ser Pro Leu Thr Gly Arg Asp Leu
225                 230                 235                 240

Asp Met Ser Gly Cys Phe Leu Arg Ser Met Ser Pro Cys His Leu Gln
                245                 250                 255

Phe Leu Lys Asp Met Gly Val Arg Ala Thr Leu Ala Val Ser Leu Val
            260                 265                 270

Val Gly Gly Lys Leu Trp Gly Leu Val Val Cys His His Tyr Leu Pro
        275                 280                 285

Arg Phe Ile Arg Phe Glu Leu Arg Ala Ile Cys Lys Arg Leu Ala Glu
    290                 295                 300

Arg Ile Ala Thr Arg Ile Thr Ala Leu Glu Ser
305                 310                 315

<210>  23
<211>  5
<212>  PRT
<213>  Artificial Sequence

<220>
<223>  Helix liner 5

<400>  23

Glu Ala Ala Ala Ala
1               5

<210>  24
<211>  411
<212>  PRT
<213>  Artificial Sequence

<220>
<223>  miRFP670-affimer

<400>  24

Met Val Ala Gly His Ala Ser Gly Ser Pro Ala Phe Gly Thr Ala Ser
1               5                   10                  15

His Ser Asn Cys Glu His Glu Glu Ile His Leu Ala Gly Ser Ile Gln
            20                  25                  30

Pro His Gly Ala Leu Leu Val Val Ser Glu His Asp His Arg Val Ile
        35                  40                  45

Gln Ala Ser Ala Asn Ala Ala Glu Phe Leu Asn Leu Gly Ser Val Leu
    50                  55                  60

Gly Val Pro Leu Ala Glu Ile Asp Gly Asp Leu Leu Ile Lys Ile Leu
65                  70                  75                  80

Pro His Leu Asp Pro Thr Ala Glu Gly Met Pro Val Ala Val Arg Cys
                85                  90                  95

Arg Ile Gly Asn Pro Ser Thr Glu Tyr Cys Gly Leu Met His Arg Pro
            100                 105                 110

Pro Glu Gly Gly Leu Ile Ile Glu Leu Glu Arg Ala Gly Pro Ser Ile
        115                 120                 125

Asp Leu Ser Gly Thr Leu Ala Pro Ala Leu Glu Arg Ile Arg Thr Ala
    130                 135                 140

Gly Ser Leu Arg Ala Leu Cys Asp Asp Thr Val Leu Leu Phe Gln Gln
145                 150                 155                 160

Cys Thr Gly Tyr Asp Arg Val Met Val Tyr Arg Phe Asp Glu Gln Gly
                165                 170                 175

His Gly Leu Val Phe Ser Glu Cys His Val Pro Gly Leu Glu Ser Tyr
            180                 185                 190

Phe Gly Asn Arg Tyr Pro Ser Ser Thr Val Pro Gln Met Ala Arg Gln
        195                 200                 205

Leu Tyr Val Arg Gln Arg Val Arg Val Leu Val Asp Val Thr Tyr Gln
    210                 215                 220

Pro Val Pro Leu Glu Pro Arg Leu Ser Pro Leu Thr Gly Arg Asp Leu
225                 230                 235                 240

Asp Met Ser Gly Cys Phe Leu Arg Ser Met Ser Pro Cys His Leu Gln
                245                 250                 255

Phe Leu Lys Asp Met Gly Val Arg Ala Thr Leu Ala Val Ser Leu Val
            260                 265                 270

Val Gly Gly Lys Leu Trp Gly Leu Val Val Cys His His Tyr Leu Pro
        275                 280                 285

Arg Phe Ile Arg Phe Glu Leu Arg Ala Ile Cys Lys Arg Leu Ala Glu
    290                 295                 300

Arg Ile Ala Thr Arg Ile Thr Ala Leu Glu Ser Glu Ala Ala Ala Ala
305                 310                 315                 320

Ser Leu Glu Ile Glu Glu Leu Ala Arg Phe Ala Val Asp Glu His Asn
                325                 330                 335

Lys Lys Glu Asn Ala Leu Leu Glu Phe Val Arg Val Val Lys Ala Lys

340                 345                 350

Glu Gln Ser Ser Val Pro His Trp Trp Trp Thr Thr Met Tyr Tyr Leu
        355                 360                 365

Thr Leu Glu Ala Lys Asp Gly Gly Lys Lys Lys Leu Tyr Glu Ala Lys
    370                 375                 380

Val Trp Val Lys Arg Asp Pro Asn Met Ile Phe Lys Ile Asn Phe Lys
385                 390                 395                 400

Glu Leu Gln Glu Phe Lys Pro Val Gly Asp Ala
                405                 410

<210>  25
<211>  379
<212>  PRT
<213>  Artificial Sequence

<220>
<223>  Nanobody-cpmTurquoise2

<400>  25

Met Arg Gly Ser His His His His His His Gly Met Ala Ser Met Thr
1               5                   10                  15

Gly Gly Gln Gln Met Gly Arg Gly Ser Glu Phe Met Gln Val Gln Leu
            20                  25                  30

Val Glu Ser Gly Gly Gly Leu Val Gln Ser Gly Gly Ser Leu Thr Leu
        35                  40                  45

Thr Cys Ala Ala Ser Gly Phe Thr Phe Ser Ala Ala Ser Met Arg Trp
    50                  55                  60

Val Arg Gln Val Pro Gly Lys Gly Leu Glu Trp Val Ala Thr Ile Asp
65                  70                  75                  80

Gly Thr Gly Ala Asn Ser Tyr Tyr Ser Glu Ser Ala Lys Gly Arg Phe
                85                  90                  95

Thr Ile Ser Arg Asp Asn Ala Arg Asn Thr Leu Arg Leu Gln Met Asn
            100                 105                 110

Asn Leu Lys Pro Asp Asp Thr Ala Val Tyr Tyr Cys Ala Asn Phe Gly
        115                 120                 125

Arg Asn Tyr Trp Gly Lys Gly Thr Gln Val Thr Val Val Pro Ile
    130                 135                 140

Leu Val Glu Leu Asp Gly Asp Val Asn Gly His Lys Phe Ser Val Ser
145                 150                 155                 160

Gly Glu Gly Glu Gly Asp Ala Thr Tyr Gly Lys Leu Thr Leu Lys Phe
                165                 170                 175

Ile Cys Thr Thr Gly Lys Leu Pro Val Pro Trp Pro Thr Leu Val Thr
            180                 185                 190

Thr Leu Ser Trp Gly Val Gln Cys Phe Ala Arg Tyr Pro Asp His Met
        195                 200                 205

Lys Gln His Asp Phe Phe Lys Ser Ala Met Pro Glu Gly Tyr Val Gln
    210                 215                 220

Glu Arg Thr Ile Phe Phe Lys Asp Asp Gly Asn Tyr Lys Thr Arg Ala
225                 230                 235                 240

Glu Val Lys Phe Glu Gly Asp Thr Leu Val Asn Arg Ile Glu Leu Lys
                245                 250                 255

Gly Ile Asp Phe Lys Glu Asp Gly Asn Ile Leu Gly His Lys Leu Glu
            260                 265                 270

Tyr Asn Tyr Phe Ser Asp Asn Val Tyr Ile Thr Ala Asp Lys Gln Lys
        275                 280                 285

Asn Gly Ile Lys Ala Asn Phe Lys Ile Arg His Asn Ile Glu Asp Gly
    290                 295                 300

Gly Val Gln Leu Ala Asp His Tyr Gln Gln Asn Thr Pro Ile Gly Asp
305                 310                 315                 320

Gly Pro Val Leu Leu Pro Asp Asn His Tyr Leu Ser Thr Gln Ser Lys
                325                 330                 335

Leu Ser Lys Asp Pro Asn Glu Lys Arg Asp His Met Val Leu Leu Glu
            340                 345                 350

Phe Val Thr Ala Ala Gly Ile Thr Leu Gly Met Asp Glu Leu Tyr Lys
        355                 360                 365

Gly Gly Gly Ser Ser Lys Gly Glu Glu Leu Phe
    370                 375

<210>  26

<211>  379
<212>  PRT
<213>  Artificial Sequence

<220>
<223>  Nanobody-cpmVenus

<400>  26

Met Arg Gly Ser His His His His His His Gly Met Ala Ser Met Thr
1               5                   10                  15

Gly Gly Gln Gln Met Gly Arg Gly Ser Glu Phe Met Gln Val Gln Leu
            20                  25                  30

Val Glu Ser Gly Gly Gly Leu Val Gln Ser Gly Gly Ser Leu Thr Leu
        35                  40                  45

Thr Cys Ala Ala Ser Gly Phe Thr Phe Ser Ala Ala Ser Met Arg Trp
    50                  55                  60

Val Arg Gln Val Pro Gly Lys Gly Leu Glu Trp Val Ala Thr Ile Asp
65                  70                  75                  80

Gly Thr Gly Ala Asn Ser Tyr Tyr Ser Glu Ser Ala Lys Gly Arg Phe
                85                  90                  95

Thr Ile Ser Arg Asp Asn Ala Arg Asn Thr Leu Arg Leu Gln Met Asn
            100                 105                 110

Asn Leu Lys Pro Asp Asp Thr Ala Val Tyr Tyr Cys Ala Asn Phe Gly
        115                 120                 125

Arg Asn Tyr Trp Gly Lys Gly Thr Gln Val Thr Val Val Val Pro Ile
    130                 135                 140

Leu Val Glu Leu Asp Gly Asp Val Asn Gly His Lys Phe Ser Val Ser
145                 150                 155                 160

Gly Glu Gly Glu Gly Asp Ala Thr Tyr Gly Lys Leu Thr Leu Lys Leu
                165                 170                 175

Ile Cys Thr Thr Gly Lys Leu Pro Val Pro Trp Pro Thr Leu Val Thr
            180                 185                 190

Thr Leu Gly Tyr Gly Leu Gln Cys Phe Ala Arg Tyr Pro Asp His Met
        195                 200                 205

Lys Gln His Asp Phe Phe Lys Ser Ala Met Pro Glu Gly Tyr Val Gln
    210                 215                 220

Glu Arg Thr Ile Phe Phe Lys Asp Asp Gly Asn Tyr Lys Thr Arg Ala
225                 230                 235                 240

Glu Val Lys Phe Glu Gly Asp Thr Leu Val Asn Arg Ile Glu Leu Lys
                245                 250                 255

Gly Ile Asp Phe Lys Glu Asp Gly Asn Ile Leu Gly His Lys Leu Glu
            260                 265                 270

Tyr Asn Tyr Asn Ser His Asn Val Tyr Ile Thr Ala Asp Lys Gln Lys
        275                 280                 285

Asn Gly Ile Lys Ala Asn Phe Lys Ile Arg His Asn Ile Glu Asp Gly
    290                 295                 300

Gly Val Gln Leu Ala Asp His Tyr Gln Gln Asn Thr Pro Ile Gly Asp
305                 310                 315                 320

Gly Pro Val Leu Leu Pro Asp Asn His Tyr Leu Ser Tyr Gln Ser Lys
                325                 330                 335

Leu Ser Lys Asp Pro Asn Glu Lys Arg Asp His Met Val Leu Leu Glu
            340                 345                 350

Phe Val Thr Ala Ala Gly Ile Thr Leu Gly Met Asp Glu Leu Tyr Lys
        355                 360                 365

Gly Gly Gly Ser Ser Lys Gly Glu Glu Leu Phe
    370                 375

<210>  27
<211>  508
<212>  PRT
<213>  Artificial Sequence

<220>
<223>  scFv-cpmTurquoise2

<400>  27

Met Arg Gly Ser His His His His His His Gly Met Ala Ser Met Thr
1               5                   10                  15

Gly Gly Gln Gln Met Gly Arg Gly Ser Glu Phe Met Ala Glu Val Gln
            20                  25                  30

Leu Val Glu Ser Gly Gly Gly Leu Val Gln Pro Gly Gly Ser Leu Arg
        35                  40                  45

Leu Ser Cys Ala Ala Ser Gly Phe Thr Phe Ser Ser Tyr Ala Met Ser
    50                  55                  60

Trp Val Arg Gln Ala Pro Gly Lys Gly Leu Glu Trp Val Ser Ala Ile
65                  70                  75                  80

Ser Gly Ser Gly Gly Ser Thr Tyr Tyr Ala Asp Ser Val Lys Gly Arg
                85                  90                  95

Phe Thr Ile Ser Arg Asp Asn Ser Lys Asn Thr Leu Tyr Leu Gln Met
            100                 105                 110

Asn Ser Leu Arg Ala Glu Asp Thr Ala Val Tyr Tyr Cys Ala Arg Gly
        115                 120                 125

Leu Phe Ser Trp Leu Leu Trp Gly Gln Gly Thr Leu Val Thr Val Ser
    130                 135                 140

Ser Gly Gly Gly Gly Ser Gly Gly Gly Gly Ser Gly Gly Ser Ala Leu
145                 150                 155                 160

Gln Ser Val Leu Thr Gln Pro Pro Ser Ala Ser Gly Thr Pro Gly Gln
                165                 170                 175

Arg Val Thr Ile Ser Cys Ser Gly Ser Ser Ser Asn Ile Gly Ser Asn
            180                 185                 190

Tyr Val Tyr Trp Tyr Gln Gln Leu Pro Gly Thr Ala Pro Lys Leu Leu
        195                 200                 205

Ile Tyr Arg Asn Asn Gln Arg Pro Ser Gly Val Pro Asp Arg Phe Ser
    210                 215                 220

Gly Ser Lys Ser Gly Thr Ser Ala Ser Leu Ala Ile Ser Gly Leu Arg
225                 230                 235                 240

Ser Glu Asp Glu Ala Asp Tyr Tyr Cys Ala Ala Trp Asp Asp Ser Leu
                245                 250                 255

Leu Ala Leu Val Phe Gly Gly Gly Thr Lys Leu Thr Val Val Val Pro
            260                 265                 270

Ile Leu Val Glu Leu Asp Gly Asp Val Asn Gly His Lys Phe Ser Val
        275                 280                 285

Ser Gly Glu Gly Glu Gly Asp Ala Thr Tyr Gly Lys Leu Thr Leu Lys
    290                 295                 300

Phe Ile Cys Thr Thr Gly Lys Leu Pro Val Pro Trp Pro Thr Leu Val
305                 310                 315                 320

Thr Thr Leu Ser Trp Gly Val Gln Cys Phe Ala Arg Tyr Pro Asp His
                325                 330                 335

Met Lys Gln His Asp Phe Phe Lys Ser Ala Met Pro Glu Gly Tyr Val
            340                 345                 350

Gln Glu Arg Thr Ile Phe Phe Lys Asp Asp Gly Asn Tyr Lys Thr Arg
        355                 360                 365

Ala Glu Val Lys Phe Glu Gly Asp Thr Leu Val Asn Arg Ile Glu Leu
    370                 375                 380

Lys Gly Ile Asp Phe Lys Glu Asp Gly Asn Ile Leu Gly His Lys Leu
385                 390                 395                 400

Glu Tyr Asn Tyr Phe Ser Asp Asn Val Tyr Ile Thr Ala Asp Lys Gln
                405                 410                 415

Lys Asn Gly Ile Lys Ala Asn Phe Lys Ile Arg His Asn Ile Glu Asp
            420                 425                 430

Gly Gly Val Gln Leu Ala Asp His Tyr Gln Gln Asn Thr Pro Ile Gly
        435                 440                 445

Asp Gly Pro Val Leu Leu Pro Asp Asn His Tyr Leu Ser Thr Gln Ser
    450                 455                 460

Lys Leu Ser Lys Asp Pro Asn Glu Lys Arg Asp His Met Val Leu Leu
465                 470                 475                 480

Glu Phe Val Thr Ala Ala Gly Ile Thr Leu Gly Met Asp Glu Leu Tyr
                485                 490                 495

Lys Gly Gly Gly Ser Ser Lys Gly Glu Glu Leu Phe
            500                 505

<210>  28
<211>  293
<212>  PRT
<213>  Artificial Sequence

<220>
<223>  Haloalkane dehalogenase

<400>  28

Met Ser Glu Ile Gly Thr Gly Phe Pro Phe Asp Pro His Tyr Val Glu
1               5                   10                  15

Val Leu Gly Glu Arg Met His Tyr Val Asp Val Gly Pro Arg Asp Gly
            20                  25                  30

Thr Pro Val Leu Phe Leu His Gly Asn Pro Thr Ser Ser Tyr Leu Trp
        35                  40                  45

Arg Asn Ile Ile Pro His Val Ala Pro Ser His Arg Cys Ile Ala Pro
    50                  55                  60

Asp Leu Ile Gly Met Gly Lys Ser Asp Lys Pro Asp Leu Asp Tyr Phe
65                  70                  75                  80

Phe Asp Asp His Val Arg Tyr Leu Asp Ala Phe Ile Glu Ala Leu Gly
                85                  90                  95

Leu Glu Glu Val Val Leu Val Ile His Asp Trp Gly Ser Ala Leu Gly
            100                 105                 110

Phe His Trp Ala Lys Arg Asn Pro Glu Arg Val Lys Gly Ile Ala Cys
        115                 120                 125

Met Glu Phe Ile Arg Pro Ile Pro Thr Trp Asp Glu Trp Pro Glu Phe
    130                 135                 140

Ala Arg Glu Thr Phe Gln Ala Phe Arg Thr Ala Asp Val Gly Arg Glu
145                 150                 155                 160

Leu Ile Ile Asp Gln Asn Ala Phe Ile Glu Gly Ala Leu Pro Lys Cys
                165                 170                 175

Val Val Arg Pro Leu Thr Glu Val Glu Met Asp His Tyr Arg Glu Pro
            180                 185                 190

Phe Leu Lys Pro Val Asp Arg Glu Pro Leu Trp Arg Phe Pro Asn Glu
        195                 200                 205

Leu Pro Ile Ala Gly Glu Pro Ala Asn Ile Val Ala Leu Val Glu Ala
    210                 215                 220

Tyr Met Asn Trp Leu His Gln Ser Pro Val Pro Lys Leu Leu Phe Trp
225                 230                 235                 240

Gly Thr Pro Gly Val Leu Ile Pro Pro Ala Glu Ala Ala Arg Leu Ala
                245                 250                 255

Glu Ser Leu Pro Asn Cys Lys Thr Val Asp Ile Gly Pro Gly Leu His
            260                 265                 270

Tyr Leu Gln Glu Asp Asn Pro Asp Leu Ile Gly Ser Glu Ile Ala Arg
        275                 280                 285

Trp Leu Pro Ala Leu
    290

<210>  29
<211>  293
<212>  PRT
<213>  Artificial Sequence

<220>
<223>  Haloalkane dehalogenase H272F

<400>  29

Met Ser Glu Ile Gly Thr Gly Phe Pro Phe Asp Pro His Tyr Val Glu
1               5                   10                  15

Val Leu Gly Glu Arg Met His Tyr Val Asp Val Gly Pro Arg Asp Gly
            20                  25                  30

Thr Pro Val Leu Phe Leu His Gly Asn Pro Thr Ser Ser Tyr Leu Trp
        35                  40                  45

Arg Asn Ile Ile Pro His Val Ala Pro Ser His Arg Cys Ile Ala Pro
    50                  55                  60

Asp Leu Ile Gly Met Gly Lys Ser Asp Lys Pro Asp Leu Asp Tyr Phe
65                  70                  75                  80

Phe Asp Asp His Val Arg Tyr Leu Asp Ala Phe Ile Glu Ala Leu Gly
                85                  90                  95

Leu Glu Glu Val Val Leu Val Ile His Asp Trp Gly Ser Ala Leu Gly
            100                 105                 110

Phe His Trp Ala Lys Arg Asn Pro Glu Arg Val Lys Gly Ile Ala Cys
        115                 120                 125

Met Glu Phe Ile Arg Pro Ile Pro Thr Trp Asp Glu Trp Pro Glu Phe
    130                 135                 140

Ala Arg Glu Thr Phe Gln Ala Phe Arg Thr Ala Asp Val Gly Arg Glu
145                 150                 155                 160

Leu Ile Ile Asp Gln Asn Ala Phe Ile Glu Gly Ala Leu Pro Lys Cys
                165                 170                 175

Val Val Arg Pro Leu Thr Glu Val Glu Met Asp His Tyr Arg Glu Pro
            180                 185                 190

Phe Leu Lys Pro Val Asp Arg Glu Pro Leu Trp Arg Phe Pro Asn Glu
        195                 200                 205

Leu Pro Ile Ala Gly Glu Pro Ala Asn Ile Val Ala Leu Val Glu Ala
    210                 215                 220

Tyr Met Asn Trp Leu His Gln Ser Pro Val Pro Lys Leu Leu Phe Trp
225                 230                 235                 240

Gly Thr Pro Gly Val Leu Ile Pro Pro Ala Glu Ala Ala Arg Leu Ala
                245                 250                 255

Glu Ser Leu Pro Asn Cys Lys Thr Val Asp Ile Gly Pro Gly Leu Phe
            260                 265                 270

Tyr Leu Gln Glu Asp Asn Pro Asp Leu Ile Gly Ser Glu Ile Ala Arg
        275                 280                 285

Trp Leu Pro Ala Leu
    290

<210>  30
<211>  241
<212>  PRT
<213>  Artificial Sequence

<220>
<223>  cp176-172mVenus

<400>  30

Val Gln Leu Ala Asp His Tyr Gln Gln Asn Thr Pro Ile Gly Asp Gly
1               5                   10                  15

Pro Val Leu Leu Pro Asp Asn His Tyr Leu Ser Tyr Gln Ser Lys Leu
            20                  25                  30

Ser Lys Asp Pro Asn Glu Lys Arg Asp His Met Val Leu Leu Glu Phe
        35                  40                  45

Val Thr Ala Ala Gly Ile Thr Leu Gly Met Asp Glu Leu Tyr Lys Gly
    50                  55                  60

Gly Ser Gly Gly Met Val Ser Lys Gly Glu Glu Leu Phe Thr Gly Val
65                  70                  75                  80

Val Pro Ile Leu Val Glu Leu Asp Gly Asp Val Asn Gly His Lys Phe
                85                  90                  95

Ser Val Ser Gly Glu Gly Glu Gly Asp Ala Thr Tyr Gly Lys Leu Thr
            100                 105                 110

Leu Lys Leu Ile Cys Thr Thr Gly Lys Leu Pro Val Pro Trp Pro Thr
        115                 120                 125

Leu Val Thr Thr Leu Gly Tyr Gly Leu Gln Cys Phe Ala Arg Tyr Pro
    130                 135                 140

Asp His Met Lys Gln His Asp Phe Phe Lys Ser Ala Met Pro Glu Gly
145                 150                 155                 160

Tyr Val Gln Glu Arg Thr Ile Phe Phe Lys Asp Asp Gly Asn Tyr Lys
                165                 170                 175

Thr Arg Ala Glu Val Lys Phe Glu Gly Asp Thr Leu Val Asn Arg Ile
            180                 185                 190

Glu Leu Lys Gly Ile Asp Phe Lys Glu Asp Gly Asn Ile Leu Gly His
        195                 200                 205

Lys Leu Glu Tyr Asn Tyr Asn Ser His Asn Val Tyr Ile Thr Ala Asp
    210                 215                 220

Lys Gln Lys Asn Gly Ile Lys Ala Asn Phe Lys Ile Arg His Asn Ile
225                 230                 235                 240

Glu

<210>  31
<211>  383
<212>  PRT
<213>  Artificial Sequence

<220>
<223>  Nanobody-ValVal-cp176-172mVenus

<400>  31
   Met Arg Gly Ser His His His His His His Gly Met Ala Ser Met Thr
   1               5                   10                  15
   Gly Gly Gln Gln Met Gly Arg Gly Ser Glu Phe Met Gln Val Gln Leu
   20                  25                  30
   Val Glu Ser Gly Gly Gly Leu Val Gln Ser Gly Gly Ser Leu Thr Leu
           35                  40                  45
   Thr Cys Ala Ala Ser Gly Phe Thr Phe Ser Ala Ala Ser Met Arg Trp
       50                  55                  60
   Val Arg Gln Val Pro Gly Lys Gly Leu Glu Trp Val Ala Thr Ile Asp
   65                  70                  75                  80
   Gly Thr Gly Ala Asn Ser Tyr Tyr Ser Glu Ser Ala Lys Gly Arg Phe
                   85                  90                  95
   Thr Ile Ser Arg Asp Asn Ala Arg Asn Thr Leu Arg Leu Gln Met Asn
               100                 105                 110
   Asn Leu Lys Pro Asp Asp Thr Ala Val Tyr Tyr Cys Ala Asn Phe Gly
           115                 120                 125
   Arg Asn Tyr Trp Gly Lys Gly Thr Gln Val Thr Val Val Val Val Gln
       130                 135                 140
   Leu Ala Asp His Tyr Gln Gln Asn Thr Pro Ile Gly Asp Gly Pro Val
   145                 150                 155                 160
   Leu Leu Pro Asp Asn His Tyr Leu Ser Tyr Gln Ser Lys Leu Ser Lys
                   165                 170                 175
   Asp Pro Asn Glu Lys Arg Asp His Met Val Leu Leu Glu Phe Val Thr
               180                 185                 190
   Ala Ala Gly Ile Thr Leu Gly Met Asp Glu Leu Tyr Lys Gly Gly Ser
           195                 200                 205
   Gly Gly Met Val Ser Lys Gly Glu Glu Leu Phe Thr Gly Val Val Pro
       210                 215                 220
   Ile Leu Val Glu Leu Asp Gly Asp Val Asn Gly His Lys Phe Ser Val
   225                 230                 235                 240
   Ser Gly Glu Gly Glu Gly Asp Ala Thr Tyr Gly Lys Leu Thr Leu Lys
                   245                 250                 255
   Leu Ile Cys Thr Thr Gly Lys Leu Pro Val Pro Trp Pro Thr Leu Val
               260                 265                 270
   Thr Thr Leu Gly Tyr Gly Leu Gln Cys Phe Ala Arg Tyr Pro Asp His
           275                 280                 285
   Met Lys Gln His Asp Phe Phe Lys Ser Ala Met Pro Glu Gly Tyr Val
       290                 295                 300
   Gln Glu Arg Thr Ile Phe Phe Lys Asp Asp Gly Asn Tyr Lys Thr Arg
   305                 310                 315                 320
   Ala Glu Val Lys Phe Glu Gly Asp Thr Leu Val Asn Arg Ile Glu Leu
                   325                 330                 335
   Lys Gly Ile Asp Phe Lys Glu Asp Gly Asn Ile Leu Gly His Lys Leu
               340                 345                 350
   Glu Tyr Asn Tyr Asn Ser His Asn Val Tyr Ile Thr Ala Asp Lys Gln
           355                 360                 365
   Lys Asn Gly Ile Lys Ala Asn Phe Lys Ile Arg His Asn Ile Glu
       370                 375                 380

<210>  32
<211>  512
<212>  PRT
<213>  Artificial Sequence

<220>
<223>  scFv-ValVal-cp176-172mVenus

<400>  32

Met Arg Gly Ser His His His His His His Gly Met Ala Ser Met Thr
1               5                   10                  15

Gly Gly Gln Gln Met Gly Arg Gly Ser Glu Phe Met Ala Glu Val Gln
            20                  25                  30

Leu Val Glu Ser Gly Gly Gly Leu Val Gln Pro Gly Gly Ser Leu Arg
        35                  40                  45

Leu Ser Cys Ala Ala Ser Gly Phe Thr Phe Ser Ser Tyr Ala Met Ser
    50                  55                  60

Trp Val Arg Gln Ala Pro Gly Lys Gly Leu Glu Trp Val Ser Ala Ile
65                  70                  75                  80

Ser Gly Ser Gly Gly Ser Thr Tyr Tyr Ala Asp Ser Val Lys Gly Arg
                85                  90                  95

Phe Thr Ile Ser Arg Asp Asn Ser Lys Asn Thr Leu Tyr Leu Gln Met

100                 105                 110

Asn Ser Leu Arg Ala Glu Asp Thr Ala Val Tyr Tyr Cys Ala Arg Gly
        115                 120                 125

Leu Phe Ser Trp Leu Leu Trp Gly Gln Gly Thr Leu Val Thr Val Ser
    130                 135                 140

Ser Gly Gly Gly Gly Ser Gly Gly Gly Gly Ser Gly Gly Ser Ala Leu
145                 150                 155                 160

Gln Ser Val Leu Thr Gln Pro Pro Ser Ala Ser Gly Thr Pro Gly Gln
                165                 170                 175

Arg Val Thr Ile Ser Cys Ser Gly Ser Ser Ser Asn Ile Gly Ser Asn
            180                 185                 190

Tyr Val Tyr Trp Tyr Gln Gln Leu Pro Gly Thr Ala Pro Lys Leu Leu
        195                 200                 205

Ile Tyr Arg Asn Asn Gln Arg Pro Ser Gly Val Pro Asp Arg Phe Ser
    210                 215                 220

Gly Ser Lys Ser Gly Thr Ser Ala Ser Leu Ala Ile Ser Gly Leu Arg
225                 230                 235                 240

Ser Glu Asp Glu Ala Asp Tyr Tyr Cys Ala Ala Trp Asp Asp Ser Leu
                245                 250                 255

Leu Ala Leu Val Phe Gly Gly Gly Thr Lys Leu Thr Val Val Val Val
            260                 265                 270

Gln Leu Ala Asp His Tyr Gln Gln Asn Thr Pro Ile Gly Asp Gly Pro
        275                 280                 285

Val Leu Leu Pro Asp Asn His Tyr Leu Ser Tyr Gln Ser Lys Leu Ser
    290                 295                 300

Lys Asp Pro Asn Glu Lys Arg Asp His Met Val Leu Leu Glu Phe Val
305                 310                 315                 320

Thr Ala Ala Gly Ile Thr Leu Gly Met Asp Glu Leu Tyr Lys Gly Gly
                325                 330                 335

Ser Gly Gly Met Val Ser Lys Gly Glu Glu Leu Phe Thr Gly Val Val
            340                 345                 350

Pro Ile Leu Val Glu Leu Asp Gly Asp Val Asn Gly His Lys Phe Ser
        355                 360                 365

Val Ser Gly Glu Gly Glu Gly Asp Ala Thr Tyr Gly Lys Leu Thr Leu
    370                 375                 380

Lys Leu Ile Cys Thr Thr Gly Lys Leu Pro Val Pro Trp Pro Thr Leu
385                 390                 395                 400

Val Thr Thr Leu Gly Tyr Gly Leu Gln Cys Phe Ala Arg Tyr Pro Asp
                405                 410                 415

His Met Lys Gln His Asp Phe Phe Lys Ser Ala Met Pro Glu Gly Tyr
            420                 425                 430

Val Gln Glu Arg Thr Ile Phe Phe Lys Asp Asp Gly Asn Tyr Lys Thr
        435                 440                 445

Arg Ala Glu Val Lys Phe Glu Gly Asp Thr Leu Val Asn Arg Ile Glu
    450                 455                 460

Leu Lys Gly Ile Asp Phe Lys Glu Asp Gly Asn Ile Leu Gly His Lys
465                 470                 475                 480

Leu Glu Tyr Asn Tyr Asn Ser His Asn Val Tyr Ile Thr Ala Asp Lys
                485                 490                 495

Gln Lys Asn Gly Ile Lys Ala Asn Phe Lys Ile Arg His Asn Ile Glu
            500                 505                 510

<210> 33
<211> 248
<212> PRT
<213> Artificial Sequence

<220>
<223> Actin-binding region of Utrophin

<400> 33

Met Ala Lys Tyr Gly Glu His Glu Ala Ser Pro Asp Asn Gly Gln Asn
1               5                   10                  15

Glu Phe Ser Asp Ile Ile Lys Ser Arg Ser Asp Glu His Asn Asp Val
            20                  25                  30

Gln Lys Lys Thr Phe Thr Lys Trp Ile Asn Ala Arg Phe Ser Lys Ser
        35                  40                  45

Gly Lys Pro Pro Ile Asn Asp Met Phe Thr Asp Leu Lys Asp Gly Arg

50                  55                  60

Lys Leu Leu Asp Leu Leu Glu Gly Leu Thr Gly Thr Ser Leu Pro Lys
65                  70                  75                  80

Glu Arg Gly Ser Thr Arg Val His Ala Leu Asn Asn Val Asn Arg Val
                85                  90                  95

Leu Gln Val Leu His Gln Asn Asn Val Glu Leu Val Asn Ile Gly Gly
            100                 105                 110

Thr Asp Ile Val Asp Gly Asn His Lys Leu Thr Leu Gly Leu Leu Trp
        115                 120                 125

Ser Ile Ile Leu His Trp Gln Val Lys Asp Val Met Lys Asp Val Met
    130                 135                 140

Ser Asp Leu Gln Gln Thr Asn Ser Glu Lys Ile Leu Leu Ser Trp Val
145                 150                 155                 160

Arg Gln Thr Thr Arg Pro Tyr Ser Gln Val Asn Val Leu Asn Phe Thr
                165                 170                 175

Thr Ser Trp Thr Asp Gly Leu Ala Phe Asn Ala Val Leu His Arg His
            180                 185                 190

Lys Pro Asp Leu Phe Ser Trp Asp Lys Val Val Lys Met Ser Pro Ile
        195                 200                 205

Glu Arg Leu Glu His Ala Phe Ser Lys Ala Gln Thr Tyr Leu Gly Ile
    210                 215                 220

Glu Lys Leu Leu Asp Pro Glu Asp Val Ala Val Arg Leu Pro Asp Lys
225                 230                 235                 240

Lys Ser Ile Ile Met Tyr Leu Phe
                245

<210>  34
<211>  233
<212>  PRT
<213>  Artificial Sequence

<220>
<223>  mEGFP

<400>  34

Glu Leu Phe Thr Gly Val Val Pro Ile Leu Val Glu Leu Asp Gly Asp
1               5                   10                  15

Val Asn Gly His Lys Phe Ser Val Ser Gly Glu Gly Glu Gly Asp Ala
            20                  25                  30

Thr Tyr Gly Lys Leu Thr Leu Lys Phe Ile Cys Thr Thr Gly Lys Leu
        35                  40                  45

Pro Val Pro Trp Pro Thr Leu Val Thr Thr Leu Thr Tyr Gly Val Gln
    50                  55                  60

Cys Phe Ser Arg Tyr Pro Asp His Met Lys Gln His Asp Phe Phe Lys
65                  70                  75                  80

Ser Ala Met Pro Glu Gly Tyr Val Gln Glu Arg Thr Ile Phe Phe Lys
                85                  90                  95

Asp Asp Gly Asn Tyr Lys Thr Arg Ala Glu Val Lys Phe Glu Gly Asp
            100                 105                 110

Thr Leu Val Asn Arg Ile Glu Leu Lys Gly Ile Asp Phe Lys Glu Asp
        115                 120                 125

Gly Asn Ile Leu Gly His Lys Leu Glu Tyr Asn Tyr Asn Ser His Asn
    130                 135                 140

Val Tyr Ile Met Ala Asp Lys Gln Lys Asn Gly Ile Lys Val Asn Phe
145                 150                 155                 160

Lys Ile Arg His Asn Ile Glu Asp Gly Ser Val Gln Leu Ala Asp His
                165                 170                 175

Tyr Gln Gln Asn Thr Pro Ile Gly Asp Gly Pro Val Leu Leu Pro Asp
            180                 185                 190

Asn His Tyr Leu Ser Thr Gln Ser Lys Leu Ser Lys Asp Pro Asn Glu
        195                 200                 205

Lys Arg Asp His Met Val Leu Leu Glu Phe Val Thr Ala Ala Gly Ile
    210                 215                 220

Thr Leu Gly Met Asp Glu Leu Tyr Lys
225                 230

<210>  35
<211>  487
<212>  PRT
<213>  Artificial Sequence

<220>
<223>  UtrCH-mEGFP

<400>  35
   Met Ala Lys Tyr Gly Glu His Glu Ala Ser Pro Asp Asn Gly Gln Asn
   1               5                   10                  15
   Glu Phe Ser Asp Ile Ile Lys Ser Arg Ser Asp Glu His Asn Asp Val
               20                  25                  30
   Gln Lys Lys Thr Phe Thr Lys Trp Ile Asn Ala Arg Phe Ser Lys Ser
           35                  40                  45
   Gly Lys Pro Pro Ile Asn Asp Met Phe Thr Asp Leu Lys Asp Gly Arg
       50                  55                  60
   Lys Leu Leu Asp Leu Leu Glu Gly Leu Thr Gly Thr Ser Leu Pro Lys
   65                  70                  75                  80
   Glu Arg Gly Ser Thr Arg Val His Ala Leu Asn Asn Val Asn Arg Val
                   85                  90                  95
   Leu Gln Val Leu His Gln Asn Asn Val Glu Leu Val Asn Ile Gly Gly
               100                 105                 110
   Thr Asp Ile Val Asp Gly Asn His Lys Leu Thr Leu Gly Leu Leu Trp
           115                 120                 125
   Ser Ile Ile Leu His Trp Gln Val Lys Asp Val Met Lys Asp Val Met
       130                 135                 140
   Ser Asp Leu Gln Gln Thr Asn Ser Glu Lys Ile Leu Leu Ser Trp Val
   145                 150                 155                 160
   Arg Gln Thr Thr Arg Pro Tyr Ser Gln Val Asn Val Leu Asn Phe Thr
                   165                 170                 175
   Thr Ser Trp Thr Asp Gly Leu Ala Phe Asn Ala Val Leu His Arg His
               180                 185                 190
   Lys Pro Asp Leu Phe Ser Trp Asp Lys Val Val Lys Met Ser Pro Ile
           195                 200                 205
   Glu Arg Leu Glu His Ala Phe Ser Lys Ala Gln Thr Tyr Leu Gly Ile
       210                 215                 220
   Glu Lys Leu Leu Asp Pro Glu Asp Val Ala Val Arg Leu Pro Asp Lys
   225                 230                 235                 240
   Lys Ser Ile Ile Met Tyr Leu Phe Glu Ala Ala Ala Lys Ala Glu Leu
                   245                 250                 255
   Phe Thr Gly Val Val Pro Ile Leu Val Glu Leu Asp Gly Asp Val Asn
               260                 265                 270
   Gly His Lys Phe Ser Val Ser Gly Glu Gly Glu Gly Asp Ala Thr Tyr
           275                 280                 285
   Gly Lys Leu Thr Leu Lys Phe Ile Cys Thr Thr Gly Lys Leu Pro Val
       290                 295                 300
   Pro Trp Pro Thr Leu Val Thr Thr Leu Thr Tyr Gly Val Gln Cys Phe
   305                 310                 315                 320
   Ser Arg Tyr Pro Asp His Met Lys Gln His Asp Phe Phe Lys Ser Ala
                   325                 330                 335
   Met Pro Glu Gly Tyr Val Gln Glu Arg Thr Ile Phe Phe Lys Asp Asp
               340                 345                 350
   Gly Asn Tyr Lys Thr Arg Ala Glu Val Lys Phe Glu Gly Asp Thr Leu
           355                 360                 365
   Val Asn Arg Ile Glu Leu Lys Gly Ile Asp Phe Lys Glu Asp Gly Asn
       370                 375                 380
   Ile Leu Gly His Lys Leu Glu Tyr Asn Tyr Asn Ser His Asn Val Tyr
   385                 390                 395                 400
   Ile Met Ala Asp Lys Gln Lys Asn Gly Ile Lys Val Asn Phe Lys Ile
                   405                 410                 415
   Arg His Asn Ile Glu Asp Gly Ser Val Gln Leu Ala Asp His Tyr Gln
               420                 425                 430
   Gln Asn Thr Pro Ile Gly Asp Gly Pro Val Leu Leu Pro Asp Asn His
           435                 440                 445
   Tyr Leu Ser Thr Gln Ser Lys Leu Ser Lys Asp Pro Asn Glu Lys Arg
       450                 455                 460
   Asp His Met Val Leu Leu Glu Phe Val Thr Ala Ala Gly Ile Thr Leu
   465                 470                 475                 480
   Gly Met Asp Glu Leu Tyr Lys
   485

<210>  36
<211>  224
<212>  PRT
<213>  Artificial Sequence

<220>
<223>  N-terminal trancated mScarlet

<400>  36

Ile Lys Glu Phe Met Arg Phe Lys Val His Met Glu Gly Ser Met Asn
1               5                   10                  15

Gly His Glu Phe Glu Ile Glu Gly Glu Gly Glu Gly Arg Pro Tyr Glu
            20                  25                  30

Gly Thr Gln Thr Ala Lys Leu Lys Val Thr Lys Gly Gly Pro Leu Pro
        35                  40                  45

Phe Ser Trp Asp Ile Leu Ser Pro Gln Phe Met Tyr Gly Ser Arg Ala
    50                  55                  60

Phe Thr Lys His Pro Ala Asp Ile Pro Asp Tyr Tyr Lys Gln Ser Phe
65                  70                  75                  80

Pro Glu Gly Phe Lys Trp Glu Arg Val Met Asn Phe Glu Asp Gly Gly
                85                  90                  95

Ala Val Thr Val Thr Gln Asp Thr Ser Leu Glu Asp Gly Thr Leu Ile
            100                 105                 110

Tyr Lys Val Lys Leu Arg Gly Thr Asn Phe Pro Pro Asp Gly Pro Val
        115                 120                 125

Met Gln Lys Lys Thr Met Gly Trp Glu Ala Ser Thr Glu Arg Leu Tyr
    130                 135                 140

Pro Glu Asp Gly Val Leu Lys Gly Asp Ile Lys Met Ala Leu Arg Leu
145                 150                 155                 160

Lys Asp Gly Gly Arg Tyr Leu Ala Asp Phe Lys Thr Thr Tyr Lys Ala
                165                 170                 175

Lys Lys Pro Val Gln Met Pro Gly Ala Tyr Asn Val Asp Arg Lys Leu
            180                 185                 190

Asp Ile Thr Ser His Asn Glu Asp Tyr Thr Val Val Glu Gln Tyr Glu
        195                 200                 205

Arg Ser Glu Gly Arg His Ser Thr Gly Gly Met Asp Glu Leu Tyr Lys
    210                 215                 220

<210>  37
<211>  315
<212>  PRT
<213>  Artificial Sequence

<220>
<223>  Fusion protein of affimer binding to F-actin and trancated
       mScarlet

<400>  37

Met Gly Ala Ser Leu Glu Ile Glu Glu Leu Ala Arg Phe Ala Val Asp
1               5                   10                  15

Glu His Asn Lys Lys Glu Asn Ala Leu Leu Glu Phe Val Arg Val Val
            20                  25                  30

Lys Ala Lys Glu Gln Ser Ser Val Pro His Trp Trp Trp Thr Thr Met
        35                  40                  45

Tyr Tyr Leu Thr Leu Glu Ala Lys Asp Gly Gly Lys Lys Lys Leu Tyr
    50                  55                  60

Glu Ala Lys Val Trp Val Lys Arg Asp Pro Asn Met Ile Phe Lys Ile
65                  70                  75                  80

Asn Phe Lys Glu Leu Gln Glu Phe Lys Pro Val Ile Lys Glu Phe Met
                85                  90                  95

Arg Phe Lys Val His Met Glu Gly Ser Met Asn Gly His Glu Phe Glu
            100                 105                 110

Ile Glu Gly Glu Gly Glu Gly Arg Pro Tyr Glu Gly Thr Gln Thr Ala
        115                 120                 125

Lys Leu Lys Val Thr Lys Gly Gly Pro Leu Pro Phe Ser Trp Asp Ile
    130                 135                 140

Leu Ser Pro Gln Phe Met Tyr Gly Ser Arg Ala Phe Thr Lys His Pro
145                 150                 155                 160

Ala Asp Ile Pro Asp Tyr Tyr Lys Gln Ser Phe Pro Glu Gly Phe Lys
                165                 170                 175

Trp Glu Arg Val Met Asn Phe Glu Asp Gly Gly Ala Val Thr Val Thr
            180                 185                 190

Gln Asp Thr Ser Leu Glu Asp Gly Thr Leu Ile Tyr Lys Val Lys Leu
        195                 200                 205

Arg Gly Thr Asn Phe Pro Pro Asp Gly Pro Val Met Gln Lys Lys Thr
    210                 215                 220

Met Gly Trp Glu Ala Ser Thr Glu Arg Leu Tyr Pro Glu Asp Gly Val
225                 230                 235                 240

Leu Lys Gly Asp Ile Lys Met Ala Leu Arg Leu Lys Asp Gly Gly Arg
                245                 250                 255

Tyr Leu Ala Asp Phe Lys Thr Thr Tyr Lys Ala Lys Lys Pro Val Gln
            260                 265                 270

Met Pro Gly Ala Tyr Asn Val Asp Arg Lys Leu Asp Ile Thr Ser His
        275                 280                 285

Asn Glu Asp Tyr Thr Val Val Glu Gln Tyr Glu Arg Ser Glu Gly Arg
    290                 295                 300

His Ser Thr Gly Gly Met Asp Glu Leu Tyr Lys
305                 310                 315

<210>  38
<211>  221
<212>  PRT
<213>  Artificial Sequence

<220>
<223>  N-terminal trancated mNeonGreen

<400>  38

Thr His Glu Leu His Ile Phe Gly Ser Ile Asn Gly Val Asp Phe Asp
1               5                   10                  15

Met Val Gly Gln Gly Thr Gly Asn Pro Asn Asp Gly Tyr Glu Glu Leu
            20                  25                  30

Asn Leu Lys Ser Thr Lys Gly Asp Leu Gln Phe Ser Pro Trp Ile Leu
        35                  40                  45

Val Pro His Ile Gly Tyr Gly Phe His Gln Tyr Leu Pro Tyr Pro Asp
    50                  55                  60

Gly Met Ser Pro Phe Gln Ala Ala Met Val Asp Gly Ser Gly Tyr Gln
65                  70                  75                  80

Val His Arg Thr Met Gln Phe Glu Asp Gly Ala Ser Leu Thr Val Asn
                85                  90                  95

Tyr Arg Tyr Thr Tyr Glu Gly Ser His Ile Lys Gly Glu Ala Gln Val
            100                 105                 110

Lys Gly Thr Gly Phe Pro Ala Asp Gly Pro Val Met Thr Asn Ser Leu
        115                 120                 125

Thr Ala Ala Asp Trp Cys Arg Ser Lys Lys Thr Tyr Pro Asn Asp Lys
    130                 135                 140

Thr Ile Ile Ser Thr Phe Lys Trp Ser Tyr Thr Thr Gly Asn Gly Lys
145                 150                 155                 160

Arg Tyr Arg Ser Thr Ala Arg Thr Thr Tyr Thr Phe Ala Lys Pro Met
                165                 170                 175

Ala Ala Asn Tyr Leu Lys Asn Gln Pro Met Tyr Val Phe Arg Lys Thr
            180                 185                 190

Glu Leu Lys His Ser Lys Thr Glu Leu Asn Phe Lys Glu Trp Gln Lys
        195                 200                 205

Ala Phe Thr Asp Val Met Gly Met Asp Glu Leu Tyr Lys
    210                 215                 220

<210>  39
<211>  312
<212>  PRT
<213>  Artificial Sequence

<220>
<223>  Fusion protein of affimer binding to F-actin and mNeonGreen

<400>  39

Met Gly Ala Ser Leu Glu Ile Glu Glu Leu Ala Arg Phe Ala Val Asp
1               5                   10                  15

Glu His Asn Lys Lys Glu Asn Ala Leu Leu Glu Phe Val Arg Val Val
            20                  25                  30

Lys Ala Lys Glu Gln Ser Ser Val Pro His Trp Trp Trp Thr Thr Met
        35                  40                  45

Tyr Tyr Leu Thr Leu Glu Ala Lys Asp Gly Gly Lys Lys Lys Leu Tyr
    50                  55                  60

Glu Ala Lys Val Trp Val Lys Arg Asp Pro Asn Met Ile Phe Lys Ile
65                  70                  75                  80

Asn Phe Lys Glu Leu Gln Glu Phe Lys Pro Val Thr His Glu Leu His
                85                  90                  95

Ile Phe Gly Ser Ile Asn Gly Val Asp Phe Asp Met Val Gly Gln Gly
            100                 105                 110

Thr Gly Asn Pro Asn Asp Gly Tyr Glu Glu Leu Asn Leu Lys Ser Thr
        115                 120                 125

Lys Gly Asp Leu Gln Phe Ser Pro Trp Ile Leu Val Pro His Ile Gly
    130                 135                 140

Tyr Gly Phe His Gln Tyr Leu Pro Tyr Pro Asp Gly Met Ser Pro Phe
145                 150                 155                 160

Gln Ala Ala Met Val Asp Gly Ser Gly Tyr Gln Val His Arg Thr Met
                165                 170                 175

Gln Phe Glu Asp Gly Ala Ser Leu Thr Val Asn Tyr Arg Tyr Thr Tyr
            180                 185                 190

Glu Gly Ser His Ile Lys Gly Glu Ala Gln Val Lys Gly Thr Gly Phe
        195                 200                 205

Pro Ala Asp Gly Pro Val Met Thr Asn Ser Leu Thr Ala Ala Asp Trp
    210                 215                 220

Cys Arg Ser Lys Lys Thr Tyr Pro Asn Asp Lys Thr Ile Ile Ser Thr
225                 230                 235                 240

Phe Lys Trp Ser Tyr Thr Thr Gly Asn Gly Lys Arg Tyr Arg Ser Thr
                245                 250                 255

Ala Arg Thr Thr Tyr Thr Phe Ala Lys Pro Met Ala Ala Asn Tyr Leu
            260                 265                 270

Lys Asn Gln Pro Met Tyr Val Phe Arg Lys Thr Glu Leu Lys His Ser
        275                 280                 285

Lys Thr Glu Leu Asn Phe Lys Glu Trp Gln Lys Ala Phe Thr Asp Val
    290                 295                 300

Met Gly Met Asp Glu Leu Tyr Lys
305                 310

<210>  40
<211>  13
<212>  PRT
<213>  Artificial Sequence

<220>
<223>  ALFA tag

<400>  40

Ser Arg Leu Glu Glu Glu Leu Arg Arg Arg Leu Thr Glu
1               5                   10


<210>  41
<211>  103
<212>  PRT
<213>  Artificial Sequence

<220>
<223>  F-actin-binding affimer having ALFA tag

<400>  41

Met Gly Ser Arg Leu Glu Glu Glu Leu Arg Arg Arg Leu Thr Glu Ile
1               5                   10                  15


Glu Glu Leu Ala Arg Phe Ala Val Asp Glu His Asn Lys Lys Glu Asn
            20                  25                  30


Ala Leu Leu Glu Phe Val Arg Val Val Lys Ala Lys Glu Gln Ser Ser
        35                  40                  45


Val Pro His Trp Trp Trp Thr Thr Met Tyr Tyr Leu Thr Leu Glu Ala
    50                  55                  60


Lys Asp Gly Gly Lys Lys Lys Leu Tyr Glu Ala Lys Val Trp Val Lys
65                  70                  75                  80


Arg Asp Pro Asn Met Ile Phe Lys Ile Asn Phe Lys Glu Leu Gln Glu
                85                  90                  95


Phe Lys Pro Val Gly Asp Ala
            100


<210>  42
<211>  122
<212>  PRT
<213>  Artificial Sequence

<220>
<223>  Anti-ALFA tag nanobody

<400>  42

Met Ala Glu Val Gln Leu Gln Glu Ser Gly Gly Gly Leu Val Gln Pro
1               5                   10                  15

Gly Gly Ser Leu Arg Leu Ser Cys Thr Ala Ser Gly Val Thr Ile Ser
            20                  25                  30

Ala Leu Asn Ala Met Ala Met Gly Trp Tyr Arg Gln Ala Pro Gly Glu
        35                  40                  45

Arg Arg Val Met Val Ala Ala Val Ser Glu Arg Gly Asn Ala Met Tyr
    50                  55                  60

Arg Glu Ser Val Gln Gly Arg Phe Thr Val Thr Arg Asp Phe Thr Asn
65                  70                  75                  80

Lys Met Val Ser Leu Gln Met Asp Asn Leu Lys Pro Glu Asp Thr Ala
                85                  90                  95

Val Tyr Tyr Cys His Val Leu Glu Asp Arg Val Asp Ser Phe His Asp
            100                 105                 110

Tyr Trp Gly Gln Gly Thr Gln Val Thr Val
        115                 120

<210>  43
<211>  363
<212>  PRT
<213>  Artificial Sequence

<220>
<223>  Fusion protein of anti-ALFA tag nanobody and cp176-172mVenus

<400>  43

Met Ala Glu Val Gln Leu Gln Glu Ser Gly Gly Gly Leu Val Gln Pro
1               5                   10                  15

Gly Gly Ser Leu Arg Leu Ser Cys Thr Ala Ser Gly Val Thr Ile Ser
            20                  25                  30

Ala Leu Asn Ala Met Ala Met Gly Trp Tyr Arg Gln Ala Pro Gly Glu
        35                  40                  45

Arg Arg Val Met Val Ala Ala Val Ser Glu Arg Gly Asn Ala Met Tyr
    50                  55                  60

Arg Glu Ser Val Gln Gly Arg Phe Thr Val Thr Arg Asp Phe Thr Asn
65                  70                  75                  80

Lys Met Val Ser Leu Gln Met Asp Asn Leu Lys Pro Glu Asp Thr Ala

85                  90                  95

Val Tyr Tyr Cys His Val Leu Glu Asp Arg Val Asp Ser Phe His Asp
            100                 105                 110

Tyr Trp Gly Gln Gly Thr Gln Val Thr Val Val Gln Leu Ala Asp His
        115                 120                 125

Tyr Gln Gln Asn Thr Pro Ile Gly Asp Gly Pro Val Leu Leu Pro Asp
    130                 135                 140

Asn His Tyr Leu Ser Tyr Gln Ser Lys Leu Ser Lys Asp Pro Asn Glu
145                 150                 155                 160

Lys Arg Asp His Met Val Leu Leu Glu Phe Val Thr Ala Ala Gly Ile
                165                 170                 175

Thr Leu Gly Met Asp Glu Leu Tyr Lys Gly Gly Ser Gly Gly Met Val
            180                 185                 190

Ser Lys Gly Glu Glu Leu Phe Thr Gly Val Val Pro Ile Leu Val Glu
        195                 200                 205

Leu Asp Gly Asp Val Asn Gly His Lys Phe Ser Val Ser Gly Glu Gly
    210                 215                 220

Glu Gly Asp Ala Thr Tyr Gly Lys Leu Thr Leu Lys Leu Ile Cys Thr
225                 230                 235                 240

Thr Gly Lys Leu Pro Val Pro Trp Pro Thr Leu Val Thr Thr Leu Gly
                245                 250                 255

Tyr Gly Leu Gln Cys Phe Ala Arg Tyr Pro Asp His Met Lys Gln His
            260                 265                 270

Asp Phe Phe Lys Ser Ala Met Pro Glu Gly Tyr Val Gln Glu Arg Thr
        275                 280                 285

Ile Phe Phe Lys Asp Asp Gly Asn Tyr Lys Thr Arg Ala Glu Val Lys
    290                 295                 300

Phe Glu Gly Asp Thr Leu Val Asn Arg Ile Glu Leu Lys Gly Ile Asp
305                 310                 315                 320

Phe Lys Glu Asp Gly Asn Ile Leu Gly His Lys Leu Glu Tyr Asn Tyr
                325                 330                 335

Asn Ser His Asn Val Tyr Ile Thr Ala Asp Lys Gln Lys Asn Gly Ile
            340                 345                 350

Lys Ala Asn Phe Lys Ile Arg His Asn Ile Glu
        355                 360

## Description

### Technical Field

The present invention relates to a fusion protein of an antigen-binding protein and a fluorescent protein or a fluorescence-labeled tag protein.

### Background Art

Techniques have been developed to link a fluorescent protein or a fluorescence-labeled tag protein to a target protein for visualizing the protein. These techniques are used for observing localization of the target protein by fluorescence microscopy.

Fluorescence is known to have polarization. For example, it is disclosed that GFP, which is a green fluorescent protein, has polarization (Non Patent Literature 1). Further, a case in which polarization of a protein called septin was observed by linking GFP to septin is disclosed (Non Patent Literature 2). In Non Patent Literature 2, it was revealed for the first time that the orientation of septin fibers on the cell division plane of Saccharomyces cerevisiae changed over time, by observing its polarization.

### Citation List

### [Non Patent Literature]

Non Patent Literature 1: Inoue et al., PNAS, 99: 4272-4277, 2002
Non Patent Literature 2: Vrabioiu and Mitchison, Nature, 28, 466-469, 2006

### Summary of Invention

The present invention provides a fusion protein of an antigen-binding protein and a fluorescent protein or a fluorescence-labeled tag protein.

The inventors have obtained a large number of fusion proteins of an antigen-binding protein and a fluorescent protein or a fluorescence-labeled tag protein - those in which helices of two proteins are linked and those in which β-sheets are linked. The inventors have found that the obtained fusion proteins retain the binding property to antigens and are suitable for observation of fluorescence. The inventors have also found that the fusion proteins obtained are suitable for observation of fluorescence polarization.

That is, the present invention provides the following industrially applicable invention.
(1) A fusion protein of an antigen-binding protein and a fluorescent protein or a fluorescence-labeled tag, wherein the antigen-binding protein is an antigen-binding protein having a helix structure or a β-sheet structure at a terminus, the fluorescent protein or the fluorescence-labeled tag is a fluorescent protein or a fluorescence-labeled tag having a helix structure or a β-sheet structure at a terminus, and the helix at the terminus and the helix at the terminus are linked, or the β-sheet structure at the terminus and the β-sheet structure at the terminus are linked.
(2) The fusion protein according to (1) above, wherein the antigen-binding protein has a helix structure at the N-terminus, the fluorescent protein or the fluorescence-labeled tag has a helix structure at the C-terminus, and the helix at the terminus of the antigen-binding protein and the helix at the terminus of the fluorescent protein or the fluorescence-labeled tag are linked.
(3) The fusion protein according to (2) above, wherein the antigen-binding protein is an Affimer.
(4) The fusion protein according to (2) or (3) above, wherein the fluorescent protein is a fluorescent protein having a helix structure at the C-terminus, selected from GFP and a fluorescent protein having a GFP-like β-barrel structure, and a circular permutated mutant of a point mutant.
(5) The fusion protein according to (1) above, wherein the antigen-binding protein has a β-sheet structure at the C-terminus, the fluorescent protein has a β-sheet structure at the N-terminus, and the β-sheet structure at the C-terminus and the β-sheet structure at the N-terminus are linked.
(6) The fusion protein according to (5) above, wherein the antigen-binding protein is a Nanobody or a scFv.
(7) The fusion protein according to (5) or (6) above, wherein the fluorescent protein is a fluorescent protein having a β-sheet structure at the N-terminus, selected from the group consisting of GFP and a fluorescent protein having a GFP-like β-barrel structure, and a point mutant, and a circular permutated mutant and a deletion mutant thereof.
(8) The fusion protein according to (2) or (3) above, wherein the fluorescence-labeled tag is a dehalogenation domain of haloalkane dehalogenase having an amino acid sequence corresponding to the amino acid sequence set forth in SEQ ID No: 9 or 29, having a helix structure at the C-terminus.
(9) The fusion protein according to (2) or (3) above, wherein the fluorescent protein is a phytochrome- or cyanobacteriochrome-based near-infrared fluorescent protein having a helix structure at the C-terminus.
(10) A composition comprising the fusion protein according to any one of (1) to (9) above.
(11) A nucleic acid encoding the fusion protein according to any one of (1) to (9) above.
(12) A gene expression vector, or a cell transformed with the vector, for use in expressing a fusion protein in a cell, comprising the nucleic acid of (11) above, wherein the nucleic acid is operably linked to a regulatory sequence, or a cell introduced with a mRNA comprising the nucleic acid of (11) above.
(13) A method for observing an antigen, comprising observing an antigen bound to the fusion protein according to any one of (1) to (9) above.
(14) The method according to (13) above, wherein the antigen is an antigen in an aqueous solution or a cell, or on a cell surface.
(15) The method according to (13) or (14) above, comprising:
   observing each of: a first antigen bound to a first fusion protein which is the fusion protein according to any one of (1) to (9) above; and a second antigen bound to a second fusion protein which is the fusion protein according to any one of (1) to (9) above {with the proviso that the first fusion protein and the second fusion protein have different fluorescence wavelengths}.
(16) The method according to (15) above, comprising performing fluorescence correlation spectroscopy and fluorescence cross-correlation spectroscopy on the first fusion protein and the second fusion protein.
(17) The method according to (16) above, wherein the fluorescence correlation spectroscopy and the fluorescence cross-correlation spectroscopy are each fluorescence polarization correlation spectroscopy.
(18) A method for designing an amino acid sequence of a fusion protein, comprising: providing a first amino acid sequence which is an amino acid sequence of an antigen-binding protein and a second amino acid sequence which is an amino acid sequence of a fluorescent protein, wherein the N-terminus of the first amino acid sequence and the C-terminus of the second amino acid sequence or the C-terminus of the first amino acid sequence and the N-terminus of the second amino acid sequence are both helix structures or β-sheet structures, together form a helix structure upon excision of a part of the N-terminus or the C-terminus, or together form a β-sheet structure upon excision of a part of the N-terminus or the C-terminus; and linking the N-terminus of the first amino acid sequence and the C-terminus of the second amino acid sequence or the C-terminus of the first amino acid sequence and the N-terminus of the second amino acid sequence, to obtain an amino acid sequence with the junction serving as a part of a helix structure or a β-sheet structure.
   (18A) The method according to (18) above, comprising obtaining a nucleic acid encoding the amino acid sequence obtained.
   (18B) A method for producing a gene expression vector for expressing the nucleic acid, comprising operably linking the nucleic acid obtained in (18A) above to a promoter.
   (18C) An amino acid sequence to be obtained by the method according to (18) above.
   (18D) A nucleic acid to be obtained by the method according to (18A) above.
   (18E) A gene expression vector to be obtained by the method according to (18B) above.

### Brief Description of Drawings

[Figure 1A] Figure 1A illustrates a method for linking two proteins in the fusion protein of the present invention (method for linking helices to each other).
[Figure 1B] Figure 1B shows a three-dimensional structural model of a fusion protein of cp-sfGFP and Affimer obtained by estimation from the crystal structure of each protein.
[Figure 1C] Figure 1C shows the results of crystal structure analysis for the fusion protein of cp-sfGFP and Affimer.
[Figure 2] Figure 2 shows the results of fluorescence polarization microscopic observation of cells expressing the fusion protein of the present invention in which the fluorescent protein is cp-sfGFP, and the Affimer is an Affimer that binds to actin.
[Figure 3] Figure 3 shows the results of fluorescence polarization microscopic observation of cells expressing the fusion protein of the present invention in which the fluorescent protein is cpmVenus, and the Affimer is an Affimer that binds to actin.
[Figure 4] Figure 4 shows the results of fluorescence polarization microscopic observation of cells expressing the fusion protein of the present invention in which the fluorescent protein is cpmTurquoise2, and the Affimer is an Affimer that binds to actin.
[Figure 5] Figure 5 illustrates a method of linking the fusion protein of the present invention in which the fluorescence-labeled tag is a HaloTag, and the Affimer is an Affimer that binds to actin.
[Figure 6A] Figure 6A shows a three-dimensional structural model of a fusion protein of HaloTag and Affimer to actin and the results of fluorescence polarization microscopic observation of cells expressing the fusion protein.
[Figure 6B] Figure 6B shows a three-dimensional structural model of a fusion protein of HaloTag and Affimer to actin and the results of fluorescence polarization microscopic observation of cells expressing the fusion protein. The number of turns of the helix around the junction is different from that in Figure 6A.
[Figure 6C] Figure 6C shows a three-dimensional structural model of a fusion protein of HaloTag and Affimer to actin and the results of fluorescence polarization microscopic observation of cells expressing the fusion protein. The number of turns around the junction of the helix is different from that in Figure 6A and Figure 6B.
[Figure 7A] Figure 7A illustrates a linking method of a fusion protein of cp-sfGFP and Nanobody.
[Figure 7B] Figure 7B shows the results of fluorescence polarization microscopic observation of cells expressing a fusion protein of cp-sfGFP and Nanobody that binds to vimentin linked by the linking method of Figure 7A.
[Figure 7C] Figure 7C shows the results of fluorescence polarization microscopic observation of cells expressing a fusion protein of Nanobody that binds to vimentin and cp176-172mVenus linked by the same linking method as in Figure 7A.
[Figure 8A] Figure 8A illustrates a method of linking a fusion protein of mApple and Nanobody.
[Figure 8B] Figure 8B shows the results of fluorescence polarization microscopic observation of cells expressing a fusion protein of mApple and Nanobody that binds to vimentin linked by the linking method of Figure 8A.
[Figure 9A] Figure 9A illustrates a linking method of a fusion protein of cp-sfGFP and scFv.
[Figure 9B] Figure 9B shows the results of fluorescence polarization microscopic observation of cells expressing a fusion protein of cp-sfGFP and scFv that binds to non-muscle myosin IIA linked by the linking method of Figure 9A.
[Figure 9C] Figure 9C shows the results of fluorescence polarization microscopic observation of cells expressing a fusion protein of scFv that binds to myosin and cp176-172mVenus linked by the same linking method as in Figure 7C.

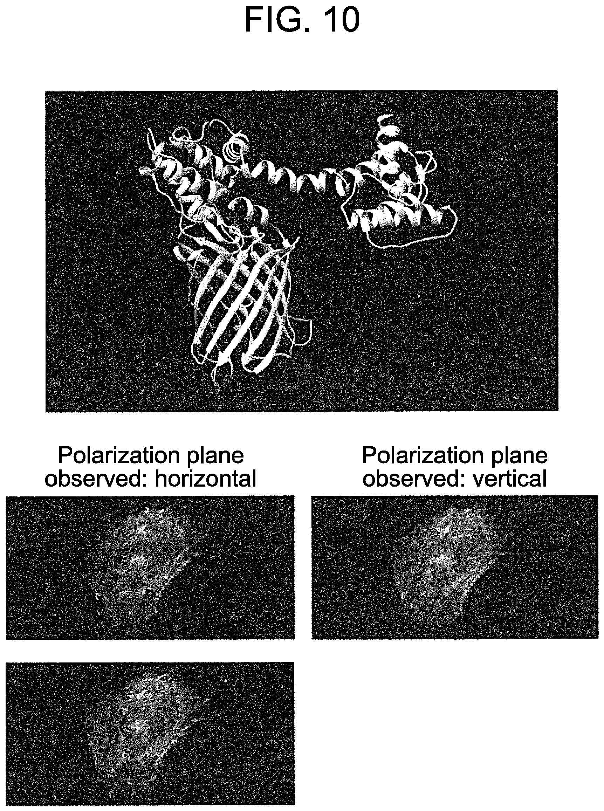

[Figure 10] Figure 10 shows a three-dimensional structural model of a fusion protein (SEQ ID No: 35) of the actin binding domain of utrophin shown in Table 1 and mEGFP and the results of fluorescence polarization microscopic observation of cells expressing the fusion protein.
[Figure 11] Figure 11 shows a three-dimensional structural model of a fusion protein of Affimer that binds to actin and mScarlet and the results of fluorescence polarization microscopic observation of cells using the fusion protein.
[Figure 12] Figure 12 shows a three-dimensional structural model of a fusion protein of Affimer that binds to actin and mNeonGreen and the results of fluorescence polarization microscopic observation of cells using the fusion protein.
[Figure 13] Figure 13 shows a three-dimensional structural model of a fusion protein of Nanobody that binds to an ALFA-tag and cp176-172mVenus and the results of fluorescence polarization microscopic observation of cells co-expressing the fusion protein and a fusion protein of Affimer and an ALFA-tag (that is, a fusion protein in which an alpha helix at the N-terminus of the Affimer that binds to actin is truncated, and an ALFA-tag sequence is connected to the truncated alpha helix so as to form consecutive alpha helixes).

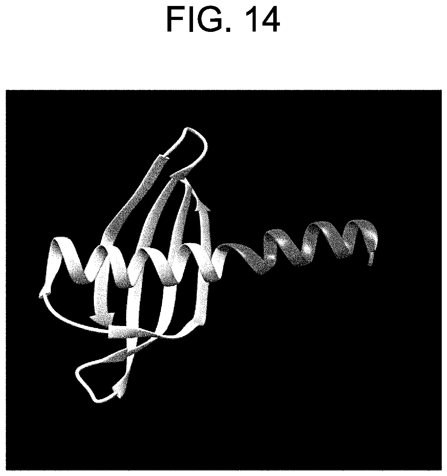

[Figure 14] Figure 14 shows a three-dimensional structural model of an Affimer that binds to actin linked to an ALFA-tag. In the figure, the ALFA-tag is shown by dark gray, and the Affimer is shown by light gray.

### Description of Embodiments

In this description, the "antigen-binding protein" refers to a protein that binds to a specific entity. The antigen-binding protein can be a modified protein or a non-natural protein, or an antigen-binding fragment thereof. The antigen-binding protein is not specifically limited as long as it can obtain a binding affinity to an antigen by modification of an amino acid sequence and is a protein having a β-sheet structure or a helix structure at the N-terminus or the C-terminus (for example, a scaffold protein). Examples thereof include antibodies, antigen binding fragments thereof (such as scFv), single-chain variable domains (V_{HH} domains) of heavy-chain antibodies of camels and llamas (such as Nanobody (trademark)), scaffold proteins with an albumin-binding domain of bacteria serving as the basic skeleton (such as ABD (trademark)), scaffold proteins with the 10th domain of human fibronectin serving as the basic skeleton (such as Adnectin (trademark), FingR (trademark), or Monobody (trademark)), scaffold proteins with the Z domain of staphylococcus protein A serving as the basic skeleton (such as Affibody (trademark)), scaffold proteins with human γ-B-crystallin serving as the basic skeleton (such as Affilin (trademark)), scaffold proteins with a DNA-binding protein, Sac7d, of archaebacteria serving as the basic skeleton (such as Affitin (trademark)), scaffold proteins with a triple antiparallel helix serving as the basic skeleton (such as Alphabody (trademark)), scaffold proteins with human or insect lipocalin serving as the basic skeleton (such as Anticalin (trademark)), scaffold proteins with armadillo protein serving as the basic skeleton (such as armadillo repeat protein), scaffold proteins with human C-type lectin domain CTLD₃ serving as the basic skeleton (such as Atrimer (trademark)), scaffold proteins with a polymerized LDLR-A module serving as the basic skeleton (such as Avimer (trademark)), scaffold proteins with the Fn3 domain of human tenascin C serving as the basic skeleton (such as Centyrin (trademark)), scaffold proteins with the SH3 domain of human Fyn tyrosine kinase serving as the basic skeleton (such as Fynomer (trademark)), scaffold proteins with human BPTI/LACI-D1/ITI-D2/APPI serving as the basic skeleton (such as Knitz domain (trademark)), scaffold proteins with the OB shape of aspartyl-tRNA synthetase of pyrobaculum aerophilum serving as the basic skeleton (such as Obody (trademark)), scaffold proteins with the 14th extracellular domain of human fibronectin III serving as the basic skeleton (such as Pronectin (trademark)), scaffold proteins with a leucine-rich repeat module of a variable lymphocyte receptor of Agnatha serving as the basic skeleton (such as Repebody (trademark)), scaffold proteins with a human ankyrin repeat protein serving as the basic skeleton (such as DARPin (trademark)), and scaffold proteins with stefin A or cystatin serving as the basic skeleton (such as Affimer (trademark)) (Skrlec, K. et al., Trends in Biotechnology, Vol. 33, No. 7: 408-418, 2015). The β-sheet structure or the helix structure at the N-terminus or the C-terminus of each of these proteins can be used for the linkage in the fusion protein of the present invention. In such a scaffold protein, the site of the amino acid involved in the antigen-binding affinity is known, and a scaffold protein having a desired antigen-binding property can be obtained by selecting an amino acid at the site suitable for binding to the antigen by ribosome display or phage display. Among other antigen-binding proteins, an antigen-binding protein having a helix or β-sheet at the N-terminus or the C-terminus may be used in a fusion protein with the fluorescent protein in the present invention. The scaffold protein refers to a protein having a function of retaining a paratope (particularly, retaining a three-dimensional shape thereof) and having a skeleton similar to that of the parent protein (protein before modification) in the tertiary structure. The basic skeleton refers to a part of the scaffold protein other than the intended modification site (such as a paratope). The paratope refers to a part that binds to the antigen in the antigen-binding protein.

In this description, the "fluorescent protein" refers to a protein which absorbs energy by irradiation with short wavelength electromagnetic waves to excite electrons and emits longer wavelength electromagnetic waves when the electrons return to the ground state. Examples of the fluorescent protein include fluorescent proteins emitting visible light and near-infrared fluorescent proteins emitting near-infrared light. Fluorescent proteins suitable for observation of fluorescence polarization can be used preferably for observation of fluorescence polarization. Fluorescent proteins suitable for observation of fluorescence polarization and fluorescent proteins unsuitable for observation of fluorescence polarization can be used simply for fluorescence observation.

In this description, the "Affimer" is a protein which is biologically inactive and has a variant of physically stable stefin A or cystatin as the basic skeleton wherein the protein has antigen-binding sites in two loops presented on the same side from the four β-sheet structures possessed by the basic skeleton. The amino acid sequences of the loop portions can have diversity. The diversity of the amino acid sequences of the loop portions enables Affimers that bind to various antigens to be obtained by phage display. As the basic skeleton of the Affimer, a variant of stefin A having the amino acid sequence corresponding to SEQ ID No: 1 disclosed in WO2009/136182 can be used, for example, and the variant of stefin A in which the 4th glycine of stefin A is optionally substituted with arginine is a protein having heterologous amino acid sequences in two loops presented on the same side from the four β-sheet structures possessed by the basic skeleton (such as the 46th to 54th amino acid sites and the 67th to 84th amino acid sites of stefin A). The Affimer has an α-helix structure at the N-terminus. The Affimer can also be a protein with plant-derived cystatin serving as the basic skeleton and may also be a protein having antigen-binding sites in two loops presented on the same side from the four β-sheet structures possessed by the basic skeleton. Examples of the Affimer with cystatin serving as the basic skeleton include any one sequence of SEQ ID Nos: 1 to 6 disclosed in WO2014/125290. The molecular weight of the Affimer can be about 12 to 14 kDa.

In the present invention, the "Nanobody" is an antigen-binding protein based on variable region domains of an antibody consisting only of heavy chains found in certain animals, whereas antibodies generally consist of heavy chains and light chains. The antibody consisting only of heavy chains is an antibody commonly found in Arabian camels, Bactrian camels, llamas, and alpacas and can bind to an antigen only in the variable region domains of the above heavy chains. In recent years, similar antibodies consisting only of heavy chains have been found in cartilaginous fishes (sharks or the like). The Nanobody has three complementarity determining regions (CDR) and binds to an antigen in these three CDRs. The Nanobody can be obtained by immunizing the above animals which produce an antibody consisting only of the heavy chains with an antigen, isolating B cells from the animals immunized, obtaining a cDNA library containing variable regions, incorporating them into a phage display library using M13 phage, and screening them with the antigen. The Nanobody has a β-sheet structure to which a fluorescent entity can be connected in the present invention at each of the N-terminus and the C-terminus. In the present invention, the β-sheet structure at the N-terminus and/or the C-terminus of Nanobody, for example, the β-sheet structure at the C-terminus can be linked to the β-sheet structure of the fluorescent protein or the fluorescence-labeled tag.

In this description, the "scFv" is a single-chain antigen-binding protein in which a heavy-chain variable region (V_{H}) and a light-chain variable region (V_{L}) of an antibody are linked by a flexible peptide linker. The heavy-chain variable region and the light-chain variable region are connected by the flexible linker, in order to promote the association between the heavy-chain variable region and the light-chain variable region while retaining the antigen specificity of the original antibody by antigen recognition in the two regions. The flexible linker which can freely change its structure corresponding to the association state between the scFv and the antigen, and the like may be used. For example, a glycine-rich sequence of about 15 amino acids (for example, serine may be inserted to ensure hydrophilicity) may be preferably used. As the flexible linker, a linker having an amino acid sequence of -(GGGGS)₃- can be used, for example. The scFv can be obtained by selecting, from a phage library, using a desired antigen binding property as an index. The phage library can be obtained by randomizing antigen-binding sites of scFv, which has framework sequences (for example, framework sequences of mammals such as humans). The scFv has a β-sheet structure at the N-terminus and the C-terminus to which the fluorescent entity can be connected in the present invention. In the present invention, the β-sheet structure at the N-terminus and/or the C-terminus of the scFv, for example, the β-sheet structure at the C-terminus can be linked to the β-sheet structure of the fluorescent protein or the fluorescence-labeled tag.

In this description, the "DARPin" is an antigen-binding protein developed by Molecular partners AG. The DARPin is an artificial protein having ankyrin repeat units (generally, about 2 to 30), and each repeat unit contains skeleton residues and target interaction residues (see WO2002/020565, for example). The ankyrin repeat units have a common folded structure consisting of two antiparallel α-helices followed by a β-hairpin {where the β-hairpin has a loop that binds to the next repeat unit}. The DARPin forms a curved structure by the ankyrin repeat units stacked on one another. The target interaction residues can be present in the β-hairpin of the ankyrin repeat units and a part where the first α-helix exposes. The DARPin can be obtained by selection based on the binding property to the antigen. The DARPin can be obtained, for example, using a method such as phage display, ribosome display, and plasmid display. The DARPin has an α-helix at one end and a β-sheet at the other end and provides a site to which the fluorescent entity is connected in the present invention.

In this description, the "Monobody" is also called FingR or Adnectin and is a scaffold protein with the 10th domain of human fibronectin (fibronectin type III domain) serving as the basic skeleton. This domain has the same structure as the variable domains of an antibody, that is, it has seven β-sheet structures forming a β-sandwich and three loops corresponding to three complementarity determining regions at each side. The binding specificity to an antigen can be modified by the amino acid sequences of the loop BC between the 2nd and 3rd β-sheets, the loop DE between the 4th and 5th β-sheets, and the loop FG between the 6th and 7th β-sheets. Alternatively, it can be modified by the amino acid sequences of the 3rd, 4th, 6th, and 7th β-sheets, in addition to the loop CD between the 3rd and the 4th β-sheets and the loop FG between the 6th and 7th β-sheets. The Monobody has β-sheets at the N-terminus and the C-terminus and can provide a site to which the fluorescent entity is connected.

As used herein, the phrase "having an amino acid sequence corresponding to a certain amino acid sequence" means to include not only having the amino acid sequence concerned but also having an amino acid sequence which has the same function corresponding to the amino acid sequence concerned. For example, in the case where some species have different sequences, not only the amino acid sequence of a specific species, but also an amino acid sequence having the same function of a closely related species with a high similarity to the amino acid sequence of the specific species are included. The phrase "having an amino acid sequence corresponding to a certain amino acid sequence" can mean having an amino acid sequence which has the same function corresponding to the certain amino acid sequence. For example, in the case of a protein, it can mean a protein of another species having an ortholog relationship with a protein having the certain amino acid sequence.

In the present invention, the helix structure (such as α-helix and 3/10 helix) at the C-terminus of the fluorescent protein can be linked to the helix structure at the N-terminus of an Affimer. The two helices linked can form a large helix structure. The fusion protein of the present invention can provide a fusion protein of a fluorescent protein and the Affimer thus linked. The linkage may be carried out by directly binding the helix structures to each other or may be carried out via a linker. As the linker, a linker having a helix structure (helix linker) can be used, for example. In the case of linking the helix structures to each other, the bond angle of the fluorescent protein and the antigen-binding protein can be adjusted by adjusting the lengths of the helix structures and/or the length of the helix linker. The helix linker is not specifically limited but can have an amino acid sequence according to any of SEQ ID Nos: 3, 10, 12, 14, and 23, for example.

In the present invention, the β-sheet structure at the N-terminus of the fluorescent protein and the β-sheet structure at the C-terminus of Nanobody or scFv can be linked. The two β-sheets linked can form one large β-sheet structure. The fusion protein of the present invention can provide a fusion protein of a fluorescent protein and the Nanobody or the scFv thus linked. The linkage may be carried out by directly binding the β-sheets to each other or may be carried out via a linker. As the linker, a linker having a β-sheet structure can be used, or a structurally inflexible amino acid such as one or two valines (V) can be used, for example.

As the fluorescent protein having a helix structure at the C-terminus, a circular permutated mutant of GFP (cpGFP) can be used. Various modified fluorescent proteins of GFP are known. The circular permutated mutant fluorescent protein is a mutant in which the positions of the sequences at the N-terminus and the C-terminus are changed by linking the N-terminus and the C-terminus of the protein with or without a linker and dividing the protein at another suitable position into two. For the circular permutated mutant, it can be an important condition that the N-terminus and the C-terminus are located in the vicinity in the three-dimensional structure. Many fluorescent proteins satisfy this condition, and modification of fluorescent proteins with circular permutated mutants is a standard technique for modifying fluorescent proteins. In the present invention, by using a circular permutated mutant, a fluorescent protein having a helix structure at the C-terminus can be used for producing a fusion protein with an Affimer. In the present invention, by using a circular permutated mutant, a fluorescent protein having a β-sheet structure at the N-terminus can be used for producing a fusion protein with a Nanobody or a scFv.

As the modified fluorescent proteins of GFP {more specifically, examples thereof include GFP, fluorescent proteins having a GFP-like β-barrel structure (that is, GFP-like proteins), and point mutants thereof, or circular permutated mutants of these}, GFP-like fluorescent proteins such as superfolder GFP (sfGFP), EGFP, Citrine, Venus, mVenus, YFP, mApple, mOrange, mCherry, BFP, TagBFP, mTurquoise, Cerulean, mHoneydew, mBanana, tdTomato, mTangerine, mStrawberry, mPlum, mScarlet, mNeonGreen, mNeptune, and NirFP, and circular permutated mutants of these variant fluorescent proteins (such as fluorescent proteins which are circular permutated mutants having a helix structure at the C-terminus; it is clearly indicated that the proteins are circular permutated mutants by prefixing the notation "cp-" to the protein names) can be used, for example. There are many examples known as the modified fluorescent proteins of GFP, such as substitutes in which the order of the β-sheets constituting the β-barrel is changed and fluorescent proteins having a β-barrel structure in which the number of β-sheets is reduced. These can be used for the fusion protein of the present invention. As the fluorescent proteins having a helix structure at the C-terminus, a circular permutated mutant of mTurquiose (cpmTurquoise) can be used. Since the mTurquiose has a helix structure at the N-terminus, cpmTurquiose which is a circular permutated mutant having a helix structure at the C-terminus can be used. There are examples known as the mTurquiose, such as mTurquiose-DR, mTurquiose-GL, mTurquiose-GV, mTurquiose-RA, mTurquiose2, mTurquiose2-G, mTurquiose-146G, and mTurquiose-146S, and variant fluorescent proteins thereof. One or more selected from the group consisting of these examples can be used by converting them into circular permutated mutants having a helix structure at the C-terminus, for example. The crystal structures of these proteins are published, for example, in the Protein Data Bank (PDB), and the β-sheet structures or the helix structures present at the N-terminus or the C-terminus of these proteins can be used for the linkage in the fusion protein of the present invention.

The cp-sfGFP having a helix structure at the C-terminus to be used in the present invention is not specifically limited, as long as it has a helix structure at the C-terminus. For example, cp-sfGFP having an amino acid sequence corresponding to the amino acid sequence of SEQ ID No: 1 can be used.

Examples of the fluorescent proteins having a helix structure at the C-terminus include phytochrome-based near-infrared fluorescent proteins. Examples of the phytochrome-based near-infrared fluorescent proteins include iRFP and miRFP. The iRFP contains the PAS domain and the GAF domain (PAS-GAF) of bacterio-phytochrome RpBphP2 of *Rhodopseudomonas palustris,* emits near-infrared fluorescence by incorporating biliverdin IXa, lacks the α-helix at the C-terminus required for dimerization, and has mutations of S13L, A92T, V104I, V114I, E161K, Y193K, F198Y, D202T, I203V, Y258F, A283V, K288T, and N290Y (see Filonov et al., Nat. Biotechnol., 29(8): 757-761, 2011). The miRFP contains the PAS domain and the GAF domain (PAS-GAF) of bacterio-phytochrome RpBphP1 of *Rhodopseudomonas palustris,* emits near-infrared fluorescence by incorporating biliverdin IXa, lacks the α-helix at the C-terminus required for dimerization, and has various mutations (see Shcherbakova DM et al., Nature Communication, 7, Article number: 12405, 2016). The miRFP is not specifically limited, but examples thereof include miRFP670 (excitation/emission at 642/670 nm), miRFP709 (excitation/emission at 683/709 nm), and miRFP703 (excitation/emission at 673/703 nm), and miRFPs induced from these miRFPs. The miRFP670 can have an amino acid sequence corresponding to the amino acid sequence set forth in SEQ ID No: 22, for example. These phytochrome-based near-infrared fluorescent proteins each have a helix structure at the C-terminus of the GAF domain and can be used for producing a fusion protein with an Affimer. Further, miRFPs derived from the GAF domain of *Nostoc punctiforme-derived* cyanobacteriochrome NpR3784 such as miRFP670nano also can be used for producing the fusion protein of the present invention. As the miRFP670nano, those with 18 mutations of V7M, F25C, M26V, Y27F, P31E, S41A, A48S, N51K, Q55R, T57R, I72Y, G82N, H87Y, N99I, N117H, C119L, L136Q, and Q139V introduced into the GAF domain of NpR3784 can be used. The miRFP670nano has a helix structure at the N-terminus and can be used for the linkage with the fluorescent protein in the present invention.

Examples of the fluorescence-labeled tag protein having a helix structure at the C-terminus include a modified protein derived from the dehalogenation domain of haloalkane dehalogenase of *Rhodococcus* bacteria. The dehalogenation domain of haloalkane dehalogenase has a pocket in which a ligand is inserted near the active center, Asp106. The group, -NH-CH₂CH₂-O-CH₂CH₂-O-(CH₂)₆-Cl, contained in the ligand is inserted in this pocket. A fluorescent entity is used as the ligand, and the group, -NH-CH₂CH₂-O-CH₂CH₂-O-(CH₂)₆-Cl, is introduced into this, thereby enabling the fluorescent entity to be bound to the protein with the above tag. Examples of the dehalogenation domain of haloalkane dehalogenase which can be used for such a purpose include the dehalogenation domain of haloalkane dehalogenase which has lost the dehalogenase activity. For example, an activity mutant (H272F) in which histidine at the active center is converted to phenylalanine can be used, and examples thereof include a dehalogenation domain having an amino acid sequence corresponding to the amino acid sequence set forth in SEQ ID No: 9 or 29. The dehalogenation domain of haloalkane dehalogenase has an α-helix at the C-terminus. The binding of the ligand to the dehalogenation domain is suitable for observing polarization. The present invention can provide use of the dehalogenation domain of haloalkane dehalogenase and an activity mutant thereof in observation of fluorescence polarization. Examples of the fluorescent entity which can be used as the ligand include coumarin, Oregon Green, diAcFAM, tetramethyl rhodamine (TMR), STELLA Fluor 650, STELLA Fluor700, STELLA Fluor 720, indocyanine green, Alexa Fluor 488, and Alexa Fluor 660. They are each commercially available as a HaloTag ligand.

The length of the helix structure is not particularly limited as long as the helix structure is formed. In the linkage between helix structures, the bond angle with an Affimer can be changed by changing the lengths of the amino acids of the helices at the linkage site, which can change the polarization planes of the fluorescent protein with respect to the Affimer. In the case of an α-helix, one turn is composed of about 3.5 amino acids, and therefore the bond angle is changed by about 100 degrees by increasing the α-helix by 1 amino acid. In the case of a 3/10 helix, one turn is composed by about 3 amino acids, and therefore the bond angle is changed by about 120 degrees by increasing the helix by 1 amino acid. The length of the helix can be adjusted by one or more selected from addition, deletion, and insertion of amino acids. Further, the length of the helix can be adjusted as appropriate according to its purpose. The adjustment method is not specifically limited but may be performed by changing the length of any of the helices of the fusion protein or may be performed by inserting a peptide having a helix structure into the linkage site of the fusion protein, for example. In any case, a new helix structure containing regions before and after the linkage site is formed, thereby allowing the fusion protein to be linked more tightly.

The length of the β-sheet structure is not specifically limited as long as the β-sheet structure is formed. In the linkage between β-sheet structures, a new structurally inflexible structure containing the β-sheet structures before and after the linkage site is formed after the linkage, thereby allowing the fusion protein to be linked more tightly.

The fusion protein of the present invention can be as follow, for example:
(A) a fusion protein of a fluorescent protein having a helix structure at the C-terminus and Affimer having a helix structure at the N-terminus {with the proviso that the helix structures of the two proteins are linked to each other};
(B) a fusion protein of the dehalogenation domain of haloalkane dehalogenase having a helix structure at the C-terminus and Affimer having a helix structure at the N-terminus {with the proviso that the helix structures of the two proteins are linked to each other};
(C) a fusion protein of Nanobody having a β-sheet structure at the C-terminus and a fluorescent protein having a β-sheet structure at the N-terminus {with the proviso that the β-sheet structures of the two proteins are linked to each other}; and
(D) a fusion protein of scFv having a β-sheet structure at the C-terminus and a fluorescent protein having a β-sheet structure at the N-terminus {with the proviso that the β-sheet structures of the two proteins are linked to each other}.

The present invention provides a nucleic acid (such as DNA) encoding the fusion protein of the present invention. The nucleic acid encoding the fusion protein of the present invention can be operably linked to at least one regulatory sequence (such as promoter). The present invention provides an expression vector (such as a vector which allows mammalian cells to express the fusion protein) containing a nucleic acid encoding the fusion protein of the present invention and operably linked to at least one regulatory sequence (such as promoter). The regulatory sequence is not specifically limited, but examples thereof include RNA polymerase II promoters such as CMV immediate/early promoter, HSV thymidine kinase promoter, early/late SV40 promoter, retrovirus LTR promoter, and metallothionein I promoter. Examples of promoters to cause expression in Escherichia coli include promoters such as lac, trp, lacI, lacZ, T3, T7, gpt, lambda PR, lambda PL, and 3-phosphoglycerate kinase. Examples of promoters to cause expression in plants include promotors such as cauliflower mosaic virus (CaMV) 35S transcription initiation region, 1' or 2' promoter derived from T-DNA of *Agrobacterium tumefaciens,* and auxin. Examples of promoters to cause expression in *Saccharomyces cerevisiae* include ADH1, TDH3, PGK1, and GAL1. Examples of promoters to cause expression in fission yeasts include promoters such as adh, tef, nmt1, and CMV. Examples of the expression vector include plasmids, phages, phagemids, cosmids, fosmids, artificial chromosomes, retroviruses, lentiviruses, measles viruses, vaccinia viruses, adenoviruses, adeno-associated viruses, and Sendai viruses. The present invention provides cells (such as animal cells, plant cells, bacteria, archaebacteria, and fungi cells) containing these expression cassettes in their genomes. Other than above, a method for introducing linear or circular DNA encoding the fusion protein of the present invention and operably linked to a regulatory sequence into a cell (after the introduction, a stable expression line may be obtained), a method for introducing linear or circular DNA encoding the fusion protein of the present invention and operably linked to a regulatory sequence into a cellular genome using genome editing techniques (such as TALEN, ZFN, or CRISPR/Cas9 system), or the like may be used.

A method for introducing a nucleic acid into a cell without using a gene expression vector and a method for expressing a protein encoded by a nucleic acid are also known. The fusion protein of the present invention can be expressed in a cell by introducing a nucleic acid (such as a linear nucleic acid, e.g., linear DNA) encoding the fusion protein of the present invention and operably linked to a regulatory sequence (such as a promoter) into the cell. The fusion protein of the present invention can also be expressed in a cell by introducing mRNA encoding the fusion protein of the present invention into a cell.

According to the present invention, the nucleic acid encoding the fusion protein of the present invention can be DNA or messenger RNA (mRNA).

The present invention provides a composition containing a nucleic acid encoding the fusion protein of the present invention. The present invention provides a composition containing a gene expression vector which contains a nucleic acid encoding the fusion protein of the present invention. Such a composition can contain a nucleic acid and/or a gene expression vector, and an excipient. Examples of the excipient include excipients for stabilizing a nucleic acid and/or a gene expression vector. Examples of the excipients include salts, chelating agents, and pH adjusters.

The fusion protein of the present invention is a protein and thus can be introduced into a cell or be forcibly expressed by introduction of a nucleic acid encoding the fusion protein into a cell. Accordingly, the fusion protein of the present invention can be used for observing an antigen in a cell. The present invention can provide a composition containing the fusion protein of the present invention. The present invention can provide a composition (such as a composition for intracellular expression of the fusion protein of the present invention) containing a nucleic acid encoding the fusion protein of the present invention.

The present invention provides a method for observing an antigen (which can be hereinafter read as "method for observing the fusion protein of the present invention"). The method for observing an antigen can include observing the antigen bound to the fusion protein of the present invention. The "antigen" herein is not specifically limited and may mean an entity which can be an immunogen of an antibody, an entity which can be an immunogen of Affimer, an entity which can be an immunogen of Nanobody, or an entity which can be an immunogen of scFv. Such an antigen may bind to the fusion protein of the present invention, for example, in a solution or a cell, or on a cell surface. The antigen may or may not form a complex with another protein. The observation can be generally performed on the antigen bound to the fusion protein of the present invention in a solution or a cell, or on a cell surface. The observation can be performed by microscopy (such as fluorescence microscopy and fluorescence polarization microscopy). In the fusion protein of the present invention, the fluorescent protein and the antigen-binding protein are bound at a constant rotation angle, and the polarization of the fluorescence is constant with respect to the antigen-binding protein. Accordingly, the fusion protein of the present invention can be observed, preferably, by fluorescence polarization microscopy.

According to the present invention, examples of the antigen include antigens of animals, plants, bacteria, fungi, and archaebacteria. Examples of the antigen include intracellular antigens, extracellular antigens, and antigens on cell membranes. The antigen may be in the free form or may form a complex with any other structural component. The antigen may be in the monomer form or in the polymer form. In the present invention, the cell may be any of fertilized eggs, pluripotent stem cells, stem cells, progenitor cells, somatic cells, and germ cells (such as oocytes). In the present invention, the antigen may be components other than cells in organisms, such as components of flagella (for example, sperm or microbial flagella). According to the present invention, the antigen can be a cytoskeleton such as actin, microtubules, septin, and intermediate filaments. According to the present invention, the antigen can be an cytoskeleton such as actins (such as α-actin, β-actin, and γ-actin), microtubule tubulins (such as α-tubulin, β-tubulin, and γ-tubulin), septin, and intermediate filaments (such as keratin, GFAP (glial cell fibrous acidic protein), vimentin, neurofilament-M, neurofilament-L, neurofilament-H, α-internexin, lamin B1, lamin B2, and lamin A) in a cell. According to the present invention, the antigen is not specifically limited, but examples thereof include myosins (myosins such as myosin I, myosin II, myosin III, myosin IV, myosin V, myosin VI, myosin VIII, and myosin IX and heavy chains or light chains thereof), dyneins (such as axonemal dyneins and cytoplasmic dyneins), and kinesins (for example, but not specifically limited to, KIF1A, KIF1Bα, KIF1Bβ, KIF1C, KIF2A, KIF3, KIF4, KIF5, KIF13B, KIF17, KIF26A, KIFC1, KIFC2, and KIFC3, and other kinesins belonging to the kinesin superfamily). According to the present invention, the antigen is not specifically limited, but examples thereof include membrane proteins (such as receptor proteins, G proteins, ion transporters (ion channels and ion pumps), and transporter proteins). According to the present invention, the antigen is not specifically limited, but examples thereof include BAR domain proteins (such as Amphiphysin, EndophilinA1, FCHO2, Pacsin2, and IRSp53) and ESCRT-III proteins (such as CHMP2A, CHMP2B, CHMP4A, CHMP4B, and CHMP4C). In addition, the antigen is not particularly limited to the above examples and can be various biomolecules (such as proteins, lipids, nucleic acids, hormones, and sugars). According to the present invention, the antigen is not specifically limited and may be a lipid membrane or a lipid membrane domain {for example, fat-soluble fluorescent dyes and lipid-binding motifs can be used}. According to the present invention, cellular components other than the cytoskeleton can also be observed. For example, in the case of performing fluorescence polarization correlation spectroscopy or fluorescence polarization cross-correlation spectroscopy in the present invention, the rotational diffusion of cell components other than the cytoskeleton can be evaluated.

According to the present invention, the method for observing an antigen can include fluorescence correlation spectroscopy (FCS). The fluorescence correlation spectroscopy can comprise observing the molecular motion of the fluorescent entity by microscopy (such as confocal microscopy or a two-photon microscopy). The fluorescence correlation spectroscopy can comprise measuring the fluctuation in fluorescence intensity by irradiating the fusion protein of the present invention with light (such as laser light). The fluorescence correlation spectroscopy can comprise applying inverse Fourier transform to an intensity spectrum (time spectrum) to obtain temporal autocorrelation. The fluorescence correlation spectroscopy can evaluate the diffusion motion (translational diffusion motion) of the fusion protein of the present invention and the number of molecules in the measurement region from the results obtained. The method of the present invention can comprise determining the diffusion coefficient of the fusion protein of the present invention. According to the present invention, changes in fluorescence intensity derived from a plurality of types of fluorescent molecules can be simultaneously measured for detecting binding between a plurality of types of fusion proteins of the present invention. According to the present invention, the method for observing an antigen can comprise observing fluorescence polarization in an embodiment. The fluorescence polarization can be observed by fluorescence polarization microscopy. The rotational diffusion motion of the fusion protein can be evaluated by analyzing the intensity of the polarization through a polarizing plate. The method of the present invention can comprise determining the hydrodynamic radius of the fusion protein of the present invention. According to the present invention, the hydrodynamic radius can be used for evaluating the fluctuation in the structure of the fusion protein of the present invention, the fluctuation in the surrounding microscopic environment, the binding between the fusion protein of the present invention and other proteins, or the binding between the plurality of types of fusion proteins of the present invention.

According to the present invention, the method for observing an antigen can comprise observing each of an antigen bound to a first fusion protein which is the fusion protein of the present invention and a second antigen bound to a second fusion protein which is the fusion protein of the present invention {with the proviso that the first fusion protein and the second fusion protein have different fluorescence wavelengths}, in an aspect. According to the present invention, the method for observing an antigen can involve fluorescence correlation spectroscopy (FCS) of the antigen bound to a first fusion protein which is the fusion protein of the present invention and the second antigen bound to a second fusion protein which is the fusion protein of the present invention {with the proviso that the first fusion protein and the second fusion protein have different fluorescence wavelengths}. According to the present invention, the method for observing an antigen can comprise analyzing simultaneity of fluctuations in fluorescence intensity of two or more antigens (fluorescence cross-correlation spectroscopy (FCCS)). In order to measure the fluorescence intensity of two or more antigens, two or more fusion proteins of the present invention may be used (that is, the first fusion protein and the second fusion protein can be used, and a third fusion protein may be used, and a fourth fusion protein may further be used, and more than four fusion proteins of the present invention may be used, as required, for example (however, they preferably have different fluorescence wavelengths). According to the present invention, the method for observing an antigen can comprise observing fluorescence polarization in an aspect. The fluorescence polarization can be observed by fluorescence polarization microscopy. The advantage of performing fluorescence correlation spectroscopy (FCS) and fluorescence cross-correlation spectroscopy (FCCS) using a fluorescence polarization microscope is that the rotational diffusion motion of fluorescence can be detected. Accordingly, the method for observing an antigen in the present invention may further comprise detecting the rotational diffusion motion of molecules by observing fluorescence polarization, in an aspect. Since the rotational diffusion motion is sensitive to changes in particle size, it is expected that the binding state of two or more antigens can be evaluated more precisely by fluorescence polarization correlation spectroscopy. In the present invention, the method for observing an antigen can comprise evaluating the binding state of two or more antigens in cells or evaluating the binding state of two or more antigens in a solution, in an aspect.

The fluorescence polarization can be appropriately observed by those skilled in the art using a fluorescence polarization microscope. The polarization can be observed, for example, on two or more polarization planes including a first polarization plane and a second polarization plane. The polarization can be observed on four or more polarization planes including a first polarization plane, a second polarization plane, a third polarization plane, and a fourth polarization plane. It can be expected that, as the number of polarization planes to be observed increases, the sensitivity to detect the rotation of fluorescent molecules increases.

In the present invention, the fusion protein of the present invention can be observed by super-resolution microscopy. Examples of the super-resolution microscopy include stimulated emission depletion microscopy (STED), photoactivated localization microscopy (PALM), stochastic optical reconstruction microscopy, scanning near-field optical microscopy (SNOM), structured illumination microscopy, confocal laser microscopy, and two-photon excitation microscopy. In the super-resolution microscopy, the observation target can be observed with a resolution equal to or less than the diffraction limit of light (for example, a resolution of 200 nm or less). Under appropriate observation conditions such as total reflection illumination, fluorescent molecules can be detected as spots at the single molecular level. Also in super-resolution microscopy, antigens can be observed in the same manner as in fluorescence correlation spectroscopy (FCS) and fluorescence cross-correlation spectroscopy (FCCS), and the method using a luminescent entity instead of fluorescence. In the conventional super-resolution microscopy, only a position of an antigen is measured, whereas the structural correlation of antigens can be evaluated in the present invention by simultaneously observing the direction of polarization in addition to the positions of a plurality of antigens. It can be expected that this will be the experimental basis of the point image conversion algorithm which has not been conventionally present in super-resolution microscopy.

The present invention provides a protein having an amino acid sequence according to one sequence number selected from the group consisting of SEQ ID Nos: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, and 43, and a nucleic acid encoding such a protein. The present invention provides a vector for intracellular expression comprising the above nucleic acid.

The present invention provides a method for designing an amino acid sequence of a fusion protein, comprising: providing a first amino acid sequence which is an amino acid sequence of an antigen-binding protein and a second amino acid sequence which is an amino acid sequence of a fluorescent protein, wherein the N-terminus of the first amino acid sequence and the C-terminus of the second amino acid sequence or the C-terminus of the first amino acid sequence and the N-terminus of the second amino acid sequence are both helix structures or together form a helix structure upon excision of a part of the N-terminus or the C-terminus; and linking the N-terminus of the first amino acid sequence and the C-terminus of the second amino acid sequence or the C-terminus of the first amino acid sequence and the N-terminus of the second amino acid sequence, to obtain an amino acid sequence with the junction serving as a part of a helix structure. The fusion protein having the amino acid sequence designed can be suitable for observation of fluorescence polarization. Preferably, the excision of a part of the N-terminus or the C-terminus does not abolish the binding property of the antigen-binding protein and the fluorescence property of the fluorescent protein.

The present invention provides a method for designing an amino acid sequence of a fusion protein, comprising: providing a first amino acid sequence which is an amino acid sequence of an antigen-binding protein and a second amino acid sequence which is an amino acid sequence of a fluorescent protein, wherein the N-terminus of the first amino acid sequence and the C-terminus of the second amino acid sequence or the C-terminus of the first amino acid sequence and the N-terminus of the second amino acid sequence are both β-sheets or together form a β-sheet upon excision of a part of the N-terminus or the C-terminus; and linking the N-terminus of the first amino acid sequence and the C-terminus of the second amino acid sequence or the C-terminus of the first amino acid sequence and the N-terminus of the second amino acid sequence, to obtain an amino acid sequence with the junction serving as a part of a β-sheet structure. The fusion protein having the amino acid sequence designed can be suitable for observation of fluorescence polarization. Preferably, the excision of a part of the N-terminus or the C-terminus does not abolish the binding property of the antigen-binding protein and the fluorescence property of the fluorescent protein.

The present invention provides a method for designing a fusion protein, comprising: providing an amino acid sequence of a protein, wherein at least the N-terminus or the C-terminus of the amino acid sequence has a helix structure; and linking the helix and an amino acid sequence of a helix of an ALFA-tag (the ALFA-tag can have the amino acid sequence set forth in SEQ ID No: 40 or an amino acid sequence of a ALFA-tag corresponding thereto, for example), to obtain an amino acid sequence with the junction serving as a part of a helix structure. The designed fusion protein having the amino acid sequence can be detected by a fluorescent protein linked tightly to an antigen-binding protein that binds to the ALFA-tag and can be used for observation of fluorescence polarization. Preferably, the truncation of a part of the N-terminus or the C-terminus does not delete the functional region of the protein. In an aspect, the above protein can be an antigen-binding protein. In an aspect, the above protein can be a protein to be observed. Examples of the antigen-binding protein that binds to the ALFA-tag include Nanobody, scFv, Affimer, and the aforementioned examples of the antigen-binding protein that binds to the ALFA-tag, and examples of the fluorescent protein also include the aforementioned examples of the fluorescent protein. A fusion protein in which these are linked to each other via the helix structures or the β-sheets (for example, the fusion protein according to any one of (A) to (D) above that binds to the ALFA-tag) can be used for detecting the ALFA-tag. Further, the protein to be detected preferably has a helix structure at the N-terminus or the C-terminus and can be a fusion protein in which the helix structure is linked to the helix structure of the ALFA-tag so that the linkage site serves as a part of a helix structure.

The present invention provides a method comprising obtaining a nucleic acid sequence encoding the fusion protein, based on the amino acid sequence designed. The present invention can provide a method for producing a nucleic acid having a nucleic acid sequence encoding the amino acid sequence designed. The present invention can provide a nucleic acid (for example, DNA or mRNA) having a nucleic acid sequence encoding the amino acid sequence designed. The present invention further provides a gene expression vector comprising the nucleic acid having the nucleic acid sequence obtained. In the gene expression vector, the nucleic acid can be operably linked to a promoter. The gene expression vector obtained can be introduced into a cell so that the vector can be expressed, and thus can be used for synthesizing a fusion protein having the amino acid sequence designed in a cell. When the fusion protein obtained is suitable for observation of fluorescence polarization, the fusion protein and the nucleic acid encoding the protein can be useful for observation of fluorescence polarization.

The present invention provides a method for producing a fusion protein, based on the amino acid sequence designed as above.

### Examples

### Example 1: Design and observation of fusion protein of circular permutated mutant, cp-sfGFP, and Affimer

In this example, a fusion protein of a circular permutated mutant, cp-sfGFP, and an Affimer was designed.

The Affimer is a protein derived from stefin or cystatin and consisting of an α-helix and four folded β-sheet structures in contact with the α-helix. The four folded β-sheets are stabilized by the α-helix, and have a rigid tertiary structure. It is known that the four β-sheets form three loops, and amino acids can be introduced into two of them on the same side. It is also known that the Affimer can acquire selective binding affinity for antigens like antibodies, depending on the amino acids introduced. It has been also revealed that an Affimer exhibiting binding affinity for a desired antigen can be designed by techniques such as phage display.

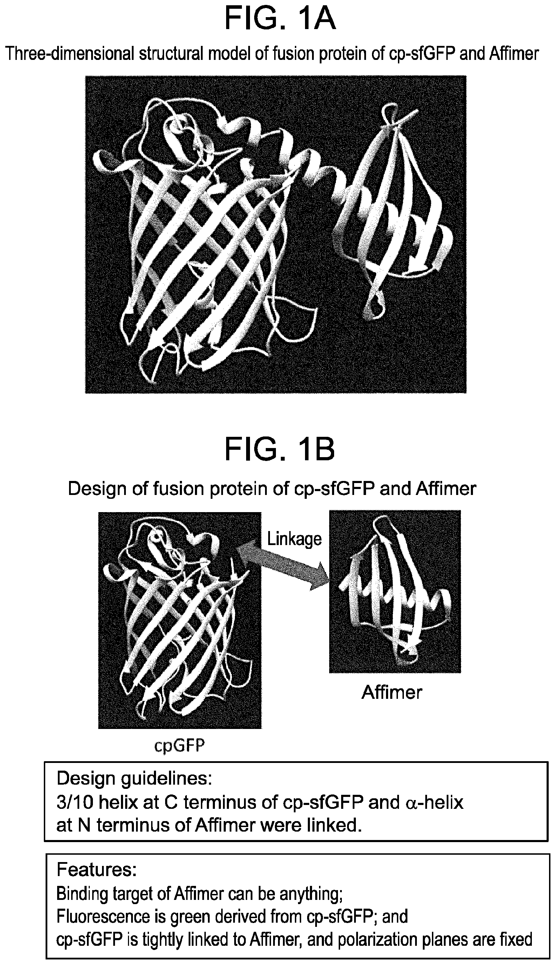

In this example, a cp-sfGFP having a helix (considered to be a 3/10 helix) at the C-terminus and an Affimer having an α-helix at the N-terminus were linked through the helix at the C-terminus and the helix at the N-terminus via a helix linker, to obtain a fusion protein (see Figure 1A).

Figure 1B shows an expected three-dimensional structural model of the fusion protein obtained.

As the cp-sfGFP, a cp-sfGFP having the amino acid sequence set forth in SEQ ID No: 1 was used. As the Affimer, an Affimer having the amino acid sequence set forth in SEQ ID No: 2 was used. This Affimer can bind selectively to actin fibers (F-actin). Using a genetic engineering technique, the amino acid sequence set forth in SEQ ID No: 1 and the amino acid sequence set forth in SEQ ID No: 2 were linked in-frame via a helix linker having the amino acid sequence set forth in SEQ ID No: 3, thereby designing a fusion protein having the amino acid sequence set forth in SEQ ID No: 4. SEQ ID No: 4 contains the amino acid sequence of a HA tag at the C-terminus.

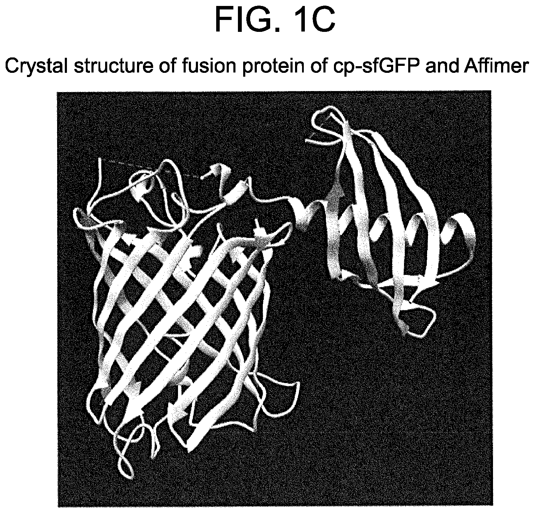

The fusion protein designed was produced in *Escherichia coli* and purified, and the fusion protein obtained was crystallized and subjected to X-ray crystal structure analysis. From the resultant X-ray diffraction image, it was revealed that the fusion protein had the crystal structure shown in Figure 1C. As shown in Figure 1C, it was revealed that the cp-sfGFP and the Affimer formed one new helix by linking the helix at the C-terminus and the helix at the N-terminus.

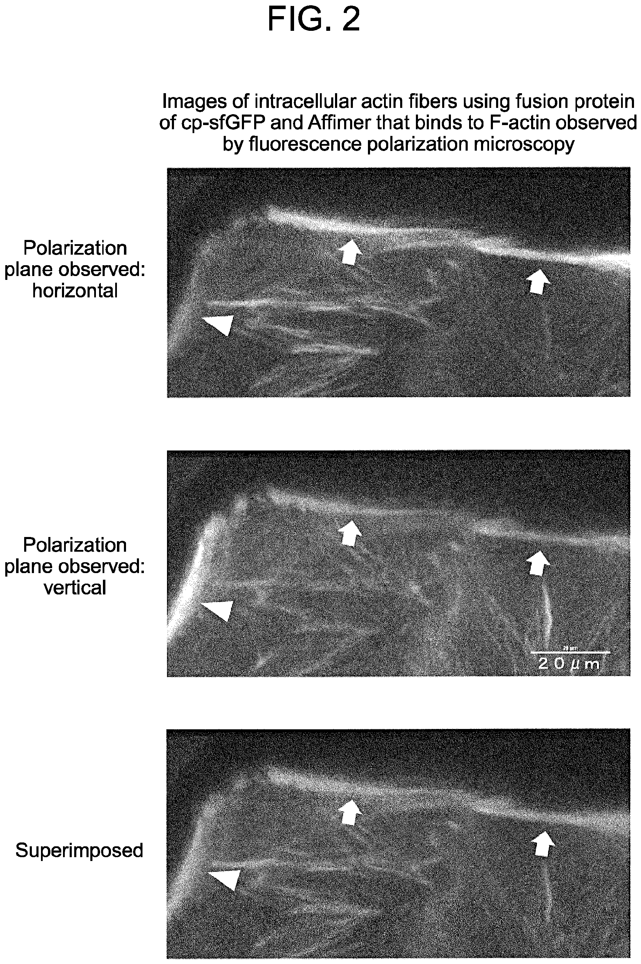

An expression vector in which DNA encoding the fusion protein designed was placed under an appropriate promoter was introduced by a lipofection method (Lipofectamine 3000 reagent), thereby allowing HeLa cells to express the above fusion protein. The cells expressing the fusion protein were observed by fluorescence polarization microscopy. It was expected that, when the fusion protein was bound to the actin fibers, if the fluorescence polarization planes of the fusion protein bound were aligned, the polarization (anisotropy) of fluorescence would be observed. Using a polarization beam splitter, images of the vertical observation polarization planes and the horizontal observation polarization planes were simultaneously obtained and compared. The results were as shown in Figure 2. As shown in Figure 2, an actin fiber bundle (see the arrowhead) in which a low fluorescence intensity was observed when the observation polarization plane was horizontal showed a high fluorescence intensity when the observation polarization plane was vertical, whereas an actin fiber bundle (see the arrow) in which a high fluorescence intensity was observed when the observation polarization plane was horizontal showed a low fluorescence intensity when the observation polarization plane was vertical. These facts indicated that the polarization planes of the fusion protein bound to actin fibers were aligned. The same experiment was performed using LLC-PK1 cells, Ptk2 cells, yeast cells, starfish oocytes, or fertilized eggs instead of HeLa cells, and then, it was confirmed that polarization of the above fusion protein can be observed in these cells.

Affimers are considered to bind to actin fibers in a uniformly aligned orientation. This is because the actin fibers are polymers composed of repeating actin monomers. The fact that the polarization planes of the fusion protein are aligned indicates that a cp-sfGFP in the fusion protein is firmly immobilized on an Affimer. Accordingly, the method for linking the helix at the C-terminus and the helix at the N-terminus in the fusion protein of the present invention provides a methodology in which a cp-sfGFP is immobilized on an Affimer, and the polarization planes of the cp-sfGFP are immobilized on the Affimer. It was shown that production of a circular permutated mutant in which the helix at the N-terminus of sfGFP is moved to the C-terminus so as to be linked to the α-helix at the N-terminus of Affimer can be useful for the methodology for immobilization.

This enables construction of a fluorescence labeling method in which binding to a desired antigen and polarized emission occur. The effectiveness of the strategy of the fluorescence polarization labeling method was further confirmed by the following examples.

### Example 2: Design and observation of fusion protein of circular permutated mutant, cpmVenus, and Affimer

Using the strategy disclosed in Example 1, a cpmVenus and an Affimer were linked. Specifically, the 3/10 helix present at the C-terminus of the cpmVenus and the α-helix present at the N-terminus of the Affimer were linked in-frame, to design a fusion protein.

As the cpmVenus, a fluorescent protein having the amino acid sequence set forth in SEQ ID No: 5 was used. As the Affimer, an Affimer having the same amino acid sequence as in Example 1 was used. Using a genetic engineering technique, the amino acid sequence set forth in SEQ ID No: 5 and the amino acid sequence set forth in SEQ ID No: 2 were linked in-frame via alanine (A), thereby designing a fusion protein of cpmVenus and Affimer having the amino acid sequence set forth in SEQ ID No: 6. SEQ ID No: 6 contains the amino acid sequence of a HA tag at the C-terminus.

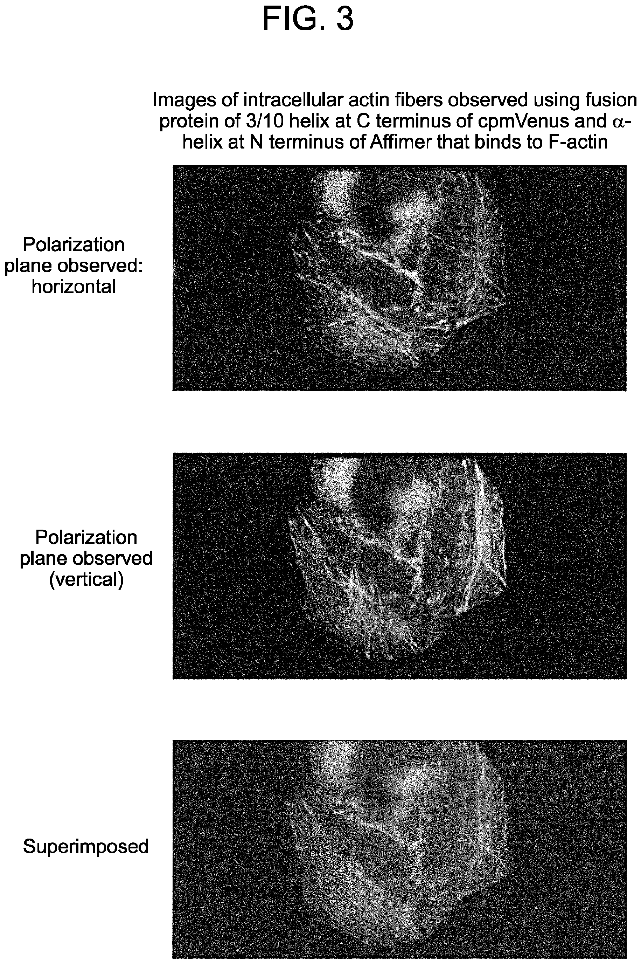

An expression vector in which DNA encoding the fusion protein designed was disposed under an appropriate promoter was introduced by a lipofection method (Avalanche-Everyday Transfection Reagent), thereby allowing HeLa cells to express the above fusion protein. The cells expressing the fusion protein were observed by fluorescence polarization microscopy. The results were as shown in Figure 3. As shown in Figure 3, an actin fiber bundle in which a low fluorescence intensity was observed when the observation polarization plane was horizontal showed a high fluorescence intensity when the observation polarization plane was vertical, whereas an actin fiber bundle in which a high fluorescence intensity was observed when the observation polarization plane was horizontal showed a low fluorescence intensity when the observation polarization plane was vertical. This indicated that the fusion protein obtained by directly linking the 3/10 helix present at the C-terminus of the cpmVenus and the α-helix present at the N-terminus of the Affimer in-frame also showed polarization.

### Example 3: Design and observation of fusion protein of circular permutated mutant, cpmTurquoise2, and Affimer

Using the strategy disclosed in Example 1, a cpmTurquoise2 and an Affimer were linked. Specifically, the 3/10 helix present at the C-terminus of the cpmTurquoise2 and the α-helix present at the N-terminus of the Affimer were linked in-frame, to design a fusion protein.

As the cpmTurquoise2, a fluorescent protein having the amino acid sequence set forth in SEQ ID No: 7 was used. As the Affimer, an Affimer having the same amino acid sequence as in Example 1 was used. Using a genetic engineering technique, the amino acid sequence set forth in SEQ ID No: 7 and the amino acid sequence set forth in SEQ ID No: 2 were linked in-frame via alanine (A), thereby designing a fusion protein of cpmTurquoise2 and Affimer having the amino acid sequence set forth in SEQ ID No: 8. SEQ ID No: 8 contains the amino acid sequence of a HA tag at the C-terminus.

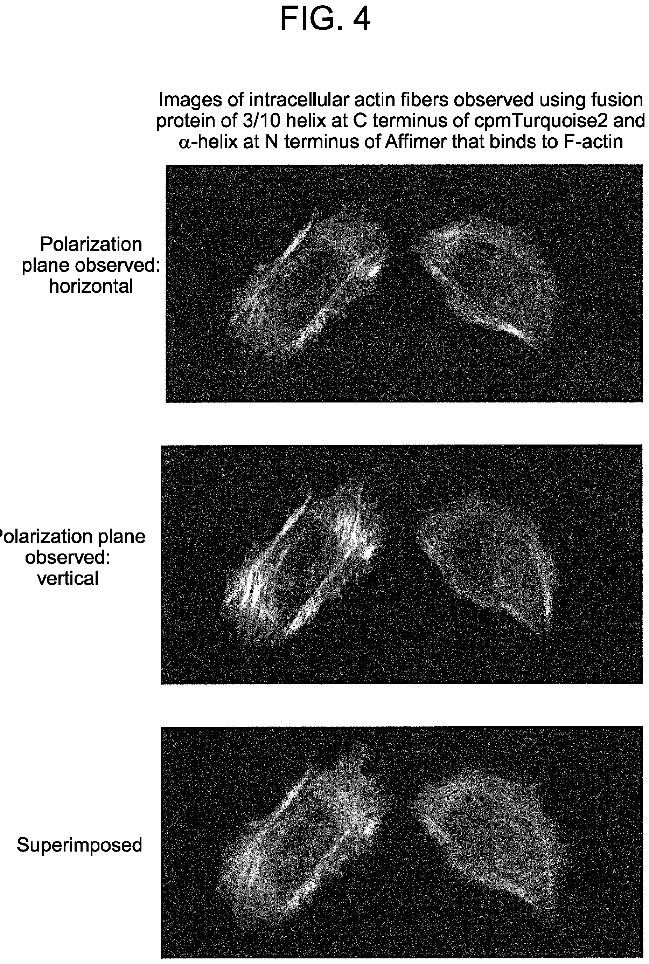

An expression vector in which DNA encoding the fusion protein designed was disposed under an appropriate promoter was introduced by a lipofection method (Avalanche-Everyday Transfection Reagent), thereby allowing HeLa cells to express the above fusion protein. The cells expressing the fusion protein were observed by fluorescence polarization microscopy. The results were as shown in Figure 4. As shown in Figure 4, an actin fiber bundle in which a low fluorescence intensity was observed when the observation polarization plane was horizontal showed a high fluorescence intensity when the observation polarization plane was vertical, whereas an actin fiber bundle in which a high fluorescence intensity was observed when the observation polarization plane was horizontal showed a low fluorescence intensity when the observation polarization plane was vertical. This indicated that the fusion protein obtained by linking the 3/10 helix present at the C-terminus of the cpmTurquoise2 and the α-helix present at the N-terminus of the Affimer in-frame also showed polarization.

From Examples 1 to 3 above, the strategy of linking the helix at the C-terminus of a fluorescent protein and the helix at the N-terminus of an Affimer enables the fluorescence polarization planes to be immobilized on the Affimer and provides a new technique for observation of fluorescence polarization of target proteins (that is, Affimer antigens).

### Example 4: Design and observation of fusion protein of fluorescence-labeled tag and Affimer

Using the strategy disclosed in Examples above, a fluorescence-labeled tag and an Affimer were linked. As the fluorescence-labeled tag, a HaloTag was used. The HaloTag is a protein labeling technique which enables binding to various cell membrane-permeable fluorescent ligands. The HaloTag can be recruited to a desired antigen by linking the HaloTag and an Affimer. An attempt was made to apply the HaloTag to observation of fluorescence polarization by using the strategy disclosed in Examples above.

### (1) Fusion protein (first)

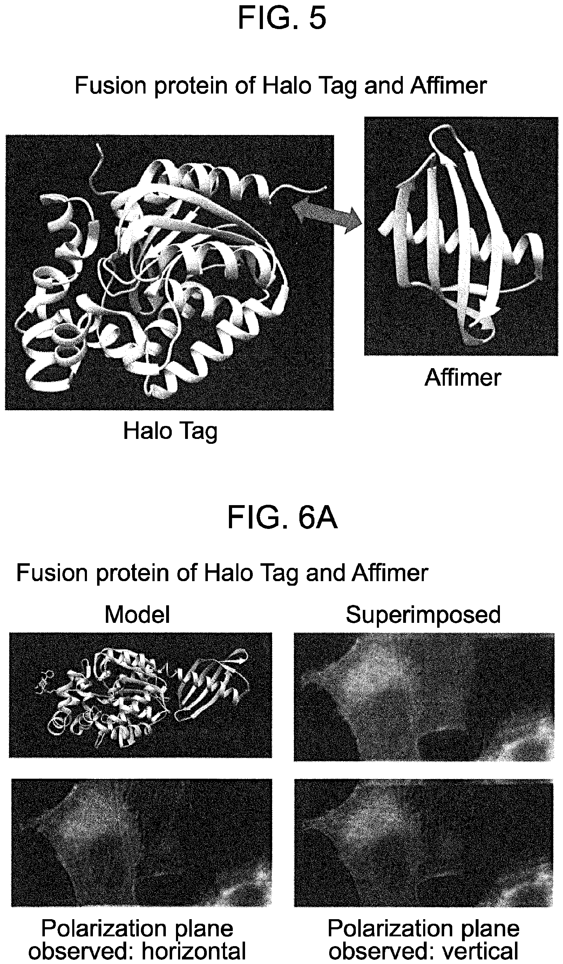

As the HaloTag, a protein having the amino acid sequence set forth in SEQ ID No: 9 was used. As the Affimer, an Affimer having the same amino acid sequence as in Example 1 was used. Using a genetic engineering technique, the amino acid sequence set forth in SEQ ID No: 9 and the amino acid sequence set forth in SEQ ID No: 2 were linked in-frame via a helix linker having the amino acid sequence of SEQ ID No: 10, thereby designing a fusion protein of HaloTag and Affimer having the amino acid sequence set forth in SEQ ID No: 11. The HaloTag has an α-helix structure at the C-terminus, as shown in Figure 5. In this example, the α-helix at the C-terminus and the α-helix of the Affimer at the N-terminus were linked via the above helix linker (see Figure 5).

An expression vector in which DNA encoding the fusion protein designed was disposed under an appropriate promoter was introduced by a lipofection method (Lipofectamine 3000 reagent), thereby allowing HeLa cells to express the above fusion protein. As the HaloTag ligand, an Oregon Green ligand was used, and the cells expressing the fusion protein were observed by fluorescence polarization microscopy. The results were as shown in Figure 6A. As shown in Figure 6A, an actin fiber bundle in which a low fluorescence intensity was observed when the observation polarization plane was horizontal showed a high fluorescence intensity when the observation polarization plane was vertical, whereas an actin fiber bundle in which a high fluorescence intensity was observed when the observation polarization plane was horizontal showed a low fluorescence intensity when the observation polarization plane was vertical. This indicated that the fusion protein obtained by directly linking the α-helix present at the C-terminus of the HaloTag and the α-helix present at the N-terminus of the Affimer in-frame also showed polarization. From the fact that polarization was shown, it was considered that there was tightly immobilized binding among the fluorescence dye, the HaloTag, and the Affimer, and the binding had almost no flexibility.

It was revealed from this that, when the α-helix present at the C-terminus of the HaloTag and the α-helix present at the N-terminus of the Affimer were linked by the strategy of this example, the HaloTag firmly bound to the Affimer, thereby enabling observation of fluorescence polarization via the fluorescence label bound to the HaloTag.

### (2) Fusion protein (second; with the number of turns changed)

The α-helix is a secondary structure of a protein in which one turn is composed of 3.5 amino acids. It was examined whether the polarization planes rotate by linking α-helices to each other with an insertion of an amino acid (the number of turns changed).

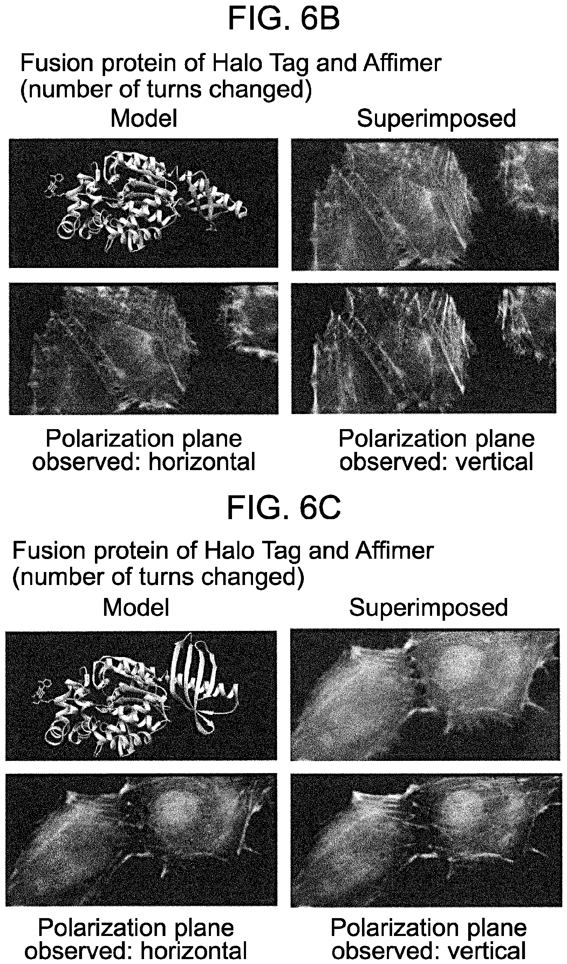

As the HaloTag, a fluorescent protein having the amino acid sequence set forth in SEQ ID No: 9 was used. As the Affimer, an Affimer having the same amino acid sequence as in Example 1 was used. Using a genetic engineering technique, the amino acid sequence set forth in SEQ ID No: 9 and the amino acid sequence set forth in SEQ ID No: 2 were linked in-frame via a helix linker having the amino acid sequence of SEQ ID No: 12, thereby designing a fusion protein of HaloTag and Affimer having the amino acid sequence set forth in SEQ ID No: 13. The length of the helix linker was different from the fusion protein shown in Figure 6A by one amino acid (that is, the rotation angle was different by about 100 degrees). As will be understood by comparing Figure 6A and Figure 6B, the bond angle of the Affimer to the HaloTag was different between the fusion proteins of Figure 6A and Figure 6B.

An expression vector in which DNA encoding the fusion protein designed was placed under an appropriate promoter was introduced by a lipofection method (Lipofectamine 3000 reagent), thereby allowing HeLa cells to express the above fusion protein. As the HaloTag ligand, an Oregon Green ligand was used, and the cells expressing the fusion protein were observed by fluorescence polarization microscopy. The results were as shown in Figure 6B. As shown in Figure 6B, an actin fiber bundle in which a low fluorescence intensity was observed when the observation polarization plane was horizontal showed a high fluorescence intensity when the observation polarization plane was vertical, whereas an actin fiber bundle in which a high fluorescence intensity was observed when the observation polarization plane was horizontal showed a low fluorescence intensity when the observation polarization plane was vertical. This indicated that the fusion protein obtained by directly linking the α-helix present at the C-terminus of the HaloTag and the α-helix present at the N-terminus of the Affimer in-frame also showed polarization.

It was clear from this that, even if the α-helix present at the C-terminus of the HaloTag and the α-helix present at the N-terminus of the Affimer were linked with the number of turns changed, the fluorescence polarization labeling strategy was established. The models disclosed in Figure 6A and Figure 6B demonstrated that the rotation angle of polarization could be freely changed in principle by changing the number of turns.

### (3) Fusion protein (third; with the number of turns further changed)

The α-helices were linked to each other with an insertion of a further amino acid (the number of turns further changed).

As the HaloTag, a fluorescent protein having the amino acid sequence set forth in SEQ ID No: 9 was used. As the Affimer, an Affimer having the same amino acid sequence as in Example 1 was used. Using a genetic engineering technique, the amino acid sequence set forth in SEQ ID No: 9 and the amino acid sequence set forth in SEQ ID No: 2 were linked in-frame via a helix linker having the amino acid sequence of SEQ ID No: 14, thereby designing a fusion protein of HaloTag and Affimer having the amino acid sequence set forth in SEQ ID No: 15. The length of the helix linker was different from the fusion protein shown in Figure 6A by 2 amino acids (that is, the rotation angle was different by about 200 degrees). As will be understood by comparing Figure 6A, Figure 6B, and Figure 6C, the bond angle of the Affimer to the HaloTag was different among the fusion proteins of Figure 6A, Figure 6B, and Figure 6C.

An expression vector in which DNA encoding the fusion protein designed was placed under an appropriate promoter was introduced by a lipofection method (Lipofectamine 3000 reagent), thereby allowing HeLa cells to express the above fusion protein. As the HaloTag ligand, an Oregon Green ligand was used, and the cells expressing the fusion protein were observed by fluorescence polarization microscopy. The results were as shown in Figure 6C. As shown in Figure 6C, an actin fiber bundle in which a low fluorescence intensity was observed when the observation polarization plane was horizontal showed a high fluorescence intensity when the observation polarization plane was vertical, whereas an actin fiber bundle in which a high fluorescence intensity was observed when the observation polarization plane was horizontal showed a low fluorescence intensity when the observation polarization plane was vertical. This indicated that the fusion protein obtained by directly linking the α-helix present at the C-terminus of the HaloTag and the α-helix present at the N-terminus of the Affimer in-frame also showed polarization. It should be noted that, even if a TMR ligand or a HMSiR ligand was used as the HaloTag ligand instead of the Oregon Green ligand, strong polarization was observed, and polarization could be observed regardless of the fluorescent entity used as the HaloTag ligand.

It was further confirmed from this that, even if the α-helix present at the C-terminus of the HaloTag and the α-helix present at the N-terminus of the Affimer were linked with the number of turns changed, the fusion proteins can work in the fluorescence polarization labeling strategy.

### Example 5: Design and observation of fusion protein of circular permutated mutant, cp-sfGFP, and Nanobody

In this example, a fusion protein of a fluorescent protein and a Nanobody was designed.

Being different from common antibodies found in humans, rabbits, and mice (antibodies with heavy and light chains), Nanobodies, which are antibodies consisting only of heavy chains (for example, an antibody of cartilaginous fishes such as sharks and chimaeras, and antibodies of camels, Arabian camels, llamas, alpacas, and the like), include those capable of binding to antigens only in the heavy chain variable regions, and those derived from the heavy chain variable regions and the light chain variable regions. Peptides containing these variable regions have a molecular weight of about 1/10 (approximately 12 to 15 kDa) of ordinary IgG antibodies and are called Nanobodies. It has also been revealed that Nanobodies exhibiting binding affinity for desired antigens can be designed by a technique such as phage display.

The sfGFP (superfolder GFP) is a variant of GFP obtained from *Aequorea victoria.* It is characterized by rapidly forming a structure (folding) in cells. In this example, an attempt to link a sfGFP and a Nanobody through their β-sheets was made. Since the Nanobody had a β-sheet at the C-terminus, a circular permutated mutant having a β-sheet at the N-terminus, cp-sfGFP (Specifically, cp10/8 (10/9) sfGFP), was constructed.

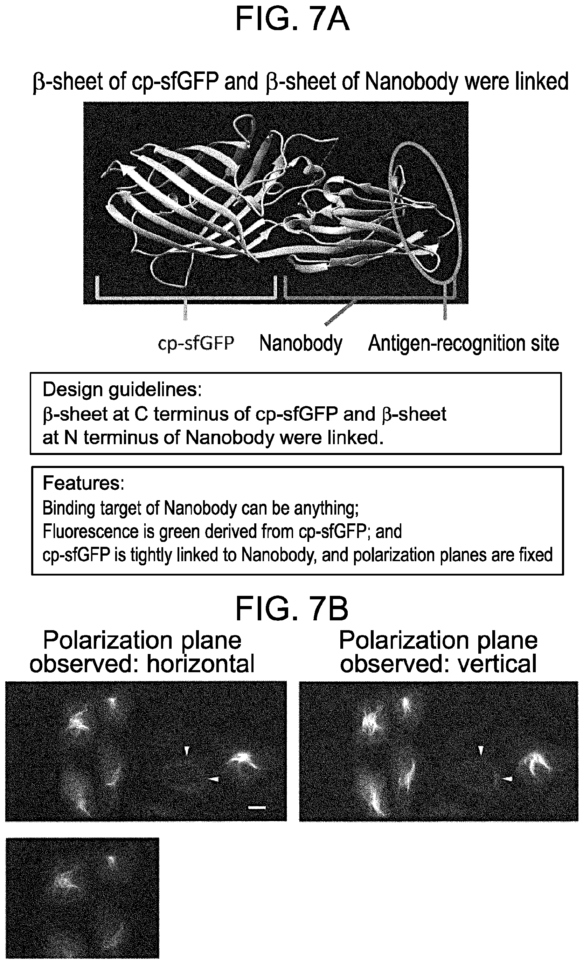

As the circular permutated mutant having a β-sheet at the N-terminus, cp-sfGFP, a fluorescent protein having the amino acid sequence set forth in SEQ ID No: 1 was used. As the Nanobody, a vimentin-binding Nanobody having the amino acid sequence set forth in SEQ ID No: 16 was used. Using a genetic engineering technique, the β-sheet at the N-terminus of the cp-sfGFP having the amino acid sequence set forth in SEQ ID No: 1 and the β-sheet at the C-terminus of the vimentin-binding Nanobody having the amino acid sequence set forth in SEQ ID No: 16 were linked, to design a fusion protein having the amino acid sequence set forth in SEQ ID No: 17. The amino acid sequence set forth in SEQ ID No: 17 had a His tag and a T7 tag at the N-terminus. Figure 7A shows an expected structural model of the fusion protein. In this model, the site where the Nanobody bound to antigens was present in the opposite direction to that of the fluorescent protein, thereby ensuring the accessibility to the antigens.

An expression vector in which DNA encoding the fusion protein designed was placed under an appropriate promoter was introduced by a lipofection method (Avalanche-Everyday Transfection Reagent), thereby allowing HeLa cells to express the above fusion protein. The cells expressing the fusion protein were observed by fluorescence polarization microscopy. The results were as shown in Figure 7B. As shown in Figure 7B, vimentin fibers in which a low fluorescence intensity was observed when the observation polarization plane was horizontal showed a high fluorescence intensity when the observation polarization plane was vertical, whereas vimentin fibers in which a high fluorescence intensity was observed when the observation polarization plane was horizontal showed a low fluorescence intensity when the observation polarization plane was vertical. In this way, the fusion protein produced could stain vimentin fibers, and its fluorescence exhibited polarization.

This suggested that the strategy to obtain a fusion protein by linking the β-sheet at the N-terminus of the cp-sfGFP and the β-sheet at the C-terminus of the Nanobody could be useful for fluorescence polarization labeling.

### Example 6: Design and observation of fusion protein of Nanobody and cp176-172mVenus

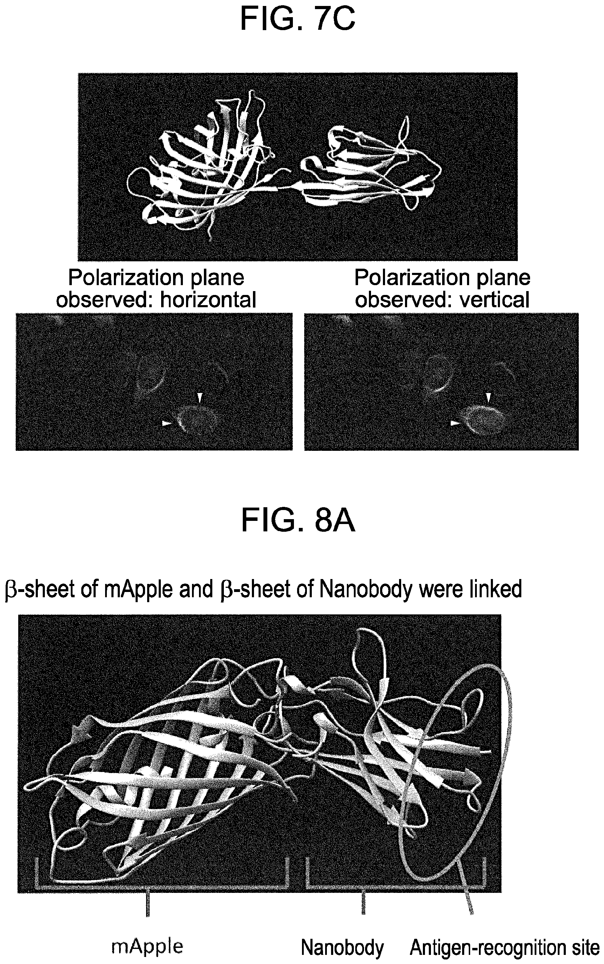

Further, a fusion protein (SEQ ID No: 31; the amino acid sequence set forth in SEQ ID No: 31 had a His tag and a T7 tag at the N-terminus) in which a Nanobody that binds to vimentin (SEQ ID No: 16) and a cp176-172mVenus (SEQ ID No: 30) were linked via VV was designed (see the panel in the upper part of Figure 7C) and expressed in HeLa cells in the same manner as in Example 5. As the cells expressing the fusion protein were observed by fluorescence polarization microscopy, intracellular vimentin could be observed, and its fluorescence exhibited polarization, as shown in Figure 7C. The orientation of polarization was different from that in the previous cp-sfGFP. The cp-sfGFP of Example 5 and the cp176-172mVenus used in this example were basically the same type of fluorescent proteins but different in the position of cp. Accordingly, this example indicated that the angle of polarization can be changed by creating circular permutated mutants of the fluorescent protein at various positions.

### Example 7: Design and observation of fusion protein of mApple and Nanobody

Then, a fusion protein of another fluorescent protein, mApple, and a Nanobody was designed.

As the mApple, a fluorescent protein having the amino acid sequence set forth in SEQ ID No: 18 was used. As the Nanobody, a vimentin-binding Nanobody having the amino acid sequence set forth in SEQ ID No: 16 was used. Using a genetic engineering technique, the β-sheet at the N-terminus of the mApple having the amino acid sequence set forth in SEQ ID No: 18 and the β-sheet at the C-terminus of the vimentin-binding Nanobody having the amino acid sequence set forth in SEQ ID No: 16 were linked via an inflexible linker ("rigid linker"), thereby designing a fusion protein having the amino acid sequence set forth in SEQ ID No: 19. The amino acid sequence set forth in SEQ ID No: 19 had a His tag and a T7 tag at the N-terminus. Figure 8A shows an expected structural model of the fusion protein. In this model, the site where the Nanobody binds to antigens is present in the opposite direction to the fluorescent protein, thereby ensuring the accessibility to the antigens.

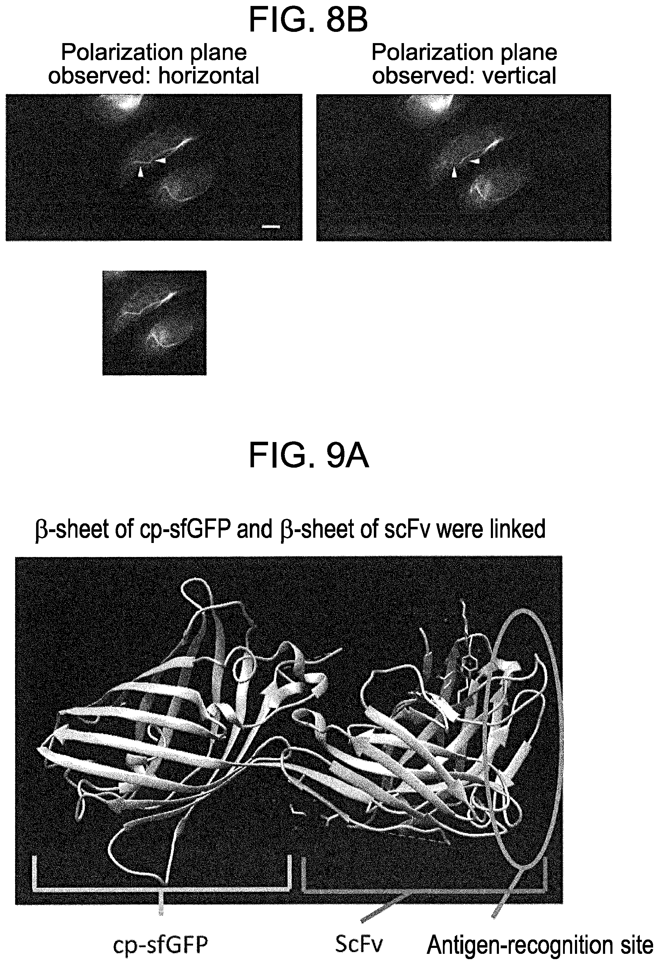

An expression vector in which DNA encoding the fusion protein designed was disposed under an appropriate promoter was introduced by a lipofection method (Avalanche-Everyday Transfection Reagent), thereby allowing HeLa cells to express the above fusion protein. The cells expressing the fusion protein were observed by fluorescence polarization microscopy. The results were as shown in Figure 8B. As shown in Figure 8B, vimentin fibers in which a low fluorescence intensity was observed when the observation polarization plane was horizontal showed a high fluorescence intensity when the observation polarization plane was vertical, whereas vimentin fibers in which a high fluorescence intensity was observed when the observation polarization plane was horizontal showed a low fluorescence intensity when the observation polarization plane was vertical. In this way, the fusion protein produced could stain vimentin fibers, and its fluorescence exhibited polarization.

In this way, it was revealed that the fluorescent protein can be labeled with the Nanobody without losing the polarization by utilizing the β-sheet structure at the C-terminus of the Nanobody and linking it to the β-sheet structure present at the N-terminus of the fluorescent protein.

### Example 8: Design and observation of fusion protein of cp-sfGFP and scFv

In this example, a cp-sfGFP having a β-sheet structure at the N-terminus was designed and linked to the β-sheet structure present at the C-terminus of a scFv, to obtain a fusion protein.

The scFv is a protein having a structure in which heavy-chain variable regions and light-chain variable regions of antibodies are linked by a flexible linker. In the case of production from antibodies by a genetic engineering technique, the binding property of the antibodies can be maintained. At present, a technique for producing a scFv by phage display is well known, which enables a scFv having a desired binding property to a desired antigen to be obtained.

As the cp-sfGFP, a circular permutated mutant of sfGFP having the amino acid sequence set forth in SEQ ID No: 1 was used. As the scFv, a scFv that has the amino acid sequence set forth in SEQ ID No: 20 and binds to non-muscle myosin IIA was used. Using a genetic engineering technique, the β-sheet structure at the N-terminus of the sfGFP having the amino acid sequence set forth in SEQ ID No: 1 and the β-sheet structure at the C-terminus of the scFv having the amino acid sequence set forth in SEQ ID No: 20 were directly linked, to design a fusion protein having the amino acid sequence set forth in SEQ ID No: 21. The amino acid sequence set forth in SEQ ID No: 21 had a His tag and a T7 tag at the N-terminus. Figure 9A shows an expected three-dimensional structure of the fusion protein. The antigen recognition site of the scFv fusion protein was exposed on the opposite side of the cp-sfGFP fused, thereby ensuring the accessibility of antigens to the antigen recognition site.

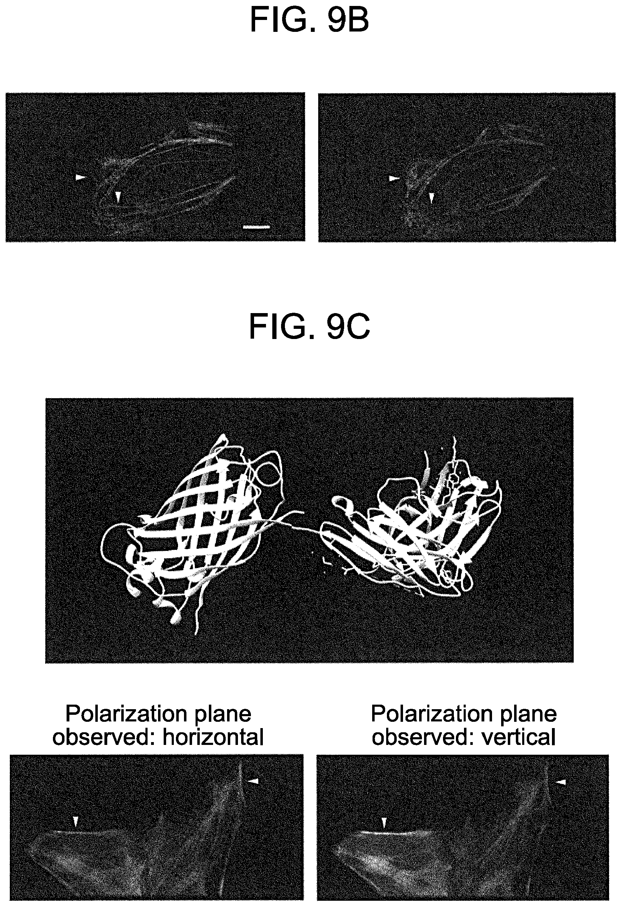

An expression vector in which DNA encoding the fusion protein designed was placed under an appropriate promoter was introduced by a lipofection method (Avalanche-Everyday Transfection Reagent), thereby allowing HeLa cells to express the above fusion protein. The cells expressing the fusion protein were observed by fluorescence polarization microscopy. The results were as shown in Figure 9B. As shown in Figure 9B, a part in which a low fluorescence intensity was observed when the observation polarization plane was horizontal showed a high fluorescence intensity when the observation polarization plane was vertical, whereas a part in which a high fluorescence intensity was observed when the observation polarization plane was horizontal showed a low fluorescence intensity when the observation polarization plane was vertical. In this way, the fusion protein produced could stain myosin, and its fluorescence exhibited polarization.

Further, a fusion protein (SEQ ID No: 32) in which a scFv (SEQ ID No: 20) and a cp176-172mVenus (SEQ ID No: 30) were linked via VV was designed (see the panel in the upper part of Figure 9C) and expressed in HeLa cells in the same manner. As the cells expressing the fusion protein were observed by fluorescence polarization microscopy, intracellular myosin could be observed, and its fluorescence exhibited polarization, as shown in Figure 9C. The orientation of polarization was different from that in the above-described cp-sfGFP-based fusion protein. The cp-sfGFP and the cp176-172mVenus in this example were basically the same type of fluorescent proteins but different in the position of cp. Accordingly, this example indicated that the angle of polarization can be changed by creating circular permutated mutants of the fluorescent protein at various positions.

In this way, it was revealed that the fluorescent protein can be labeled with the scFv without losing the polarization by utilizing the β-sheet structure at the C-terminus of the scFv and linking it to the β-sheet structure present at the N-terminus of the fluorescent protein.

### Example 9: Design and observation of fusion protein of Affimer and mScarlet

The N-terminus of a mScarlet was truncated and linked to the β-sheet at the C-terminus of an Affimer, to obtain a fusion protein of Affimer and mScarlet. As the mScarlet, a protein having the amino acid sequence set forth in SEQ ID No: 36, created by truncating amino acids at the N-terminus was used. As the Affimer, an actin-binding Affimer having the amino acid sequence set forth in SEQ ID No: 2 was used. Using a genetic engineering technique, these were linked, to design a fusion protein having the amino acid sequence set forth in SEQ ID No: 37.

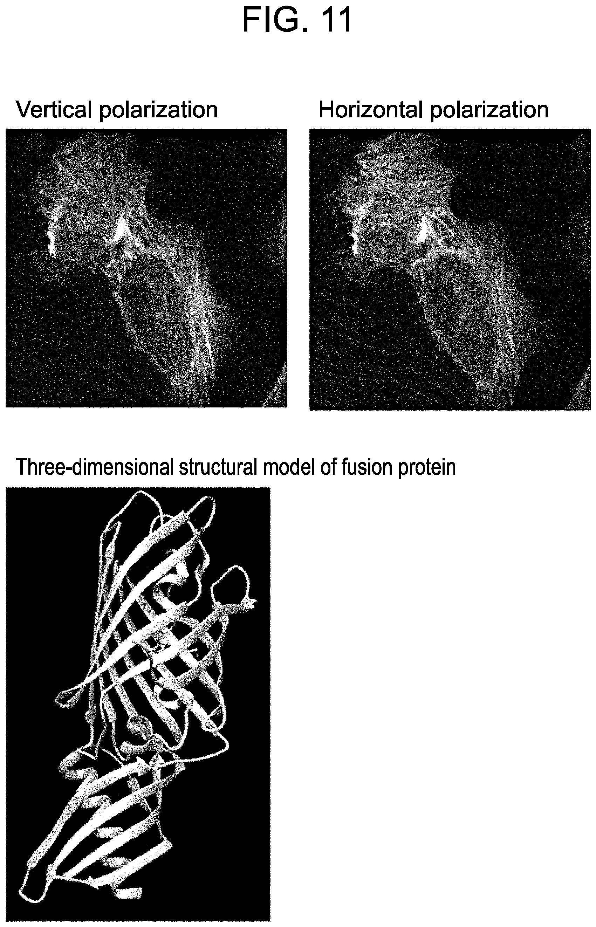

The fusion protein obtained is shown by a three-dimensional structural model in Figure 11. An expression vector in which DNA encoding the fusion protein designed was placed under an appropriate promoter was introduced by a lipofection method (Lipofectamine 3000 reagent), thereby allowing HeLa cells to express the above fusion protein. The cells expressing the fusion protein were observed by fluorescence polarization microscopy.

The results were as shown in Figure 11. As shown in Figure 11, the images observed with vertical polarization and horizontal polarization were different, and this revealed that the fusion protein obtained was a fluorescent protein that binds to an antigen (actin) and exhibit polarization under polarization microscopic observation.

### Example 10: Design and observation of fusion protein of Affimer and mNeonGreen

The β-sheet at the N-terminus of a mNeonGreen was exposed and linked to the β-sheet at the C-terminus of an Affimer, to obtain a fusion protein of Affimer and mNeonGreen. As the mNeonGreen, a protein having the amino acid sequence set forth in SEQ ID No: 38, created by truncating amino acids at the N-terminus was used. As the Affimer, an actin-binding Affimer having the amino acid sequence set forth in SEQ ID No: 2 was used. Using a genetic engineering technique, these were linked, to design a fusion protein having the amino acid sequence set forth in SEQ ID No: 39.

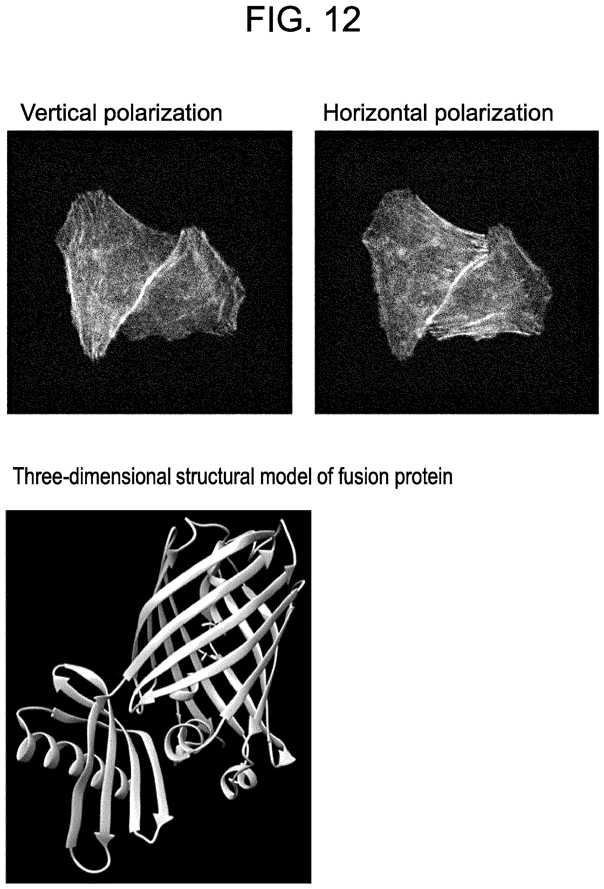

The fusion protein obtained is shown by a three-dimensional structural model in Figure 12. In Figure 12, it can be seen that the β-sheet at the C-terminus of the Affimer and the β-sheet at the N-terminus of the mNeonGreen form a stretch of β-sheets. An expression vector in which DNA encoding the fusion protein designed was placed under an appropriate promoter was introduced by a lipofection method (Lipofectamine 3000 reagent), thereby allowing HeLa cells to express the above fusion protein. The cells expressing the fusion protein were observed by fluorescence polarization microscopy.

The results were as shown in Figure 12. As shown in Figure 12, the images observed with vertical polarization and horizontal polarization were different, and this revealed that the fusion protein obtained was a fluorescent protein that binds to an antigen (actin) and exhibit polarization under polarization microscopic observation.

### Example 11: Technique for observing fluorescence polarization by introducing ALFA-tag into protein

In this example, an attempt was made to establish a technique for enabling fluorescence polarization observation of a protein of interest without obtaining a protein binding to the protein. Specifically, a system to label the protein to be observed with an ALFA-tag and detect the protein using a Nanobody that binds to the ALFA-tag was established.

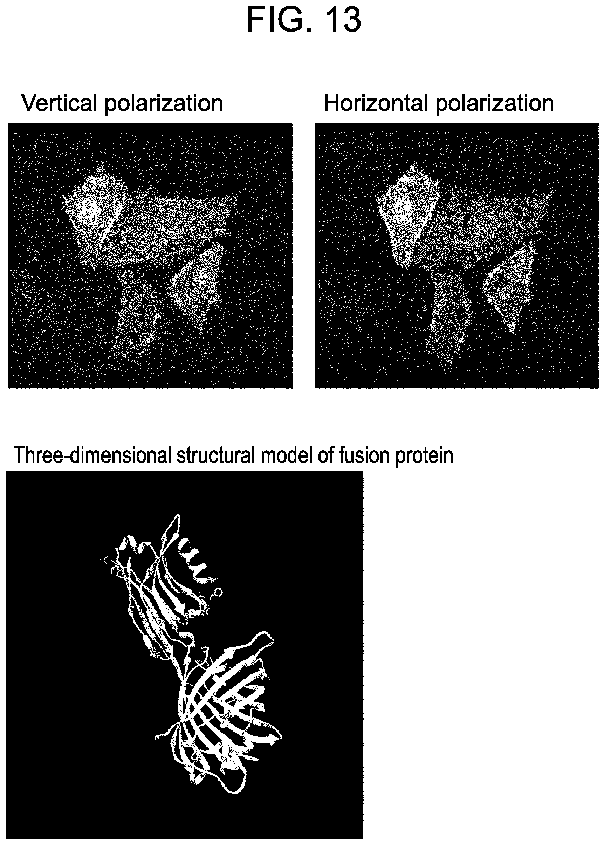

Specifically, an ALFA-tag (SEQ ID No: 40) was introduced into an Affimer (SEQ ID No: 2) that binds to F-actin as a model system for verifying the above detection system, to obtain a fusion protein having the amino acid sequence set forth in SEQ ID No: 41. The three-dimensional structural model of the fusion protein was as shown in Figure 13. This fusion protein was expressed in HeLaM cells. A fusion protein of an anti-ALFA-tag Nanobody (SEQ ID No: 42) and cp176-172mVenus (SEQ ID No: 43) were brought into contact, and fluorescence polarization was observed. The fusion protein of anti-ALFA-tagged Nanobody and cp176-172mVenus was a fusion protein suitable for observation of fluorescence polarization, in which the Nanobody and the cp176-172mVenus were tightly linked (c.f. Example 5).

The results were as shown in Figure 13. As shown in Figure 13, the images observed with vertical polarization and horizontal polarization were different, and this revealed that the fusion protein obtained was a fluorescent protein that binds to an antigen (actin) to exhibit polarization under polarization microscopic observation. In an embodiment of this example, first, the Affimer firmly recognized the F-actin. Then, the Affimer and the ALFA-tag were tightly linked, and the Nanobody firmly recognized the ALFA-tag. Thereby, the interaction and linkage between any proteins became tight, therefore it was concluded the F-actin could be observed using the fluorescence polarization of the cp176-172mVenus.

### Example 12: Other Examples

In addition, it was examined whether fluorescence polarization labeling was possible in the same manner by designing the fusion proteins shown in Table 1 below. In any of the following cases, it was confirmed that antigens could be fluorescently labeled, and the fluorescence clearly showed polarization and could be used as a fluorescence polarization labeling. Further, the Affimer, the Nanobody, and the scFv could be used as antigen-binding proteins. In Table 1, utrophin is a protein known as a protein that binds to actin. [Table 1]

**Table 1: Examples of fluorescence polarization labeling**

| Protein at N terminus (Number of amino acid sequence) | Protein at C terminus (Number of amino acid sequence) | Linkage form (Number of amino acid sequence of fusion protein) |
|---|---|---|
| miRFP670 (SEQ ID No: 22) | Affimer that binds to actin (SEQ ID No: 2) | Linkage between α-helices (SEQ ID No: 24) |
| | | *α-helices were linked via helix linker (SEQ ID No: 23). |
| Nanobody that binds to vimentin (SEQ ID No: 16) | cpmTurquoise2 (SEQ ID No: 7) | Linkage between β-sheets (SEQ ID No: 25) |
| | | *Amino acid sequence set forth in SEQ ID No: 25 had His tag and T7 tag at N terminus. |
| Nanobody that binds to vimentin (SEQ ID No: 16) | cpmVenus (SEQ ID No: 5) | Linkage between β-sheets (SEQ ID No: 26) |
| | | *Amino acid sequence set forth in SEQ ID No: 26 had His tag and T7 tag at N terminus. |
| scFv that binds to non-muscle myosin IIA (SEQ ID No: 20) | cpmTurquoise2 (SEQ ID No: 7) | Linkage between β-sheets (SEQ ID No: 27) |
| | | *Amino acid sequence set forth in SEQ ID No: 27 had His tag and T7 tag at N terminus. |
| Actin binding site of utrophin (SEQ ID No: 33) | mEGFP (SEQ ID No: 34) | Linkage between α-helices (SEQ ID No: 35) |
| | | *Helix linker of SEQ ID No: 3 was used as linker. Results are shown in Figure 10. |

As has been described above, all of the fusion proteins designed in Examples were suitable for fluorescence observation of antigens and all were suitable for fluorescence polarization observation. It was considered to be advantageous in the observation of polarization that fluorescence labels and antigens were linked in a constrained manner.

According to the results of Examples, it can be effective in fluorescence polarization labeling that a helix structure at a terminus of a fluorescent protein or a fluorescence-labeled tag protein and a helix structure at a terminus of an antigen-binding protein are linked, and that a β-sheet structure at a terminus of a fluorescent protein and a β-sheet structure at a terminus of an antigen-binding protein are linked. Regarding fluorescent proteins with no helix or no β-sheet structure at a terminus, it can be a useful technique for protein modification to produce a circular permutated mutant of a fluorescent protein so as to have a helix or a β-sheet structure at a terminus, for example.

Further, it was revealed that helices might be directly linked to each other or might be linked via a linker that may have a helix structure. In the case of linkage via a linker that may have a helix structure, the bond angle of the two protein parts in the fusion protein can be rotated by about 100 degrees by changing the length of the linker by one amino acid, thereby possibly widening the range of the strategy of techniques for producing fusion proteins. It was revealed that, also in the case of linkage between β-sheet structures, they might be directly linked, might be linked by deleting a terminus, or might be linked via a rigid amino acid residue such as valine (V). It is advantageous for observing polarization that a fluorescence label and an antigen were linked in a constrained manner, and it turned out that all the various linking methods shown in Examples were effective for observing polarization.

### SEQUENCE LISTING

SEQ ID No: 1: Example of amino acid sequence of cp-sfGFP
SEQ ID No: 2: Example of amino acid sequence of Affimer that binds to actin
SEQ ID No: 3: Example of amino acid sequence of helix linker
SEQ ID No: 4: Example of amino acid sequence of fusion protein of cp-sfGFP and Affimer
SEQ ID No: 5: Example of amino acid sequence of cpmVenus
SEQ ID No: 6: Example of amino acid sequence of fusion protein of cpmVenus and Affimer
SEQ ID No: 7: Example of amino acid sequence of cpmTurquoise2
SEQ ID No: 8: Example of amino acid sequence of fusion protein of cpmTurquoise2 and Affimer
SEQ ID No: 9: Example of amino acid sequence of HaloTag
SEQ ID No: 10: Example of amino acid sequence of helix linker 2
SEQ ID No: 11: Example of amino acid sequence of fusion protein of HaloTag and Affimer
SEQ ID No: 12: Example of amino acid sequence of helix linker 3
SEQ ID No: 13: Example of amino acid sequence of fusion protein of HaloTag and Affimer
SEQ ID No: 14: Example of amino acid sequence of helix linker 4
SEQ ID No: 15: Example of amino acid sequence of fusion protein of HaloTag and Affimer
SEQ ID No: 16: Example of amino acid sequence of Nanobody that binds to vimentin
SEQ ID No: 17: Example of amino acid sequence of fusion protein of Nanobody and cp-sfGFP
SEQ ID No: 18: Amino acid sequence of mApple
SEQ ID No: 19: Example of amino acid sequence of fusion protein of Nanobody and mApple
SEQ ID No: 20: Example of amino acid sequence of scFv that binds to non-muscle myosin IIA
SEQ ID No: 21: Example of amino acid sequence of fusion protein of scFv and cp-sfGFP
SEQ ID No: 22: Amino acid sequence of miRFP670
SEQ ID No: 23: Example of amino acid sequence of helix linker 5
SEQ ID No: 24: Example of amino acid sequence of fusion protein of miRFP670 and Affimer
SEQ ID No: 25: Example of amino acid sequence of fusion protein of Nanobody and cpmTurquoise2
SEQ ID No: 26: Example of amino acid sequence of fusion protein of Nanobody and cpmVenus
SEQ ID No: 27: Example of amino acid sequence of fusion protein of scFv and cpmTurquoise2
SEQ ID No: 28: Amino acid sequence of Haloalkane dehalogenase
SEQ ID No: 29: Amino acid sequence of H272F mutant of Haloalkane dehalogenase
SEQ ID No: 30: Example of amino acid sequence of cp176-172mVenus
SEQ ID No: 31: Example of amino acid sequence of fusion protein of Nanobody and cp176-172mVenus
SEQ ID No: 32: Example of amino acid sequence of fusion protein of scFV and cp176-172mVenus
SEQ ID No: 33: Example of amino acid sequence of actin binding domain of utrophin
SEQ ID No: 34: Example of amino acid sequence of mEGFP
SEQ ID No: 35: Example of amino acid sequence of fusion protein of actin binding domain of utrophin and mEGFP
SEQ ID No: 36: Example of amino acid sequence of mScarlet fragment with N-terminus excised
SEQ ID No: 37: Example of amino acid sequence of fusion protein of Affimer that binds to actin and mScarlet
SEQ ID No: 38: Example of amino acid sequence of mNeonGreen fragment with N-terminus excised
SEQ ID No: 39: Example of amino acid sequence of fusion protein of Affimer that binds to actin and mNeonGreen
SEQ ID No: 40: Example of amino acid sequence of ALFA-tag
SEQ ID No: 41: Example of amino acid sequence of Affimer with ALFA-tag introduced
SEQ ID No: 42: Example of amino acid sequence of anti-ALFA-tag Nanobody
SEQ ID No: 43: Example of amino acid sequence of fusion protein of anti-ALFA-tag Nanobody and cp176-172mVenus

## Claims

1. A fusion protein of an antigen-binding protein and a fluorescent protein or a fluorescence-labeled tag, wherein
the antigen-binding protein is an antigen-binding protein having a helix structure or a β-sheet structure at a terminus,
the fluorescent protein or the fluorescence-labeled tag is a fluorescent protein or a fluorescence-labeled tag having a helix structure or a β-sheet structure at a terminus, and
the helix at the terminus and the helix at the terminus are linked, or
the β-sheet structure at the terminus and the β-sheet structure at the terminus are linked.

2. The fusion protein according to claim 1, wherein
the antigen-binding protein has a helix structure at the N-terminus,
the fluorescent protein or the fluorescence-labeled tag has a helix structure at the C-terminus, and
the helix at the terminus of the antigen-binding protein and the helix at the terminus of the fluorescent protein or the fluorescence-labeled tag are linked.

3. The fusion protein according to claim 2, wherein the antigen-binding protein is an Affimer.

4. The fusion protein according to claim 2 or 3,
wherein the fluorescent protein is a fluorescent protein having a helix structure at the C-terminus, selected from GFP and a fluorescent protein having a GFP-like β-barrel structure, and a circular permutated mutant of a point mutant.

5. The fusion protein according to claim 1, wherein
the antigen-binding protein has a β-sheet structure at the C-terminus,
the fluorescent protein has a β-sheet structure at the N-terminus, and
the β-sheet structure at the C-terminus and the β-sheet structure at the N-terminus are linked.

6. The fusion protein according to claim 5, wherein the antigen-binding protein is a Nanobody or a scFv.

7. The fusion protein according to claim 5 or 6,
wherein the fluorescent protein is a fluorescent protein having a β-sheet structure at the N-terminus, selected from the group consisting of GFP and a fluorescent protein having a GFP-like β-barrel structure, and a point mutant thereof, and a circular permutated mutant and a deletion mutant thereof.

8. The fusion protein according to claim 2 or 3,
wherein the fluorescence-labeled tag is a dehalogenation domain of haloalkane dehalogenase having an amino acid sequence corresponding to the amino acid sequence set forth in SEQ ID No: 9 or 29, having a helix structure at the C-terminus.

9. The fusion protein according to claim 2 or 3,
wherein the fluorescent protein is a phytochrome- or cyanobacteriochrome-based near-infrared fluorescent protein having a helix structure at the C-terminus.

10. A composition comprising the fusion protein according to any one of claims 1 to 9.

11. A nucleic acid encoding the fusion protein according to any one of claims 1 to 9.

12. A gene expression vector for use in expressing a fusion protein in a cell, or a cell transformed with the vector, comprising
the nucleic acid of claim 11,
wherein the nucleic acid is operably linked to a regulatory sequence, or
a cell introduced with a mRNA comprising the nucleic acid of claim 11.

13. A method for observing an antigen, comprising
observing an antigen bound to the fusion protein according to any one of claims 1 to 9.

14. The method according to claim 13, wherein the antigen is an antigen in an aqueous solution or a cell, or on a cell surface.

15. The method according to claim 13 or 14, comprising observing each of:
a first antigen bound to a first fusion protein which is the fusion protein according to any one of claims 1 to 9; and
a second antigen bound to a second fusion protein which is the fusion protein according to any one of claims 1 to 9, with the proviso that the first fusion protein and the second fusion protein have different fluorescence wavelengths.

16. The method according to claim 15, comprising
performing fluorescence correlation spectroscopy or fluorescence cross-correlation spectroscopy on the first fusion protein and the second fusion protein.

17. The method according to claim 16, wherein the fluorescence correlation spectroscopy and the fluorescence cross-correlation spectroscopy are each fluorescence polarization correlation spectroscopy.

18. A method for designing an amino acid sequence of a fusion protein, comprising:
providing a first amino acid sequence which is an amino acid sequence of an antigen-binding protein and a second amino acid sequence which is an amino acid sequence of a fluorescent protein,
wherein the N-terminus of the first amino acid sequence and the C-terminus of the second amino acid sequence or the C-terminus of the first amino acid sequence and the N-terminus of the second amino acid sequence are both helix structures or β-sheet structures, together form a helix structure upon excision of a part of the N-terminus or the C-terminus, or together form a β-sheet structure upon excision of a part of the N-terminus or the C-terminus; and
linking the N-terminus of the first amino acid sequence and the C-terminus of the second amino acid sequence or the C-terminus of the first amino acid sequence and the N-terminus of the second amino acid sequence, to obtain an amino acid sequence with the junction serving as a part of a helix structures or a β-sheet structures.
